(19) Europäisches Patentamt
European Patent Office
Office européen des brevets

(11) EP 0 778 264 B1

(12) EUROPÄISCHE PATENTSCHRIFT

(45) Veröffentlichungstag und Bekanntmachung des Hinweises auf die Patenterteilung:
**11.07.2001 Patentblatt 2001/28**

(21) Anmeldenummer: **96118872.9**

(22) Anmeldetag: **26.11.1996**

(51) Int Cl.[7]: **C07C 217/46**, C07C 217/20, C07C 255/30, C07D 211/46, C07D 211/58, C07C 239/12, C07C 323/23, C07C 235/78, A61K 31/13, A61K 31/275, A61K 31/445, A61K 31/16

(54) **Tertiäre Amine mit antimykotischer und cholesterinsenkender Wirkung**

Tertiary amines having antimycotic and cholesterol lowering activity

Amines tertiaires ayant une activité antimycotique et hypocholestérolémique

(84) Benannte Vertragsstaaten:
**AT BE CH DE DK ES FI FR GB GR IE IT LI LU NL PT SE**

(30) Priorität: **08.12.1995 CH 347995**

(43) Veröffentlichungstag der Anmeldung:
**11.06.1997 Patentblatt 1997/24**

(73) Patentinhaber: **F. HOFFMANN-LA ROCHE AG
4070 Basel (CH)**

(72) Erfinder:
- **Aebi, Johannes
4051 Basle (CH)**
- **Lengsfeld, Hans
4054 Basel (CH)**
- **Dehmlow, Henrietta
79639 Grenzach-Wyhlen (DE)**
- **Morand, Olivier
68220 Hegenheim (FR)**
- **Himber, Jacques
68500 Guebwiller (FR)**
- **Schmid, Gérard
4468 Kienberg (CH)**
- **Jolidon, Synèse
4223 Blauen (CH)**
- **Ji, Yu-Hua
4058 Basle (CH)**

(74) Vertreter: **Witte, Hubert, Dr. et al
F.Hoffmann-La Roche AG
Patent Department (PLP),
124 Grenzacherstrasse
4070 Basel (CH)**

(56) Entgegenhaltungen:
**EP-A- 0 410 359**
**EP-A- 0 464 465**
**EP-A- 0 636 367**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

EP 0 778 264 B1

**Beschreibung**

Die vorliegende Erfindung betrifft neue tertiäre Amine, Verfahren zu deren Herstellung, pharmazeutische Präparate, die solche Verbindungen enthalten, sowie die Verwendung dieser Verbindungen bei der Herstellung pharmazeutischer Präparate.

Tertiäre Amine, welche eine verwandte chemische Struktur aufweisen, sind aus den europäischen Patentanmeldungen Nr. 410 359, 464 465 und 636 367 bekannt.

Die Erfindung betrifft insbesondere tertiäre Amine der Formel

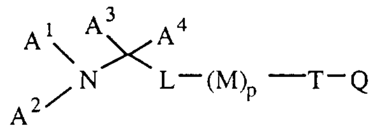

I

worin

| | |
|---|---|
| $A^1$ | Alkyl oder Alkenyl und |
| $A^2$ | Cycloalkyl, Cycloalkyl-alkyl oder eine gegebenenfalls durch eine Gruppe $R^1$, $CONH_2$ oder CN substituierte Alkyl- oder Alkenylgruppe und, falls $A^1$ Alkyl ist, $A^2$ auch OH sein kann, |
| $A^3$ und $A^4$ | Wasserstoff oder Alkyl sind oder |
| $A^1$ | zusammen mit $A^2$ oder mit $A^3$ eine gegebenenfalls durch $R^1$ substituierte Alkylen-, Alkenylen- oder Alkadienylengruppe $A^1$-$A^2$ bzw. $A^1$-$A^3$ mit bis zu 5 C-Atomen bildet, wobei in einer Gruppe $A^1$-$A^2$ oder $A^1$-$A^3$ bis zu 2 C-Atome durch ein (oder zwei) N-Atom(e) und/oder durch eine Gruppe N-Alkyl ersetzt sein können, |
| $R^1$ | an einem gesättigten C-Atom von $A^2$, $A^1$-$A^2$ oder $A^1$-$A^3$ gebundenes OH, Oxo, Alkyl (O oder S) oder Dialkylamino ist, wobei ein durch $R^1$ substituiertes C-Atom bzw. ein in $A^1$, $A^2$, $A^1$-$A^2$ oder $A^1$-$A^3$ enthaltenes ungesättigtes C-Atom in einer anderen als der $\alpha$-Stellung zu $N(A^1A^2)$ gebunden sein sollen, |
| p = 1 und L | Phenylen oder direkt oder über O, NH oder N(Alkyl oder Alkanoyl) an M gebundenes Alkylen oder Alkenylen mit insgesamt bis zu 11 C-Atomen und zumindest 4 bzw. 3 C-Atomen zwischen den zwei freien Valenzen oder Cycloalkylen-alkylen oder |
| p = 0 und L an T | gebundenes $C_{6\text{-}11}$-Alkenylen oder $C_{6\text{-}11}$-Alkadienylen, |
| M | Thienylen, Pyridylen, 1,4-Phenylen, durch einen oder mehreren Substituenten aus der Gruppe von Alkyl, Halogen, $N(R^2,R^{21})$, $CONH_2$, CN, $NO_2$, $CF_3$, OH, Alkyl (O oder S), 1,2,4-Triazol-1-yl oder Tetrazol-1-yl substituiertes 1,4-Phenylen oder eine Gruppe der Formel |

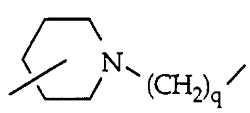

($M^1$)

| | |
|---|---|
| q | 1 oder 0, |
| $R^2$ und $R^{21}$ | H, Alkyl, Alkenyl, Alkanoyl oder $SO_2$-Alkyl, |
| T | CO, $CH(R^3)$, $C(R^4,R^5)$ oder $C{=}NOR^6$ und, falls M eine Gruppe $M^1$ und q = 0 ist, T auch $SO_2$ sein kann, |
| $R^3$ | OH, F, Alkoxy oder Alkanoyloxy, |
| $R^4$ OH und $R^5$ | Alkyl, Alkenyl, Alkinyl, Cycloalkyl oder $CF_3$ oder |
| $R^4$ zusammen mit $R^5$ | die Gruppe $CH_2$, $CH_2O$ oder $CH_2CH_2$, |
| $R^6$ | H, Alkyl oder Alkenyl, |
| Q | Cycloalkyl, $C(R^7,R^8)$, durch einen oder mehreren Substituenten aus der Gruppe von Alkyl, Halogen, $N(R^9,R^{10})$, $CONH_2$, CN, $NO_2$, $CF_3$, 1,2,4-Triazol-1-yl und Tetrazol-1-yl substituiertes Phenyl oder eine geradkettige Alkyl-, Alkenyl-, Alkadie- |

nyl- oder Alkatrienylgruppe Q' mit 0 bis 3 Methylsubstituenten und insgesamt 6 bis 13 C-Atomen ist, wobei eine solche Gruppe Q' durch OH und/oder durch N ($R^9$,$R^{10}$) substituiert sein kann,

$R^7$ und $R^8$ $C_{5-11}$-Alkyl, $C_{5-11}$-Alkenyl oder $C_{5-11}$-Alkadienyl und

$R^9$ und $R^{10}$ H, Alkyl, Alkenyl oder Alkanoyl sind, mit den Massnahmen, dass a) in einer Verbindung der Formel I, in der T eine Gruppe CO oder CHOH, L Phenylen oder eine direkt oder über O oder N-Alkyl an M gebundene Alkylen- oder Alkenylengruppe, M 1,4-Phenylen oder durch Alkyl, Alkoxy, Halogen, CN, $NO_2$ oder $CF_3$ monosubstituiertes 1,4-Phenylen und Q substituiertes Phenyl, eine Alkenylgruppe oder eine gegebenenfalls durch OH substituierte Alkylgruppe ist, $A^2$ nicht Alkyl oder Alkenyl sein soll oder $A^1$ zusammen mit $A^2$ nicht Alkylen sein soll,

b) in einer Verbindung der Formel I, in der $A^1$ zusammen mit $A^2$ Alkylen oder durch $R^1$ substituiertes Alkylen, $A^2$ Hydroxyalkyl oder $A^1$ und $A^2$ je eine Alkylgruppe sind, M nicht Pyridylen sein soll und

c) in einer Verbindung der Formel I, in der T eine Gruppe C(OH, $R^{51}$), worin $R^{51}$ Alkyl, Alkenyl, Alkinyl oder Cycloalkyl, M 1,4-Phenylen oder substituiertes 1,4-Phenylen und L eine über ein O-Atom an M gebundene Alkylengruppe ist, letztere zumindest 5 C-Atomen zwischen den 2 freien Valenzen und insgesamt bis zu 11 C-Atomen enthalten soll,

und Säureadditionssalze davon.

Im Rahmen der vorliegenden Erfindung bezeichnen Ausdrücke, wie "Alkyl", "Alkenyl", "Alkadienyl" und "Alkatrienyl" allein oder in Kombination, wie in Cycloalkyl-alkyl, einbindige und wenn nicht anders angegeben, geradkettige oder verzweigte Gruppen mit bis zu 20, insbesondere bis zu 13 C-Atomen; ferner bei Alkyl, Alkenyl und Alkadienyl bis zu 8 C-Atomen, bei Alkyl und Alkenyl bis zu 6, insbesondere bis zu 4 C-Atome. Beispiele für Alkyl sind Methyl, Aethyl, Propyl, Isopropyl, n-, s- und t-Butyl, Pentyl, Hexyl, Decyl und Dodecyl, für Alkanoyl: Formyl und Acetyl, für Alkenyl: Vinyl, Allyl, Propenyl, Butenyl, 3-Methyl-2-butenyl, 4-Methyl-3-pentenyl und Undodecenyl, für Alkinyl: Aethinyl, für Alkadienyl: 4-Methyl-1,3-pentadienyl; 3,7-Dimethyl-2,6-octadienyl und 4,8-Dimethyl-3,7-nonadienyl, für Alkatrienyl: 4,8-Dimethyl-1,3,7-nonatrienyl. "Alkylen", "Alkenylen" und "Alkadienylen" bezeichnen den weiter oben definierten einbindigen Alkyl-, Alkenyl- bzw. Alkadienylgruppen entsprechende zweibindige Gruppen, wie Pentylen und 3-Methylpentylen; Propenylen und 2,6-Dimethyl-1-hexenylen; 1,5-Dimethyl-1,5-hexadienylen; 2,6-Dimethyl-1,5-hexadienylen; 2,6-Dimethyl-1,5-octadienylen und 3,7-Dimethyl-3,7-octadienylen. "Cycloalkyl" und "Cycloalkylen" allein oder in Kombination enthalten vorzugsweise 3 bis 6 C-Atome, wie z.B. Cyclopropyl und Cyclohexyl bzw. Cyclopropylen. Beispiele von "Thienylen-" und "Pyridylen"-gruppen sind 2,5-Thienylen bzw. 2,5- oder 3,6-Pyridylen.

Vorzugsweise steht $A^1$ für Methyl, Aethyl oder Allyl; $A^2$ für Methyl, Aethyl, Allyl, Hydroxy, Hydroxypropyl, 2-Methoxyäthyl, 2-Methylsulfanyläthyl, Carbamoylmethyl, 2-Oxo-1-propyl, 2-Cyanäthyl, Cyclopropyl, Cyclopropylmethyl; N($A^1$,$A^2$) für Imidazolyl, 4-Hydroxy-piperidin-1-yl und 4-Dimethylamino-piperidin-1-yl; $A^3$ und $A^4$ für Wasserstoff oder Methyl; ($A^1$,$A^2$)N-C($A^3$,$A^4$)- für 1-Methylpyrrolidin-2-yl; L für $(CH_2)_5O$, $CH=CHCH_2O$, 1,4-Phenylen, Cyclopropylen-methylenoxy, $(CH_2)_5$-NH, $CH=CHCH_2NH$, $(CH_2)_5$N(Acetyl), $CH=CHCH_2$N(Acetyl), $CH=C(CH_3)CH_2CH_2CH_2CH(CH_3)$, $CH=C(CH_3)CH_2CH_2CH=C(CH_3)$, $C(CH_3)=CHCH_2CH_2C(CH_3)=CH$, $CH=C(CH_3)CH_2CH_2CH=C(CH_3)CH_2CH_2$ und $CH_2CH_2C(CH_3)=CHCH_2CH_2C(CH_3)=CH$; M für 1,4-Phenylen, das durch Fluor, OH, $NH_2$, $NHCH_3$, $N(CH_3)_2$, NH(CHO), $NH(SO_2CH_3)$, $SCH_3$ oder 1,2,4-Triazol-1-yl monosubstituiert, durch Fluor und Methyl substituiert oder durch Fluor di- oder tetrasubstituiert sein kann; T für CO, CHOH, $SO_2$, $C=CH_2$, $C(CH_2CH_2)$, C(OH,Vinyl), CHF, $C(OH,CH_3)$, $C(OH,CF_3)$, C(OH,Cyclopropyl), C=NOH, $C=NOCH_3$, C=NO-tert.Butyl oder C=NO-Allyl; Q für Bromphenyl, Cyanphenyl, Carbamoylphenyl, Difluorphenyl, durch F und $N(CH_3)_2$ substituiertes Phenyl, Cyclohexyl, 4-Methylpentyl, 3-Butenyl, 4-Methyl-3-pentenyl, 4-Methyl-1,3-pentadienyl; 4,8-Dimethyl-1,3,7-nonatrienyl, 10-Aminodecyl, 10-Acetaminodecyl, 2-Hydroxy-12-(allyl-methyl-amino)-dodecyl, 12-(Allyl-methyl-amino)-1-dodecenyl, 2-Hydroxy-4-methyl-3-pentenyl; 4,8-Dimethyl-2-hydroxy-3,7-nonadienyl, $CH[CH_2CH=C(CH_3)_2]_2$ oder $CH[CH_2CH=C(CH_3)CH_2CH_2CH=C(CH_3)_2]_2$.

Als pharmazeutisch annehmbare Säureadditionssalze kommen Salze der Verbindungen I mit anorganischen und organischen Säuren, wie HCl, HBr, $H_2SO_4$, $HNO_3$, Citronensäure, Essigsäure, Bernsteinsäure, Fumarsäure, Weinsäure, Methansulfonsäure und p-Toluolsulfonsäure, in Betracht.

Die Verbindungen der Formel I, die ein oder mehrere asymmetrische C-Atome enthalten, können als Enantiomere, als Diastereomere oder als Gemische davon, z.B. als Racemate, vorliegen.

Bevorzugte Verbindungen der Formel I sind

A) diejenigen, worin $A^2$ Cycloalkyl oder Cycloalkyl-alkyl und T CO ist, speziell die Verbindungen der Formel

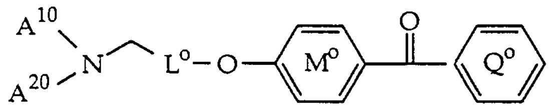

Ia

worin $A^{10}$ Alkyl, $A^{20}$ Cycloalkyl oder Cycloalkyl-alkyl, $L^o$ Alkylen oder Alkenylen mit insgesamt bis zu 11 C-Atomen und zumindest 4 bzw. 3 C-Atomen zwischen den zwei freien Valenzen oder Cycloalkylen-alkylen, $M^o$ gegebenenfalls halogeniertes 1,4-Phenylen und $Q^o$ durch Halogen oder CN substituiertes Phenyl, insbesondere worin $A^{10}$ Methyl, $A^{20}$ Cyclopropyl oder Cyclopropylmethyl, $L^o$ n-Pentylen, n-Propenylen oder Cyclopropylenmethylen, $M^o$ unsubstituiertes oder fluoriertes 1,4-Phenylen und $Q^o$ durch Br oder CN substituiertes Phenyl ist, speziell:

(4-Brom-phenyl)-[4-[6-(cyclopropyl-methyl-amino)-hexyloxy]-2-fluorphenyl] -methanon,
(E)-(4-Brom-phenyl)-[4-[4-(cyclopropyl-methyl-amino)-but-2-enyloxy]-phenyl] -methanon,
[6- [6-(Cyclopropyl-methyl-amino)-hexyloxy] -phenyl] -(4-brom-phenyl)-methanon,
(E)-4-[4-[4-(Cyclopropyl-methyl-amino)-but-2-enyloxy]-3-fluor-benzoyl]-benzonitril,
(4-Brom-phenyl)-[4-[6-(cyclopropylmethyl-methyl-amino)-hexyloxy]-phenyl] -methanon,
(1RS,2RS)-(4-Brom-phenyl)-[4-[2-[(cyclopropyl-methyl-amino)-methyl]-cyclopropylmethoxy] -phenyl] -methanon,
(1RS,2RS)-(4-Brom-phenyl)-[4-[2-[(cyclopropyl-methyl-amino)-methyl]-cyclopropylmethoxy] -3-fluor-phenyl] -methanon

sowie folgende Verbindungen:

1-[4-[6-(Cyclopropylmethyl-methyl-amino)-hexyloxy]-2-fluor-phenyl]-5-methyl-hex-4-en-1-on,
1-[4-[6-(Cyclopropyl-methyl-amino)-hexyloxy]-2-fluor-phenyl]-5-methyl-hex-4-en-1-on,
(E)-(4-Brom-phenyl)-[4-[4-(cyclopropylmethyl-methyl-amino)-but-2-enyloxy] -phenyl] -methanon,
(E)-4-[4-[4-(Cyclopropyl-methyl-amino)-but-2-enyloxy]-3-fluor-benzoyl]-benzamid,
(1RS,2RS)-4-[4-[2-[(Cyclopropyl-methyl-amino)-methyl]-cyclopropyl-methoxy]-3-fluor-benzoyl]-benzonitril,
(1RS,2RS)-(4-Brom-phenyl)- [4- [2- [(cyclopropylmethyl-methyl-amino)-methyl]-cyclopropylmethoxy]-phenyl]-methanon,
(1RS,2RS)-(4-Brom-phenyl)-[4-[2-[(cyclopropyl-methyl-amino)-methyl]-cyclopropylmethoxy]-2-fluor-phenyl]-methanon,
(1RS,2RS)-[4-[2-[(Allyl-cyclopropyl-amino)-methyl]-cyclopropylmethoxy]-phenyl]-(4-brom-phenyl)-methanon,
(1RS,2RS)-1-[4-[2-[(Cyclopropyl-methyl-amino)-methyl]-cyclopropyl-methoxy]-phenyl]-5-methyl-hexan-1-on,

B) diejenigen, worin T eine Gruppe CHOH, CHF, $C(R^4,R^5)$ oder $C=NOR^6$ ist und $R^4$, $R^5$ und $R^6$ die gleiche Bedeutung wie weiter oben angegeben, haben, insbesondere worin T eine Gruppe C(OH,Alkyl), C(OH, Alkenyl), $C=CH_2$ oder C=NO-Alkyl ist, speziell die Verbindungen der Formel

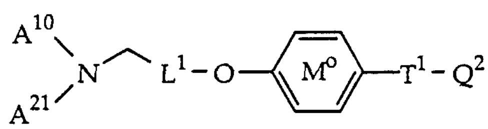

Ib

worin $A^{10}$ Alkyl, $A^{21}$ Alkenyl, $L^1$ Alkylen oder Alkenylen mit insgesamt bis zu 11 C-Atomen und zumindest 4 bzw. 3 C-Atomen zwischen den zwei freien Valenzen, $M^o$ gegebenenfalls halogeniertes 1,4-Phenylen, $T^1$ eine Gruppe C(OH, Alkyl), C(OH,Alkenyl), $C=CH_2$ oder C=NO-Alkyl und $Q^2$ Halophenyl oder Alkenyl mit 0 bis 3 Methylsubstituenten und insgesamt 6 bis 13 C-Atomen, insbesondere worin $A^{10}$ Methyl, $A^{21}$ Allyl, $L^1$ n-Pentylen oder n-Propenylen, $M^o$ unsubstituiertes oder fluoriertes 1,4-Phenylen und $Q^2$ Bromophenyl oder 4-Methylpent-3-enyl, insbesondere:

(E)-(RS)-1-[4-[4-(Allyl-methyl-amino)-but-2-enyloxy]-phenyl]-(4-bromphenyl)-ethanol,
(E)-(RS)-1-[4-[4-(Allyl-methyl-amino)-but-2-enyloxy]-phenyl]-1-(4-bromphenyl)-prop-2-en-1-ol,
(E)-Allyl-[4-[4-[1-(4-brom-phenyl)-vinyl]-phenoxy]-but-2-enyl]-methylamin,
(RS)-1-[4-[6-(Allyl-methyl-amino)-hexyloxy]-2-fluor-phenyl]-1-(4-bromphenyl)-ethanol,

(E)-(RS)-2- [4- [4-(Allyl-methyl-amino)-but-2-enyloxy] -phenyl] -6-methyl-hept-5-en-2-ol

sowie folgende Verbindungen:

(E)-(RS)-[4-[4-(Allyl-methyl-amino)-but-2-enyloxy]-phenyl]-(4-bromphenyl)-cyclopropyl-methanol,
(E)-(RS)-1-[4-[4-(Allyl-methyl-amino)-but-2-enyloxy]-phenyl]-1-(4-bromphenyl)-2,2,2-trifluor-ethanol,
(RS)-1-[4-[6-(Allyl-methyl-amino)-hexyloxy]-phenyl]-1-(4-brom-phenyl)-2,2,2-trifluor-ethanol,
(E)-Allyl-[4-[4-[1-(4-brom-phenyl)-cyclopropyl]-phenoxy]-but-2-enyl]-methyl-amin,
(E)-(R oder S)-1-[4-[4-(Allyl-methyl-amino)-but-2-enyloxy]-phenyl]-(4-brom-phenyl)-ethanol,
(E)-(S oder R)-1-[4-[4-(Allyl-methyl-amino)-but-2-enyloxy]-phenyl]-(4-brom-phenyl)-ethanol,
Allyl-[6-[4-[1-(4-brom-phenyl)-vinyl]-3-fluor-phenoxy]-hexyl]-methylamin,
(E)-(RS)-1-[4-[4-(Allyl-methyl-amino)-but-2-enyloxy]-3-fluor-phenyl]-(4-brom-phenyl)-prop-2-en-1-ol,
(RS)-1-[4-[(Allyl-methyl-amino)-methyl]-biphenyl-4-yl]-1-(4-bromophenyl)-ethanol,
(RS)-5-[6-(Allyl-methyl-amino)-hexyloxy]-2-[1-(4-brom-phenyl)-1-hydroxy-allyl] -phenol,
(RS)-1-[4-[6-(Allyl-methyl-amino)-hexyloxy]-2-amino-phenyl]-1-(4-bromphenyl)-prop-2-en-1-ol,
(RS)-Allyl-[4'-[(4-brom-phenyl)-fluor-methyl]-biphenyl-4-ylmethyl]-methyl-amin,
(E)- und/oder (Z)-[4-[(E)-4-(Allyl-methyl-amino)-but-2-enyloxy]-phenyl]-(4-brom-phenyl)-methanon-O-methyl-oxim,
(E)- und/oder (Z)-[4-[(E)-4-(Allyl-methyl-amino)-but-2-enyloxy]-phenyl]-(4-brom-phenyl)-methanon-oxim,
(E)- und/oder (Z)-[4-[(E)-4-Allyl-methyl-amino)-but-2-enyloxy]-phenyl-(4-brom-phenyl)-methanon-O-tert-butyl-oxim,
(E)- und/oder (Z)-[4-[(E)-4-(Allyl-methyl-amino)-but-2-enyloxy]-phenyl]-(4-brom-phenyl)-methanon-O-allyl-oxim,
(E)- und/oder (Z)-[4-[6-(Allyl-methyl-amino)-hexyloxy]-2-fluor-phenyl]-(4-brom-phenyl)-methanon-oxim,

C) diejenigen, worin M gegebenenfalls durch Alkyl, Halogen, $NH_2$, mono-oder di-alkyliertes Amino, Alkanoylamino, OH, Alkyl(O oder S) oder 1,2,4-Triazol-1-yl substituiertes 1,4-Phenylen, insbesondere worin M durch $NH_2$, mono-oder di-alkyliertes Amino, OH, S-Alkyl oder zwei Halogenatomen substituiertes 1,4-Phenylen ist, speziell die Verbindungen der Formel

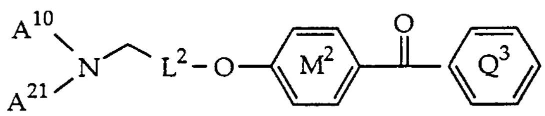

Ic

worin $A^{10}$ Alkyl, $A^{21}$ Alkenyl, $L^2$ Alkylen mit bis zu 11 C-Atomen und zumindest 4 C-Atomen zwischen den zwei freien Valenzen, $M^2$ durch $NH_2$, mono- oder dialkyliertes Amino, OH, S-Alkyl oder zwei Halogenatomen substituiertes 1,4-Phenylen und $Q^3$ halogeniertes Phenyl, insbesondere worin $A^{10}$ Methyl, $A^{21}$ Allyl, $L^2$ n-Pentylen, $M^2$ durch $NH_2$, $NHCH_3$, $N(CH_3)_2$, OH, $SCH_3$ oder durch zwei F-Atomen substituiertes 1,4-Phenylen und $Q^3$ Bromphenyl ist, insbesondere:

(E)-[4-[4-(Allyl-methyl-amino)-but-2-enyloxy]-2,5-difluor-phenyl]-(4-brom-phenyl)-methanon,
[4- [6-(Allyl-methyl-amino)-hexyloxy] -2-methylsulfanyl-phenyl] -(4-bromphenyl)-methanon,
[4-[6-(Allyl-methyl-amino)-hexyloxy]-2-methylamino-phenyl]-(4-bromphenyl)-methanon,
[4- [6-(Allyl-methyl-amino)-hexyloxy] -2-dimethylamino-phenyl] -(4-bromphenyl)-methanon,
[4-[6-(Allyl-methyl-amino)-hexyloxy]-2-hydroxy-phenyl]-(4-bromphenyl)-methanon,
[2-Amino-4-[6-(allyl-methyl-amino)-hexyloxy]-phenyl]-(4-brom-phenyl)-methanon

sowie folgende Verbindungen:

(E)-(4-Brom-phenyl)-[2,5-difluor-4-(4-dimethylamino-but-2-enyloxy]-methanon,
[4-[6-(Allyl-methyl-amino)-hexyloxy]-2,5-difluor-phenyl]-(4-bromphenyl)-methanon,
(E)-[4-[4-(Allyl-methyl-amino)-but-2-enyloxy]-2,5-difluor-phenyl]-(2,4-difluor-phenyl)-methanon,
(E)-[2,5-Difluor-4-(4-dimethylamino-but-2-enyloxy)-phenyl]-(2,4-difluorphenyl)-methanon,
[4-[6-(Allyl-methyl-amino)-hexyloxy]-2,5-difluor-phenyl]-(2,4-difluorphenyl)-methanon,
(2,4-Difluor-phenyl)-[4-(6-dimethylamino-hexyloxy)-2,5-difluorphenyl] methanon,

(E)-[4-[4-(Allyl-methyl-amino)-but-2-enyloxy]-2,3,5,6-tetrafluor-phenyl]-(4-brom-phenyl) -methanon,
(E)- [4- [4-(Allyl-methyl-amino)-but-2-enyloxy] -3-fluor-2-methyl-phenyl]-(4-brom-phenyl)-methanon,
(E)- [4- [4-(Allyl-methyl-amino)-but-2-enyloxy] -2-methylsulfanyl-phenyl]-(4-brom-phenyl)-methanon,
(E)-N-[11-[4- [4-(Allyl-methyl-amino)-but-2-enyloxy] -3-fluor-phenyl]-11-oxo-undecyl]-acetamid,
(E)-[4-(4-Allyl-methyl-amino-but-2-enyloxy)-2-hydroxy-phenyl]-(4-bromphenyl)-methanon,
(E)-1-[4-[4-(Allyl-methyl-amino)-but-2-enyloxy]-3-fluor-phenyl]-11-amino-undecan-1-on,
(E)-1-[4-[(E)-4-(Allyl-methyl-amino)-but-2-enyloxy]-3-fluor-phenyl]-2-[(E)-3,7-dimethyl-octa-2,6-dienyl]-5,9-dimethyl-deca-4,8-dien-1-on,
(E)-1-[4-[4-(Allyl-methyl-amino)-but-2-enyloxy]-3-fluor-phenyl]-5-methyl-2-(3-methyl-but-2-enyl)-hex-4-en-1-on,
(E)-(RS)-1-[4-[4-(Allyl-methyl-amino)-but-2-enyloxy]-3-fluor-phenyl]-3-hydroxy-5-methyl-hex-4-en-1-on,
(E)-(RS)-1- [4- [4-(Allyl-methyl-amino)-but-2-enyloxy] -2-fluor-phenyl] -3-hydroxy-5-methyl-hex-4-en-1-on,
(E)-(RS)-1- [4- [4-(Allyl-methyl-amino)-but-2-enyloxy] -phenyl] -3-hydroxy-5-methyl-hex-4-en-1-on,
(E)-(RS)-1-[4-[(E)-4-(Allyl-methyl-amino)-but-2-enyloxy]-3-fluor-phenyl]-3-hydroxy-5,9-dimethyl-deca-4,8-dien-1-on,
(E)-(RS)-13-(Allyl-methyl-amino)-1-[4-[4-(allyl-methyl-amino)-but-2-enyloxy]-3-fluor-phenyl]-3-hydroxy-tridecan-1-on,
(E)-1-[4-[(E)-4-(Allyl-methyl-amino)-but-2-enyloxy]-3-fluor-phenyl]-5-methyl-hexa-2,4-dien-1-on,
(E)-13-(Allyl-methyl-amino)-1-[4-[(E)-4-(allyl-methyl-amino)-but-2-enyloxy]-3-fluor-phenyl]-tridec-2-en-1-on,
(E)-1- [4- [(E)-4-(Allyl-methyl-amino)-but-2-enyloxy] -2-fluor-phenyl] -5-methyl-hexa-2,4-dien-1-on,
(E)-1-[4-[(E)-4-(Allyl-methyl-amino)-but-2-enyloxy]-phenyl]-5-methylhexa-2,4-dien-1-on,
(2E,4E)-1-[4-[(E)-4-(Allyl-methyl-amino)-but-2-enyloxy]-3-fluor-phenyl]-5,9-dimethyl-deca-2,4,8-trien-1-on,
[4-[6-(Allyl-methyl-amino)-hexyloxy]-2-1H-[1,2,4]triazol-1-yl-phenyl]-(4-brom-phenyl)-methanon,
1-[4-[6-(Allyl-methyl-amino)-hexyloxy]-2-methylamino-pheny]-4-methylhex-5-en-1-on,
(E)-1-[4-[4-(Allyl-methyl-amino)-but-2-enyloxy]-2-methylsulfanylphenyl]-5-methyl-hex-4-en-1-on,
N- [5- [6-(Allyl-methyl-amino)-hexyloxy] -2-(4-brom-benzoyl)-phenyl]-methansulfonamid,
(4-Brom-phenyl)-(4'-dimethylaminomethyl-3-hydroxy-biphenyl-4-yl)-methanon,
N-[5-[6-(Allyl-m ethyl-amino)-hexyloxy] -2-(4-brom-benzoyl)-phenyl]-formamid,

D) diejenigen, worin L eine über NH oder N-Alkanoyl an M gebundene Alkylen- oder Alkenylengruppe mit insgesamt bis zu 11 C-Atomen und zumindest 4 bzw. 3 C-Atomen zwischen den zwei freien Valenzen, speziell die Verbindungen der Formel

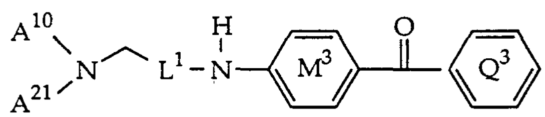
Id

worin $A^{10}$ Alkyl, $A^{21}$ Alkenyl, $L^1$ Alkylen oder Alkenylen mit insgesamt bis zu 11 C-Atomen und zumindest 4 bzw. 3 C-Atomen zwischen den zwei freien Valenzen, $M^3$ halogeniertes 1,4-Phenylen und $Q^3$ halogeniertes Phenyl, insbesondere worin $A^{10}$ Methyl, $A^{21}$ Allyl, $L^1$ n-Pentylen oder n-Propenylen, $M^3$ Fluorphenylen und $Q^3$ Bromphenyl, insbesondere:

(E)-[4-[4-(Allyl-methyl-amino)-but-2-enylamino]-3-fluor-phenyl]-(4-brom-phenyl)-methanon,
[4- [6-(Allyl-methyl-amino)-hexylamino] -3-fluor-phenyl] -(4-bromphenyl)-methanon

sowie folgende Verbindungen:

(E)-N-[4-(Allyl-methyl-amino)-but-2-enyl]-N-[4-(4-brom-benzoyl)-2-fluorphenyl]-acetamid,
N- [6-(Allyl-methyl-amino)-hexyl] -N- [4-(4-brom-benzoyl)-2-fluor-phenyl]-acetamid,

E) diejenigen, worin M eine Gruppe der Formel

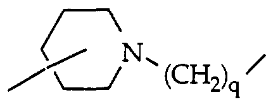

($M^1$)

und q 1 oder 0, insbesondere worin M zusammen mit T die Piperidin-1-yl-sulfonylgruppe bildet, speziell die Verbindungen der Formel

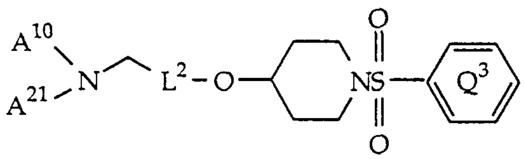

Ie

worin $A^{10}$ Alkyl, $A^{21}$ Alkenyl, $L^2$ Alkylen mit bis zu 11 C-Atomen und zumindest 4 C-Atomen zwischen den zwei freien Valenzen und $Q^3$ halogeniertes Phenyl, insbesondere worin $A^{10}$ Methyl, $A^{21}$ Allyl, $L^2$ n-Pentylen und $Q^3$ Bromphenyl ist, speziell

Allyl-[6-[1-(4-brom-phenylsulfonyl)-piperidin-4-yloxy]-hexyl]-methylamin

und die Verbindungen:

[4-[6-(Allyl-methyl-amino)-hexyloxy]-piperidin-1-yl]-1-(4-brom-phenyl) methanon,
2-[4-[6-(Allyl-methyl-amino)-hexyloxy]-piperidin-1-yl]-1-(4-bromphenyl)-ethanon,

F) diejenigen, worin $A^1$ Alkyl und $A^2$ OH oder gegebenenfalls durch eine Gruppe $R^1$, $CONH_2$ oder CN substituiertes Alkyl ist oder

$A^1$ zusammen mit $A^2$ oder mit $A^3$ eine gegebenenfalls durch $R^1$ substituiertes Alkylen-, Alkenylen- oder Alkadienylengruppe $A^1$-$A^2$ bzw. $A^1$-$A^3$ mit bis zu 5 C-Atomen bildet, wobei in einer Gruppe $A^1$-$A^2$ oder $A^1$-$A^3$ ein C-Atom durch ein N-Atom ersetzt sein kann und
$R^1$ an einem gesättigten C-Atom von $A^2$, $A^1$-$A^2$ oder $A^1$-$A^3$ gebundenes OH, Oxo, Alkyl(O oder S) oder Dialkylamino ist,
wobei ein durch $R^1$ substituiertes C-Atom bzw. ein in $A^2$, $A^1$-$A^2$ oder $A^1$-$A^3$ enthaltenes ungesättigtes C-Atom in einer anderen als der $\alpha$-Stellung zu $N(A^1A^2)$ gebunden sein sollen,

speziell die Verbindungen, worin $A^1$ zusammen mit $A^2$ durch OH substituiertes Alkylen mit bis zu 5 C-Atomen ist, sowie die Verbindungen der Formel

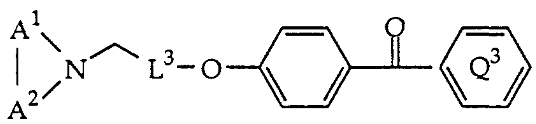

If

worin $A^1$ zusammen mit $A^2$ eine durch OH substituierte Alkylengruppe mit bis zu 5 C-Atomen, $L^3$ Alkenylen mit insgesamt bis zu 11 C-Atomen und zumindest 3 C-Atomen zwischen den zwei freien Valenzen und $Q^3$ halogeniertes Phenyl, insbesondere worin $A^1$ zusammen mit $A^2$ 4-Hydroxy-piperidin-1-yl, $L^3$ n-Propenylen und $Q^3$ Bromphenyl ist, speziell:

(E)-(4-Brom-phenyl)-[4-[4-(4-hydroxy-piperidin-1-yl)-but-2-enyloxy]-phenyl]-methanon

sowie folgende Verbindungen:

Cyclohexyl-p-[[(E)-4-(dimethylamino)-2-butenyl]oxy]phenyl-keton,
(E)-[[4-[4-(4-Brom-benzoyl)-phenoxy]-but-2-enyl]-methyl-amino]-acetonitril,
(E)-3-[[4-[4-(4-Brom-benzoyl)-phenoxy]-but-2-enyl]-methyl-amino]-propionitril,
(E)-(4-Brom-phenyl)-[4-[4-(4-dimethylamino-piperidin-1-yl)-but-2-enyloxy] -phenyl] -methanon,

(4-Brom-phenyl)-[4-[6-(hydroxy-methyl-amino)-hexyloxy]-phenyl]-methanon,
(E)-(4-Brom-phenyl)-[4-[4-(hydroxy-methyl-amino)-but-2-enyloxy]-phenyl]-methanon,
(E)-(4-Brom-phenyl)-[4-[4-[(2-methoxy-ethyl)-methyl-amino]-but-2-enyloxy]-phenyl]-methanon,
(E)-(4-Brom-phenyl)-[4-[4-[methyl-(2-methylsulfanyl-ethyl)-amino]-but-2-enyloxy] -phenyl] -methanon,
(E)-(4-Brom-phenyl)-[4-(4-imidazol-1-yl-but-2-enyloxy)-phenyl]-methanon,
(4-Brom-phenyl)-[4-(6-imidazol-1-yl-hexyloxy)-phenyl]-methanon,
(4-Brom-phenyl)-[4-[6-[(3-hydroxy-propyl)-methyl-amino]-hexyloxy]-phenyl]-methanon,
1-[[6-[4-(4-Brom-benzoyl)-phenoxy]-hexyl]-methyl-amino]-propan-2-on,
(E)-2-[[4-[4-(4-Brom-benzoyl)-phenoxy]-but-2-enyl]-methyl-amino]-acetamid,
(±)(4-Brom-phenyl)-[4'-(1-methylpyrrolidin-2-yl)-biphenyl-4-yl]-methanon,

G) diejenigen, worin p = 0 und L an T gebundenes $C_{6-11}$-Alkenylen oder $C_{6-11}$-Alkadienylen ist, speziell die Verbindungen der Formel

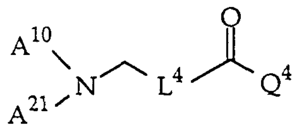
Ig

worin $A^{10}$ Alkyl, $A^{21}$ Alkenyl, $L^4$ $C_{6-11}$-Alkadienylen und $Q^4$ eine Alkenylgruppe mit 0 bis 3 Methylsubstituenten und insgesamt 6 bis 13 C-Atomen, insbesondere worin $A^{10}$ Methyl, $A^{21}$ Allyl, $L^4$ Dimethyloctadienylen und $Q^4$ 4-Methyl-3-pentenyl ist, speziell:
(9E,13E)-15-(Allyl-methyl-amino)-2,9,13-trimethyl-pentadeca-2,9,13-trien-6-on
sowie folgende Verbindungen:

(4E,8E)-10-(Allyl-methyl-amino)-1-(4-brom-phenyl)-4,8-dimethyl-deca-4,8-dien-1-on,
(4E,8E)-1-(4-Brom-phenyl)-10-dimethylamino-4,8-dimethyl-deca-4,8-dien-1-on,
(7E,11E)-13-(Allyl-methyl-amino)-2,7,11-trimethyl-trideca-2,7,11-trien-6-on,
(7E,11E)- und (7Z,11E)-13-(Allyl-methyl-amino)-2,7,11-trimethyl-trideca-2,7,11-trien-6-on,
(2E,6E)-8-(Allyl-methyl-amino)-1-(4-brom-phenyl)-2,6-dimethyl-octa-2,6-dien-1-on,
(7E,11E)-13-(Allyl-methyl-amino)-7,11-dimethyl-trideca-1,7,11-trien-6-on,
(2E,6E)-(RS)-8-(Allyl-methyl-amino)-1-(4-brom-phenyl)-2,6-dimethyl-octa-2,6-dien-1-ol,
(E)-(RS)-8-(Allyl-methyl-amino)-1-(4-brom-phenyl)-2,6-dimethyl-oct-6-en-1-on,
(2E,6E)-(RS)-10-(Allyl-methyl-amino)-1-(4-brom-phenyl)-3,7-dimethyl-deca-2,6-dien-1-ol,
(2E,6E)-(RS)-8-(Allyl-methyl-amino)-1-(4-brom-phenyl)-3,7-dimethyl-octa-2,6-dien-1-ol,
(2E,6E)-10-(Allyl-methyl-amino)-1-(4-brom-phenyl)-3,7-dimethyl-deca-2,6-dien-1-on,
(2E,6E)-8-(Allyl-methyl-amino)-1-(4-brom-phenyl)-3,7-dimethyl-octa-2,6-dien-1-on,

H) diejenigen, worin M Thienylen oder Pyridylen, insbesondere die folgenden:

(E)-1-[6-[6-(Allyl-methyl-amino)-hexyloxy]-pyridin-3-yl]-5-methyl-hexa-2,4-dien-1-on,
6-[6-(Allyl-methyl-amino)-hexyloxy]-pyridin-3-yl]-(4-brom-phenyl)-methanon,
(E)- [6- [4-(Allyl-methyl-amino)-but-2-enyloxy] -pyridin-3-yl] -(4-bromphenyl)-methanon,
[5-[6-(Allyl-methyl-amino)-hexyloxy]-pyridin-2-yl]-(4-brom-phenyl)-methanon,
5-(4-[(Allyl-methylamino)-methyl]-phenyl)-thiophen-2-yl)-(4-bromphenyl)-methanon,
5-(4-[Dimethylamino)-methyl]-phenyl)-thiophen-2-yl)-(4-bromophenyl)-methanon,
5-(4-[(Allyl-methylamino)-methyl]-phenyl)-thiophen-2-yl)-(4-(2,4-difluorophenyl))-methanon,
(2-Dimethylamino-4-fluor-phenyl)-[5-(4-dimethylaminomethyl-phenyl)-thiophen-2-yl] -methanon,

I) diejenigen, worin L über ein O-Atom an M gebundenes Cycloalkylen-alkylen ist, speziell

(1RS,2RS)-(4-Brom-phenyl)-[4-[2-[(ethyl-methyl-amino)-methyl]-cyclopropylmethoxy]-phenyl]-methanon,
(1RS,2RS)-[4-[2-[(Allyl-methyl-amino)-methyl]-cyclopropylmethoxy]-phenyl]-(4-brom-phenyl)-methanon.

Die Erfindung betrifft ferner ein Verfahren zur Herstellung der Verbindungen der Formel I. Dieses Verfahren ist dadurch gekennzeichnet, dass man

a) ein Bromid der Formel

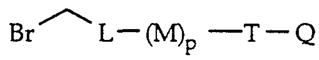 II

mit einem Amin $HN(A^1,A^2)$ umsetzt,

b) ein Amin der Formel

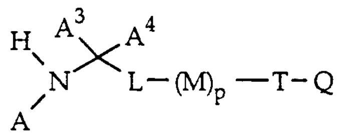 III

worin A die gleiche Bedeutung wie $A^2$ hat, methyliert,

c) ein Amin der Formel III, worin A die gleiche Bedeutung wie $A^1$ hat, mit einem Halogenid der Formel Hal-$A^o$ umsetzt, worin Hal Halogen und $A^o$ Cycloalkyl-alkyl oder durch eine Gruppe $R^1$, $CONH_2$ oder CN substituiertes Alkyl oder Alkenyl ist,

d) ein Aethanon der Formel

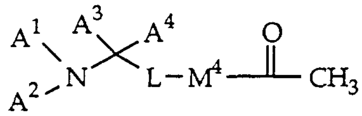 IV

worin $M^4$ für 1,4-Phenylen, das wie weiter oben für eine 1,4-Phenylengruppe M angegeben substituiert sein kann, oder für Thienylen oder Pyridylen steht,
mit einem Halogenid der Formel

Hal-$R^7$

zu einem Keton der Formel I umsetzt, worin T zusammen mit Q eine Gruppe $C(O)C(R^7)_2$ ist und $R^7$ die gleiche Bedeutung wie in der Formel I hat,

e) ein Aethanon der Formel IV mit einem Aldehyd der Formel

HC(O)Q"

worin Q" eine geradkettige Alkyl-, Alkenyl- oder Alkadienylgruppe mit 0 bis 3 Methylsubstituenten und insgesamt 4 bis 11 C-Atomen ist,
umsetzt,

f) ein β-Hydroxyketon der Formel

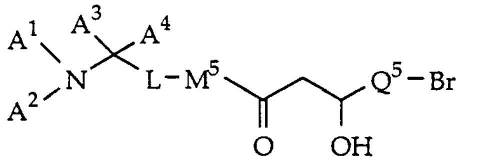

V

worin $M^5$ für 1,4-Phenylen, das wie weiter oben für eine 1,4-Phenylengruppe M angegeben substituiert sein kann, steht und $Q^5$ eine der obigen einbindigen Gruppe Q" entsprechende zweibindige Gruppe ist, mit einem Amin $HN(R^9,R^{10})$ umsetzt,

g) ein Aminoalkohol der Formel

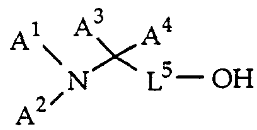

VI

worin $L^5$ Alkylen oder Alkenylen mit insgesamt bis zu 11 C-Atomen und zumindest 4 bzw. 3 C-Atomen zwischen den zwei freien Valenzen oder Cycloalkylen-alkylen ist, mit einer Verbindung der Formel

$$HO\text{-}M^6\text{-}T\text{-}Q$$

umsetzt,

worin $M^6$ für 1,4-Phenylen, das wie in Anspruch 1 angegeben substituiert sein kann, oder für Thienylen steht,

h) ein Aminoalkohol der Formel VI mit einem Chlorid der Formel

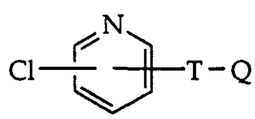

VII

umsetzt,

i) ein Säureadditionssalz eines Amins der Formel

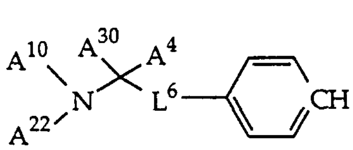

VIII

worin $A^{10}$ Alkyl, $A^{22}$ Cycloalkyl, Cycloalkyl-alkyl oder eine gegebenenfalls durch eine Gruppe $R^{10}$ oder CN substituierte Alkylgruppe,

$A^{30}$ und $A^4$ Wasserstoff oder Alkyl sind oder
$A^{10}$ zusammen mit $A^{22}$ oder mit $A^{30}$ eine gegebenenfalls durch
$R^{11}$ substituierte Alkylengruppe bildet, wobei in einer solchen Alkylengruppe ein C-Atom durch N-Alkyl ersetzt sein kann,
$R^{11}$ Oxo, Alkyl (O oder S) oder Dialkylamino und
$L^6$ Phenylen oder direkt oder über O oder N-Alkyl an den Phenylring gebundenes Alkylen mit insgesamt bis

zu 11 C-Atomen und zumindest 4 C-Atomen zwischen den zwei freien Valenzen ist,

mit einem Säurechlorid der Formel

$$ClC(O)\text{-}Q^6$$

umsetzt,

worin $Q^6$ Cycloalkyl, $C(R^{70},R^{80})$, durch einen oder mehreren Substituenten aus der Gruppe von Alkyl, Halogen, Dialkylamino, CN, $NO_2$, $CF_3$, 1,2,4-Triazol-1-yl und Tetrazol-1-yl substituiertes Phenyl oder eine geradkettige Alkylgruppe mit 0 bis 3 Methylsubstituenten und insgesamt 6 bis 13 C-Atomen ist und $R^{70}$ und $R^{80}$ für $C_{5\text{-}11}$-Alkyl stehen,

j) ein Diamin der Formel

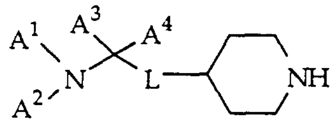

IX

mit einem Halogenid der Formel

$$ClS(O)_2\text{-}Q,\ ClC(O)\text{-}Q \text{ oder } BrCH_2C(O)\text{-}Q$$

umsetzt,

k) ein Aldehyd der Formel

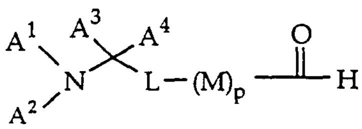

X

mit einem die Gruppe Q einführenden Mittel umsetzt, wobei Q Cycloalkyl, $C(R^7,R^8)$, durch einen oder mehreren Substituenten aus der Gruppe von Alkyl, Halogen, $N(R^{90},R^{100})$, CN, $NO_2$, $CF_3$, 1,2,4-Triazol-1-yl und Tetrazol-1-yl substituiertes Phenyl oder eine geradkettige Alkyl-, Alkenyl-, Alkadienyl-oder Alkatrienylgruppe $Q^{10}$ mit 0 bis 3 Methylsubstituenten und insgesamt 6 bis 13 C-Atomen ist, wobei eine solche Gruppe $Q^{10}$ durch $N(R^{90},R^{100})$ substituiert sein kann und $R^{90}$ und $R^{100}$ Alkyl oder Alkenyl ist,

l) gewünschtenfalls eine in einer Verbindung der Formel I enthaltene reaktionsfähige Gruppe funktionell abwandelt und

m) gewünschtenfalls ein Amin der Formel I in ein physiologisch verträgliches Säureadditionssalz oder ein Säureadditionssalz einer Verbindung der Formel I in das Amin der Formel I überführt.

Dieses Verfahren kann in an sich bekannter Weise durchgeführt werden. So lässt sich die Umsetzung a) eines Bromids II mit einem Amin $HN(A^1,A^2)$

1. in einem Lösungsmittel wie einem Alkohol, z.B. Aethanol, oder in Aceton, in Gegenwart einer Base, z.B. Kaliumcarbonat, bei erhöhter Temperatur,

2. in Dimethylacetamid (DMA) bei Raumtemperatur oder unter Abkühlung oder

3. in Gegenwart von NaH in einem Lösungsmittel, wie DMF, unter Erwärmen bewerkstelligen.

Die Methylierung eines Amins III, worin A die gleiche Bedeutung wie $A^2$ in der Formel I hat, lässt sich in Gegenwart von $NaHPO_4$ in einem Lösungsmittel, wie einem Aether, z.B. Dioxan, mittels Formaldehyd unter Erwärmen durchführen.

Die Umsetzung eines Amins III, worin A die gleiche Bedeutung wie $A^1$ in der Formel I hat, mit einem Cycloalkyl-alkylhalogenid Hal-$A^o$, z.B. einem Bromid, kann man in Gegenwart von einer Base, wie Diisopropylethylamin in einem Lösungsmittel, wie DMA, unter Erwärmen bewerkstelligen.

Die Umsetzung eines Aethanons IV mit einem Halogenid Hal-$R^7$ und/oder Hal-$R^8$ führt zum entsprechenden Aminoketon I, worin T für CO und Q für die Gruppe C($R^7$,$R^7$), C($R^8$,$R^8$) und/oder C($R^7$,$R^8$) steht. Man kann das Aethanon IV zunächst mit einer Lösung von Lithium-hexamethyl-disilazid (hergestellt aus Hexamethyldisilazan und Butyllithium) in THF und dann mit einer Lösung des Halogenids, z.B. des Bromids, der Formel Hal-$R^7$ und/oder Hal-$R^8$ in einem Lösungsmittel, wie einem Aether, z.B. THF, bei niedriger Temperatur umsetzen.

Je nachdem $M^4$ in einem Aethanon IV für Thienylen oder gegebenenfalls substituiertes Phenylen oder für Pyridylen steht, führt die Umsetzung e) des Aethanons IV mit einem Aldehyd HC(O)Q" zu einem Keton I, worin T-Q für C(O)CH=CH-Q" steht, oder zu einem β-Hydroxyketon I, worin T-Q für $C(O)CH_2CH(OH)Q"$ steht. Das Aethanon IV kann man zunächst mit einer Lösung von Lithium-diisopropylamid (hergestellt aus Diisopropylamin und Butyl-lithium) in THF und dann mit einer Lösung des Aldehyds HC(O)Q" in THF bei niedriger Temperatur umsetzen.

Die Verfahrensvariante f) führt zu einem β-Hydroxyketon I, worin T-Q eine Gruppe:

$$C(O)CH_2CH(OH)Q^5\text{-}N(R^9,R^{10})$$

ist. Zur Durchführung dieser Variante kann man eine Lösung eines Bromids V in DMA unter Abkühlung mit einem Amin HN($R^9$,$R^{10}$) umsetzen.

Die Umsetzung g) eines Aminoalkohols VI mit einer Verbindung der Formel HO-$M^6$-T-Q führt zu einem Aminoäther I, worin L-M eine Gruppe $L^5$-O-$M^6$ ist, in der $L^5$ und $M^6$ wie weiter oben angegeben definiert sind. Sie kann dadurch bewerkstelligt werden, dass man Triphenylphosphin, die Verbindung HO-$M^6$-T-Q und das Aminoalkohol VI in einem Lösungsmittel, wie einem Aether, z.B. THF, mit Diethylazodicarboxylat behandelt.

Die Umsetzung h) eines Aminoalkohols VI mit einem Chlorid VII führt zu einem Aether I, worin L-M eine Gruppe $L^5$-O-Pyridylen ist. Man kann sie in Gegenwart einer Base, wie KOH und $K_2CO_3$ in Gegenwart eines Kronenethers, wie die Dicyclohexano-[18]-krone-6, in einem Lösungsmittel, wie Toluol, unter Erhitzen durchführen.

Die Umsetzung i) eines Säureadditionssalzes des Amins VIII mit einem Säurechlorid ClC(O)-$Q^6$ führt zum entsprechenden Aminoketon I, worin T-Q für C(O)-$Q^6$ steht. Sie kann in Gegenwart von Aluminiumchlorid in Schwefelkohlenstoff unter Erhitzen bewerkstelligt werden.

Die Umsetzung j) eines Diamins IX mit einem Halogenid $ClS(O)_2$-Q, ClC(O)-Q oder $BrCH_2C(O)$-Q führt zum entsprechenden Amin I, worin T-Q für $S(O)_2$-Q, C(O)-Q bzw. $CH_2C(O)$-Q steht. Man kann sie in einem Lösungsmittel, wie Methylenchlorid, in Gegenwart von Di-isopropyl-äthylamin (Hünig-Base) durchführen.

Die Verfahrensvariante k) kann eine Grignard-Reaktion zwischen einem Aldehyd X und einem Halogenid, wie Q-MgBr sein. Falls Q gegebenenfalls substituiertes Phenyl ist, kann man zunächst ein Halogenid, wie Q-Br, in THF mit Butyl-lithium in Hexan und die entstandene Verbindung Li-Q mit einem Aldehyd X bei tiefer Temperatur, wie etwa -78°C, zum entsprechenden Keton I umsetzen.

Als funktionelle Abwandlungen von in einer Verbindung I enthaltenen reaktionsfähigen Gruppen können folgende genannt werden:

a) Die Umwandlung einer Cyanogruppe, die als Substituent einer Alkylgruppe $A^2$ und/oder einer Phenylgruppe Q vorliegt, in die Carbamoylgruppe kann man in Gegenwart von Kaliumcarbonat in DMSO bei etwa 0°C mittels einer Wasserstoffperoxidlösung durchführen.

b) Die Hydrolyse einer Alkanoylaminogruppe, die als Substituent einer Gruppe Q' vorliegt, zur Aminogruppe kann mittels Salzsäure in Aethanol erfolgen.

c) Die Dehydratisierung eines β-Hydroxyketons I, worin T für C(O) steht und Q eine in β-Stellung zu C(O) hydroxylierte Gruppe Q' ist, zum entsprechenden Keton I, worin Q eine in α-Stellung zu C(O) ungesättigte Gruppe Q' ist, kann man in Toluol mittels p-Toluolsulfonsäure durchführen.

d) Ein Amid I, worin L über N(Alkanoyl) an M gebunden ist, kann mittels einer Lösung von KOH in Aethanol ins entsprechende Amin I, worin L über NH an M gebunden ist, übergeführt werden.

e) In einem Keton I, worin p = 0, die Gruppe T-Q substituiertes Benzoyl und L Alkenylen oder Alkadienylen ist, kann eine in L enthaltene Gruppe CH=CH, die in α-Stellung zur Carbonylgruppe T vorliegt, selektiv zu $CH_2CH_2$ hydriert werden. Man kann die Hydrierung in Benzol mit einem Phasentransfer-Katalysator, wie Tricaprylmethylammoniumchlorid, in Gegenwart von einer wässrigen Lösung von Natriumhydrogencarbonat und Natriumdithionit durchführen.

f) In einem Benzophenon I, worin Q substituiertes Phenyl ist und die Phenylengruppe M in o-Stellung zur Carbonylgruppe T durch Fluor substituiert ist, kann man dieses F-Atom

1) durch Reaktion mit Methoxybenzylamin in Gegenwart einer Base, wie Kaliumcarbonat, in Toluol und dann Umsetzung mit Trifluoressigsäure in die Aminogruppe,

2) durch Reaktion in DMA mit einem geeigneten Amin in Aethanol in eine alkylierte oder alkenylierte Aminogruppe oder in eine 1,2,4-Triazol-1-yl-oder Tetrazol-1-ylgruppe oder

3) durch Reaktion mit einem Natrium-alkanolat oder einem Natriumthioalkanolat in Methanol bzw. in THF in die entsprechende Alkoxygruppe bzw. Alkylthiogruppe umwandeln.

g) Durch Aetherspaltung mittels wässriger Essigsäure/HBr-Lösung lässt sich ein Alkoxysubstituent in der Gruppe M in die Hydroxygruppe umwandeln.

h) In einer Verbindung I, worin M Aminophenylen ist, kann man die Aminogruppe durch Reaktion in Methylenchlorid mit einem Alkylsulfonylchlorid in die Alkylsulfonylaminogruppe umwandeln. Die Aminogruppe lässt mittels Ameisensäure und Formamid in die Formylaminogruppe umwandeln.

i) In an sich bekannter Weise lässt sich ein Keton I, worin T Carbonyl ist, in den entsprechenden Alkohol überführen, worin T eine Gruppe [Alkyl, Alkenyl, Alkinyl oder Cycloalkyl]-C(OH) ist. So kann man zur Umwandlung der Carbonylgruppe in die Gruppe $C(CH_3)OH$ das Keton I bei etwa -78°C mit $LiCH_3/CeCl_3$ in THF und zur Umwandlung der Carbonylgruppe in eine Gruppe Alkenyl-C(OH) das Keton I mit einer Lösung eines Alkenylmagnesiumhalogenids bei etwa 0°C in THF/Aether umsetzen.

j) Aus einem Säureadditionssalz eines Ketons I, worin T Carbonyl ist, kann man ein Oxim I, worin T für $C=N(OR^6)$ steht, durch Umsetzung mit $H_2N(OR_6)$ in Gegenwart von Natriumacetat in Aethanol unter Erhitzen erhalten.

k) Ein Alkohol I, worin T die Gruppe CH(OH) ist, kann in Methylenchlorid bei etwa -78°C mittels Diäthylaminoschwefeltrifluorid zum Fluorid I, worin T die Gruppe CHF ist, fluoriert oder in Gegenwart von Natriumcarbonat mittels Mangan(IV)-oxid zum Keton, worin T C(O) ist, oxydiert werden.

Die im obigen Verfahren eingesetzten Ausgangsmaterialien II bis X und die zu dessen Herstellung notwendigen Edukten sind bekannt oder können in Analogie zu strukturell nahverwandten Verbindungen bzw. wie in den nachstehenden Beispielen beschrieben in an sich bekannter Weise hergestellt werden.

So wird ein Bromid II, worin T für C(O) steht und L über ein O-Atom an eine gegebenenfalls substituierte Phenylgruppe M gebunden ist, ausgehend von einem Aether $H_3C$-O-M und einem Säurechlorid ClC(O)-Q, via dem Aether $H_3C$-O-M-C(O)-Q und dem entsprechenden Phenol HO-M-C(O)-Q und Reaktion dieses Phenols mit einem Dibromid $BrCH_2$-L-Br hergestellt. Analog lassen sich Bromide II, worin T für C(OH,Alkyl), $C=CH_2$ oder $C(CH_2CH_2)$ steht via den entsprechenden Phenolen HO-M-T-Q herstellen.

Ein Bromid II, worin T für C(O) steht und L über eine Gruppe N(Alkanoyl) an eine gegebenenfalls substituierte Phenylgruppe M gebunden ist, lässt sich ausgehend von einem Bromid der Formel Alkanoyl-NH-M-Br und von einer Verbindung der Formel $N(CH_3, OCH_3)C(O)$-Q, via der Verbindung der Formel Alkanoyl-NH-M-C(O)-Q und Reaktion dieser Verbindung mit einem Dibromid $BrCH_2$-L-Br herstellen.

Aus Bromtoluol und Bromthiophen erhält man via Tolyl-Thiophen und Tolyl-Thienylen-C(O)-Q, eine Verbindung II, worin L an eine Thienylengruppe M gebundenes Phenylen ist.

Generell lässt sich ein Bromid II aus dem entsprechenden Tetrahydropyranyläther durch Reaktion in Methylenchlorid bei etwa -50°C mit Triphenylphosphindibromid unter Abkühlung, vorzugsweise auf -50 bis 0°C, herstellen.

Zur Herstellung eines Ausgangsamins III, worin $A^3$ und $A^4$ für H stehen, kann man ein entsprechendes Bromid II mit einem Trifluoracetamid $F_3C$-C(O)-NH-A in $F_3C$-C(O)-N(A)-$CH_2$-L-$(M)_p$-T-Q überführen und aus letzterem die Trifluoracetylgruppe hydrolytisch abspalten.

Ein Ausgangsamin III für die Verfahrensvariante c) erhält man ausgehend von dem entsprechenden Bromid

II, via entsprechendem Azid und den Verbindungen $F_3C$-C(O)-NH-$CH_2$-L-$(M)_p$-T-Q und $F_3C$-C(O)-N(A)-$CH_2$-L-$(M)_p$-T-Q.

Aethanone IV, worin $A^3$ und $A^4$ für H stehen, erhält man durch Reaktion eines Bromids $BrCH_2$-L-$M^4$-C(O)-$CH_3$ mit einem Amin $(A^1,A^2)NH$ in DMA.

Ein β-Hydroxyketon V lässt sich ausgehend von dem entsprechenden Aethanon IV und einem Aldehyd HC(O)-$Q^5$-Br herstellen.

Ein Aminoalkohol VI, worin $A^3$ und $A^4$ für H stehen und $L^5$ für Cycloalkylen-alkylen steht, kann man aus einem Diester der Formel Alkyl-O-C(O)-Cycloalkylen-C(O)O-alkyl, via $A^2$-NH-C(O)-Cycloalkylen-C(O)O-alkyl und via $(A^1,A^2)$-N-C(O)-Cycloalkylen-COO-alkyl und Reduktion dieses Amidoesters zum Aminoalkohol VI: $(A^1,A^2)$N-$CH_2$-Cycloalkylen-$CH_2OH$ erhalten.

Ein Chlorid VII, worin T für C(O) steht, erhält man aus der entsprechenden Chlorpyridincarbonsäure, via Chlor-N-methoxy-N-methylpyridincarboxamid.

Zur Herstellung eines Amins VIII, worin $A^{10}$ zusammen mit $A^{22}$ eine Alkylengruppe bildet, kann man von einem Bromid Br-$L^6$-$C_6H_5$ ausgehen. Dieses kann z.B. mit 5-Methoxy-2H-3,4-dihydropyrrol in das in 5-Stellung durch -$L^6$-$C_6H_5$ substituierte 2H-3,4-Dihydropyrrol überführt, letzteres zu dem in 2-Stellung entsprechend substituierten Pyrrolidin hydriert und dieses zur Verbindung VIII, worin $(A^{10},A^{22})NC(A^{30},A^4)$ eine N-Methyl-2-pyrrolidinyl ist, methyliert werden.

Ein Diamin der Formel IX, worin $A^3$ und $A^4$ für H stehen und L über O an den Piperidinring gebundenes Alkenylen ist, erhält man aus dem 4-Hydroxy-piperidin-1-carbonsäure-tert-butylester, via dem in 4-Stellung durch eine Gruppe Br-Alkylen-O- substituierten Piperidin.

Ein Aldehyd X, worin $A^3$ und $A^4$ für H stehen, erhält man ausgehend von einem Aldehyd H-C(O)-L-$(M)_p$-$CH_2$-O-THP, via dem Amid der Formel $(A^1,A^2)$NC(O)-L-$(M)_p$-$CH_2$-O-THP und dem Aminoalkohol der Formel $(A^1,A^2)NCH_2$-L-$(M)_p$-$CH_2OH$ durch Oxydation des letzteren.

In den nachfolgenden Beispielen A bis G ist die Herstellung von einigen der weiter oben erwähnten Ausgangsmaterialien und Zwischenprodukten beschrieben.

A) Ausgangsverbindungen der Formel HO-M-C(O)-Q

Aa) Eine Lösung von 5.6 ml 2-Fluoranisol in 60 ml absolutem THF wird auf -78 °C gekühlt und innert 15 Min mit 31.3 ml 1.6 M Butyllithium in Hexan versetzt. Nach 15 Min werden 8.7 g 1,1,4,7,7 -Pentamethyldiethylentriamin und, nach weiteren 2 Std bei - 78 °C, 9.4 ml Methyliodid zugetropft. Über Nacht wird bei -78 °C gerührt, dann eingedampft und in Äther/1N Salzsäure aufgenommen. Die wässrige Phase wird mit Äther extrahiert, die organische Phase über Natriumsulfat getrocknet und eingedampft, um rohes 2-Fluor-3-methyl-anisol zu erhalten. 30 ml Nitrobenzol werden in einem Eisbad gekühlt und dann nacheinander mit 8.1 g Aluminiumchlorid und 11.9 g 4-Brombenzoylchlorid in 9 ml Nitrobenzol bei maximal 6 °C versetzt. Das Gemisch wird gerührt, wonach man das 2-Fluor-3-methyl-anisol so zugibt, dass die Temperatur nicht über 6 °C steigt. Die Lösung wird über Nacht auf Raumtemperatur aufwärmen gelassen, auf Eiswasser/Essigester gegossen und mit 10%-iger wässriger Natriumchlorid-Lösung gewaschen, getrocknet und eingeengt. Nach Chromatographie über Kieselgel mit Hexan/Äther (95/5) als Eluens und Kristallisation aus Hexan werden 4.0 g (4-Brom-phenyl)-(3-fluor-4-methoxy-2-methyl-phenyl)-methanon erhalten, Smp. 93-95 ° C.

Ab) Zu einer Lösung von 1.2 g 11-Acetylamino-undecansäure und 5 Tropfen DMF in 15 ml Methylenchlorid werden bei 0°C 0.5 ml Oxalylchlorid gegeben und 3 Std bei RT nachgerührt. Die Lösung des 11-Acetylamino-undecansäurechlorids wird unter Argon mit 0.6 g 2-Fluoranisol versetzt, auf -15 ° C abgekühlt und mit 1.4 g Alumiumchlorid versetzt. Nach 2 Std bei -15 ° C wird über Nacht auf Raumtemperatur aufwärmen gelassen. Die Lösung wird bei 0°C mit 15 ml 1 M Salzsäure und dann mit 20 ml Wasser versetzt. Die organische Phase wird abgetrennt und mit 1 M Salzsäure, mit Wasser und mit gesättigter Natriumbicarbonat-Lösung gewaschen, getrocknet und eingeengt. Man erhält 1.8 g N-[11-(3-Fluor-4-methoxy-phenyl)-11-oxo-undecyl]acetamid.

Eine Suspension von 21.1 g dieses Produkts in 120 ml Eisessig und 80 ml 62% ige wässrige HBr-Lösung wird unter Rückfluss gekocht, dann eingeengt und mit Toluol nachgedampft. Das Gemisch wird in 240 ml Methylenchlorid und 7.3 ml N-Methylmorpholin gelöst und bei 0 ° C mit 6.9 ml Essigsäure und 24.2 g N-(3-Dimethylaminopropyl)-N'-äthylcarbodiimide-hydrochlorid peracetyliert. Nach Aufarbeiten mit Methylenchlorid/10% iger Kaliumhydrogensulfatlösung, Trocknen der organischen Phase und Eindampfen, wird in 150 ml Methanol gelöst und mit 11.1 ml 5.4 M Natrium-methanolat bei Raumtemperatur gerührt. Die Lösung wird eingeengt, in Methylenchlorid aufgenommen und mit 8% iger Phophorsäurelösung, gesättigter Natriumbicarbonatlösung und 10% iger Natriumchloridlösung gewaschen. Nach Trocknen werden 17.0 g N-[11-(3-Fluor-4-hydroxy-phenyl)-11-oxo-undecyl]acetamid erhalten, MS: m/e 337 (M).

B) Ausgangsverbindung $H_3C$-O-M-C(O)-Q

Eine Lösung von 5.4 g 2,3,5,6-Tetrafluor-anisol in 80 ml absolutem THF wird auf - 78 ° C gekühlt und innert 15 Min mit 20.6 ml 1.6 M Butyllithium in Hexan versetzt. Nach 20 Min werden 7.4 g 4-Brom-N-methoxy-N-methylbenzamid (hergestellt aus 4-Brom-benzoylchlorid und N,O-Dimethylhydoxylamin•Hydrochlorid mit N-Methylmorpholin als Base) in 10 ml THF zugetropft und 2 Std bei - 78 ° C nachgerührt. Die Reaktionslösung wird auf kalte 10%-ige Kaliumhydrogensulfat-Lösung/Essigester gegossen, die organische Phase wird mit Wasser und 10% iger Natriumchloridlösung gewaschen und getrocknet. Nach Kristallisation aus Cyclohexan werden 5.8 g (4-Brom-phenyl)-(2,3,5,6-tetrafluor-4-methoxy-phenyl)-methanon, Smp. von 80-82 ° C erhalten.

C) Ausgangsverbindung $H_3C$-O-M-C(O)-Q

1.45 g $NaSCH_3$ (95%) werden in 80 ml THF suspendiert und über einen Zeitraum von 1.5h mit einer Lösung von 5.51 g (4-Brom-phenyl)-(2-fluor-4-methoxy-phenyl)-methanon in 100 ml THF versetzt. Die Lösung wird bei RT gerührt, erneut mit 264 mg $NaSCH_3$ versetzt und 18 h gerührt. Es wird mit 50 ml ges. $NH_4Cl$-Lösung und dann 100 ml ges. $NaHCO_3$-Lösung versetzt. Die Phasen werden getrennt, die anorganische Phase wird mit $CH_2Cl_2$ extrahiert und die organische Phase mit ges. $NaHCO_3$-Lösung sowie gesättigter Natriumchloridlösung gewaschen und getrocknet. Das Rohprodukt wird auf Kieselgel mit Essigester: Hexan 1:2 als Eluens gereinigt. Es werden 5.88 g (4-Brom-phenyl)-(4-methoxy-2-methylsulfanylphenyl)-methanon als gelbes Öl erhalten.

D) Ausgangsverbindung HO-M-C(O)-Q

Eine Lösung von 52.0 ml Diisopropylamin in 600 ml THF wird bei 0 °C mit 230 ml 1.6 M Butyl-Lithium in Hexan tropfenweise versetzt. Nach 1.5 Std bei 0 °C wird auf -78 °C gekühlt und 26.8 g 2-Fluor-4-hydroxyacetophenon in 120 ml THF zugetropft. Nach 1 Std bei -78 °C wird 23.7 ml 3,3-Dimethylallylbromid in 24 ml THF zugetropft. Man lässt auf Raumtemperatur aufwärmen, worauf bei -78 °C 34 ml Essigsäure in 100 ml Äther zugespritzt werden. Die Lösung wird auf gesättigte Ammoniumchlorid-Lösung/Äther gegossen und mit 10% iger Natriumchlorid-Lösung gewaschen. Nach Trocknen und Eindampfen der organischen Phase werden aus Äther/ Pentan 33.8 g 1-(2-Fluor-4-hydroxy-phenyl)-5-methyl-hex-4-en-1-on, Smp. von 100-101 ° C erhalten.

E) Ausgangsverbindungen der Formel HO-M-T-Q

Ea) Ein Gemisch aus 2,77g 4-Hydroxyphenyl-(4-brom-phenyl)-methanon und 50ml Hexamethyldisilazan wird 4h unter Rückfluß erhitzt, dann eingeengt und getrocknet. Das erhaltene 4-Trimethylsilyloxyphenyl-(4-brom-phenyl)-methanon wird in 60 ml Toluol gelöst und unter Argon bei Raumtemperatur mit 11ml Methylmagnesiumchloridlösung (22% in THF) versetzt. Das Gemisch wird unter Rückfluß gekocht. Nach dem Abkühlen wird das Gemisch mit gesättigter wäßriger Ammoniumchloridlösung versetzt und mit Essigester extrahiert. Die organischen Phasen werden mit gesättigter Natriumchloridlösung gewaschen und getrocknet. Nach dem Eindampfen der Essigesterextrakte werden 3.36 g (RS)-4-[1-(4-Brom-phenyl)-1-hydroxyethyl]-phenol erhalten, MS: m/e 293 ($M+H^+$, 1Br).

Eb) Zu einer Lösung von 5,58g 4-Trimethylsilyoxyphenyl-(4-brom-phenyl)methanon in 80 ml Toluol werden 30,2 ml 15% Vinylmagnesiumchlorid-lösung in THF bei 0°C zugetropft. Das Gemisch wird 2h bei 0°C, dann 2h bei Raumtemperatur gerührt, anschließend mit 40ml Ammoniumchloridlösung hydrolysiert und mit Methylenchlorid extrahiert. Die Extrakte werden getrocknet, eingedampft und über Kieselgel mit Toluol-Aceton als Eluens gereinigt. Es werden 2.8 g (RS)-4-[1-(4-Brom-phenyl)-1-hydroxy-allyl]-phenol erhalten, MS: m/e 304 ($M+H^+$, 1Br).

Ec) Analog Eb) erhält man aus einer Lösung von 4-Trimethylsilyloxyphenyl-(4-brom-phenyl)-methanon und Cyclopropylmagnesiumbromid, das zuvor aus Bromcyclopropan und Magnesium in Ether hergestellt wird, das (RS)-4-[1-(4-Brom-phenyl)-1-hydroxy-cyclopropyl-methyl]-phenol, MS: m/e 300 ($M-H_2O$, 1Br).

Ed) Zu einer einer Lösung von 3.1g 4-Trimethylsilyloxyphenyl-(4-bromphenyl)-methanon in 60 ml THF wird bei 0°C unter Argon 4ml Trifluormethyltrimethylsilan gegeben. Nach 30 min Rühren bei 0°C werden 69.3 ml 1M Tetrabutylammoniumfluoridlösung in THF zugetropft. Das Reaktionsgemisch wird auf Raumtemperatur erwärmt und gerührt, anschließend mit 40 ml Wasser versetzt, nochmals gerührt und dann mit Methylenchlorid extrahiert. Die Extrakte werden mit gesättigter Natriumchlorid-lösung gewaschen, getrocknet und eingedampft. Das Rohprodukt wird über Kieselgel mit Toluol/Aceton (98:2) als Eluens gereinigt. Es werden 3.0 g (RS)-4-[1-(4-Brom-phenyl)-2,2,2-trifluor-1-hydroxyl-ethyl]-phenol erhalten, MS: m/e 290 (M-CO, 1 Br).

Ef) 6,6g (RS)-4-[1-(4-Brom-phenyl)-1-hydroxy-ethyl]-phenol (Bsp. Ea) werden in 50 ml Ethanol gelöst, mit

0,34g p-Toluolsulfonsäure unter Rückfluß gekocht und dann bei 30°C eingedampft. Der Rückstand wird mit 150 ml gesättigter Natriumcarbonatlösung versetzt und mit Essigester extrahiert. Die organischen Phasen werden mit gesättigter Natriumchloridlösung gewaschen, getrocknet und eingedampft. Die Reinigung auf Kieselgel mit Essigester-Hexan (20 : 80) als Eluens gibt 4.1 g 4-[1-(4-Brom-phenyl)-vinyl]-phenol MS: m/e 274 ($M+H^+$, 1Br).

Eg) Ein Gemisch aus 457mg Zinkstaub und 692mg CuCl in 15ml Äther wird unter Argon unter Rückfluß erhitzt. Anschließend wird eine Lösung von 934mg [4-[1-(4-Brom-phenyl)-vinyl]-phenoxy]-trimethyl-silan in 15 ml Äther, die aus 748 mg 4-[1-(4-Brom-phenyl)-vinyl]-phenol (Bsp. Ef) und 14ml Hexamethyldisilazan unter Rückfluß hergestellt wurde, zugetropft und danach 0.56ml Methyleniodid zugegeben. Das Reaktionsgemisch wird unter Rückfluß erhitzt und mit Ether verdünnt. Der Rückstand wird mit Ether gewaschen und das Filtrat mit Wasser gewaschen, getrocknet und eingeengt. Der Rohprodukt wird über Kieselgel mit Essigester-Hexan als Eluens gereinigt. Es wird 4-(1-(4-Brom-phenyl)-cyclopropyl)-phenol als braunes Öl erhalten, MS: m/e 288 ($M+H^+$, 1Br).

F) Analog nachstehendem Beispiel 3 erhält man folgende Zwischenprodukte:

a) aus 3-Fluor-4-hydroxy-acetophenon, (E)-1,4-Dibrom-2-buten und N-Allyl-methyl-amin das (E)-1-[4-[4-(Allyl-methyl-amino)-but-2-enyloxy]-3-fluorphenyl]-ethanon, MS: m/e 278 ($M+H^+$)

b) aus 2-Fluor-4-hydroxy-acetophenon, (E)-1,4-Dibrom-2-buten und N-Allyl-methyl-amin das (E)-1-[4-[4-(Allyl-methyl-amino)-but-2-enyloxy]-2-fluor-phenyl]-ethanon, MS: m/e 278 ($M+H^+$).

c) aus 4-Hydroxy-acetophenon, (E)-1,4-Dibrom-2-buten und N-Allyl-methyl-amin das (E)-1-[4-[4-(Allyl-methyl-amino)-but-2-enyloxy]-phenyl]-ethanon, MS: m/e 260 ($M+H^+$)

d) aus 1-(2-Fluor-4-hydroxy-phenyl)-5-methyl-hex-4-en-1-on (Bsp. D), (E)-1,4-Dibrom-2-buten und N-Allyl-methyl-amin das (E)-1-[4-[(E)-4-(Allyl-methylamino)-but-2-enyloxy]-2-fluor-phenyl]-5-methyl-4-hexen-1-on, welches in das Fumarat (1:1) überführt wird, MS: m/e 345 (M)

e) aus 1-(2-Fluor-4-hydroxy-phenyl)-5-methyl-hex-4-en-1-on (Bsp. D), 1,6-Dibromhexan und N-Allyl-methyl-amin das 1-[4-[6-(Allyl-methyl-amino)-hexyloxy]-2-fluor-pheny]-5-methyl-hex-5-en-1-on, welches in das Fumarat (1:1) überführt wird, MS: m/e 375 (M),

f) aus [4-(6-Brom-hexyloxy)-phenyl]-(4-brom-phenyl)-methanon und 3-Aminopropanol das (4-Brom-phenyl)-[4-[6-[(3-hydroxy-propyl)-amino]-hexyloxy]-phenyl]-methanon, MS: m/e 434 ($M+H^+$, 1Br).

G) 490 mg 6-(Allyl-methyl-amino)-hexan-1-ol (Bsp 42.B) in 5 ml THF werden im Abstand von 1.5 h mit 240 mg NaH (55-60% Dispersion in Mineralöl) und mit 410 mg 1-(6-Chlor-pyridin-3-yl)-ethanon (Bsp 42.D.a) in 4 ml THF versetzt. Die Lösung wird bei RT gerührt, mit Wasser versetzt und filtriert und der Rückstand mit Methylenchlorid gewaschen. Die Phasen werden getrennt, die anorganische Phase wird mit Methylenchlorid und dann mit Essigester extrahiert. Die organischen Phasen werden eingeengt, in Methylenchlorid aufgenommen und mit IM HCl extrahiert. Die saure wäßrige Phase wird mit Methylenchlorid gewaschen, mit NaOH basisch gestellt und mit Methylenchlorid extrahiert. Die organische Phase wird mit $NaHCO_3$-Lösung und gesättigter Natriumchloridlösung gewaschen und getrocknet. Das Rohprodukt wird auf Kieselgel mit Methylenchlorid: Methanol (9:1) gereinigt. Es werden 177.8 mg 1-[6-[6-(Allyl-methyl-amino)-hexyloxy]-pyridin-3-yl]-ethanon als Öl erhalten. 176.4 mg davon werden mit 70 mg Fumarsäure in 1-[6-[6-(Allyl-methyl-amino)-hexyloxy]-pyridin-3-yl]-ethanon•Fumarat (1:1) überführt, MS: m/e 291 ($M+H^+$).

Die Erfindung betrifft die Verbindungen der Formel I und deren Salze zur Verwendung als therapeutische Wirkstoffe, ferner antimykotisch wirksame und Cholesterin senkende Arzneimittel, enthaltend eine Verbindung der Formel I oder ein Salz davon als Wirkstoff, gegebenenfalls neben einem therapeutisch inerten Träger, sowie die Verwendung der Verbindungen der Formel I und der Salze davon für die Herstellung von den oben genannten Arzneimittel.
Cholesterin ist ein Hauptbestandteil der atherosklerotischen Plaques. Der Zusammenhang zwischen koronarer Herzkrankheit (KHK) und hohen LDL-Cholesterinkonzentrationen im Plasma (LDL = Lipoproteine niedriger Dichte) und der therapeutische Nutzen der Senkung erhöhter LDL-Konzentrationen werden heute allgemein anerkannt (Gotto et al., Circulation 81, 1990, 1721-1733; Stein et al., Nutr. Metab. Cardiovasc. Dis. 2, 1992, 113-156). Atherosklerotische Plaques können wachsen und zum Verschluss von Blutgefässen führen, was eine Ischämie oder einen

Infarkt zur Folge hat. Studien zur Primärprophylaxe haben ergeben, dass eine Senkung der LDL-Konzentrationen im Plasma die Häufigkeit der nichttödlichen Anfälle von KHK verringert, während die Gesamtsterblichkeit unverändert bleibt. Die Senkung des LDL-Cholesterinspielgels im Plasma von Patienten mit klinisch bestätigter KHK (sekundäre Intervention) reduziert die KHK-bedingte Sterblichkeit und Morbidität; die Metaanalyse verschiedener Studien zeigt, dass diese Abnahme proportional ist zur Verringerung des LDL-Cholesterins.

Der klinische Nutzen der Cholesterinsenkung ist für Patienten mit bestätigter KHK noch grösser als für asymptomatische Personen mit Hypercholesterinämie. Für die meisten Patienten, die einen Myokardinfarkt überlebt haben, sowie für die Patienten, die an Angina pectoris oder einer anderen atherosklerotischen Erkrankung leiden, ist die Behandlung mit einem Lipidsenker zu empfehlen, wobei eine Konzentration des LDL-Cholesterins von 2,6 mmol/l angestrebt werden sollte.

Für die üblichen Standardtherapien werden Präparate, wie gallensäuresequestrierende Präparate, Fibrate, Nicotinsäure, Probucol sowie die Statine (HMG-CoA-Reduktasehemmer), wie Lovastatin und Simvastatin, verwendet. Für an KHK leidende Patienten, die hohe LDL-Cholesterinspiegel aufweisen und bei denen sich der angestrebte Wert von 2,5 bis 3,0 mmol/l mit Statinen nicht erreichen lässt, wäre ein neues cholesterinsenkendes Medikament von beträchtlichem Nutzen.

Ferner weisen die Statine unerwünschte Nebenwirkungen auf. Sie hemmen die Cholesterinproduktion in einer frühen Phase der Synthesekaskade, wodurch auch die Bildung nicht-steriner Isoprenoide gehemmt wird. Letztere sind für Zellfunktionen unerlässlich. Die Regulation des Zellcyclus, die Modifikation von Eiweissen und der Transport von Elektronen in der Atmungskette können daher durch Statine beeinträchtigt werden.

Aus diesem Grund wurden mehrere Versuche unternommen, plasmacholesterinsenkende Medikamente zu finden, welche die Cholesterinsynthese einerseits nach der Stufe des Farnesyl-Pyrophosphates hemmen, damit die Bildung nicht-steriner Isoprenoide nicht gehemmt wird, und anderseits vor dem Lanosterin, damit es nicht zur Akkumulation von Sterinzwischenprodukten kommt. Zu diesen Substanzen gehören die in der europäischen Patentanmeldung Nr. 636 367 beschriebenen Verbindungen, die die 2,3-Oxidosqualen-lanosterincyclase (OSC) hemmen und das Gesamtcholesterin im Plasma senken.

Die vorliegenden Verbindungen der Formel I hemmen die Cholesterinsynthese und verringern das Gesamtcholesterin im Plasma. Man kann sie daher bei der Therapie und Prophylaxe von Hypercholesterinämie, Hyperlipämie und Arteriosklerose verwenden. Sie besitzen gegenüber bekannten Verbindungen eine bessere Verträglichkeit und stärkere Wirksamkeit. Ferner können sie in der Therapie von Mykosen und hyperproliferativen Erkrankungen Verwendung finden. Zum Beweis der Wirksamkeit der Verbindungen der Formel I und ihrer Salze wurden folgende Versuche durchgeführt.

<u>Hemmung der humanlebermikrosomalen 2,3-Oxidosqualenlanosterincyclase(OSC)</u>

Lebermikrosomen eines gesunden Spenders wurden in Natriumphosphatpuffer(pH 7.4) zubereitet. Die OSC-Aktivität wurde im selben Puffer, der zusätzlich 1 mM EDTA und 1 mM Dithiothreitol enthielt, gemessen. Die Mikrosomen wurden in kaltem Phosphatpuffer auf 0.8 mg/ml Protein verdünnt. Trockenes [$^{14}$C]R,S-Monooxidosqualen (MOS; 12.8 mCi/mmol) wurde mit Aethanol auf 20 nCi/µl verdünnt und mit Phosphatpuffer-1% BSA (Bovine Serum Albumin) gemischt. Eine Stammlösung von 1 mM Testsubstanz in DMSO wurde mit Phosphatpuffer-1% BSA auf die gewünschte Konzentration verdünnt. Es wurden 40 µl Mikrosomen mit 20 µl der Lösung der Testsubstanz gemischt und die Reaktion anschliessend mit 20 µl der [$^{14}$C]R,S-MOS-Lösung gestartet. Die Endbedingungen waren: 0,4 mg/ml mikrosomalen Proteine und 30 µl [$^{14}$C]R,S-MOS in Phosphatpuffer, pH 7.4, enthaltend 0.5% Albumin, DMSO $<$0.1% und Aethanol $<$2%, in einem Gesamtvolumen von 80 µl.

Nach 1 Stunde bei 37°C wurde die Reaktion gestoppt durch Zugabe von 0,6 ml 10% KOH-Methanol, 0,7 ml Wasser und 0,1 ml Hexan:Aether (1:1, v/v), die je 25 µg nicht-radioaktives MOS und Lanosterin als Träger enthielten. Nach Schütteln wurde jedem Röhrchen 1 ml Hexan:Aether (1:1, v/v) zugegeben, nochmals geschüttelt und dann zentrifugiert. Die obere Phase wurde in ein Glasröhrchen transferiert, die untere Phase nochmals mit Hexan:Aether extrahiert, und mit dem ersten Extrakt vereint. Der gesamte Extrakt wurde mit Stickstoff zur Trockene eingedampft, in 50 µl Hexan: Aether suspendiert und auf eine Kieselgelplatte aufgetragen. Die chromatographische Trennung erfolgte in Hexan:Aether (1:1, v/v) als Eluens. Die Rf-Werte für das Substrat MOS und das Produkt Lanosterin betrugen 0,91 bzw. 0,54. Nach dem Trocknen wurden radioaktives MOS und Lanosterin auf der Kieselgelplatte beobachtet. Aus den radioaktiven Banden wurde das Verhältnis von MOS zu Lanosterin ermittelt, um die Hemmung der OSC zu bestimmen.

Der Test wurde einerseits mit einer konstanten Konzentration der Testsubstanz von 100 nM durchgeführt und die prozentuale Hemmung der OSC gegenüber Kontrollen berechnet. Ferner wurde der Test mit verschiedenen Konzentrationen der Testsubstanz durchgeführt und anschliessend der $IC_{50}$-Wert berechnet, d.h. die Konzentration, die erforderlich ist, um die Umwandlung von MOS in Lanosterin auf 50% des Kontrollwertes zu verringern. Die Resultate sind in der nachstehenden Tabelle angegeben:

| Produkt von Beispiel Nr. | 1 | 2l | 2p | 3 | 8a | 8b | 8f | 12 |
|---|---|---|---|---|---|---|---|---|
| Hemmung von OSC (%) | 90 | 81 | 66 | 92 | 82 | 78 | 90 | 84 |
| $IC_{50}$ (nM) | 2,4 | | | 4.9 | 18 | 23,7 | 13,5 | 19 |

| | | | | | | | | | |
|---|---|---|---|---|---|---|---|---|---|
| 13a | 13b | 15 | 29 | 30 | 31 | 32a | 33 | 34a | 35 |
| 82 | 93,5 | 83 | 91 | 93 | 85 | 85 | 98 | 98 | 97 |
| | 7,9 | 32 | 4,5 | 3,2 | 22 | 3,6 | 2,3 | 7,1 | |

| | | | | | |
|---|---|---|---|---|---|
| 36 | 38 | 39a | 49 | 51 | 54b |
| 92 | 82 | 87 | 94 | 87,5 | 73 |
| 8,9 | 21 | 12,3 | 5,5 | 12,8 | |

Cholesterinsenkung in fettgefütterten Hamstern

Männliche Goldhamster in Einzelhaltung wurden während 7 Tagen mit Kokosnussraspel haltiger Diät (40 cal. % Fett) vorbehandelt. Die Tiere wurden dann in Gruppen von 5 Tieren eingeteilt. Während der Behandlung wurden die Tiere auf derselben Diät gehalten. Jede Testsubstanz wurde zuerst in 9 ml Wasser homogenisiert und anschliessend mit der gemahlenen Diät vermischt. Die Kontrollen erhielten lediglich mit Wasser angeteigtes Futter. Die Tiere wurden mit einer Dosis der Testsubstanz von 200 µmol (etwa 70-120 mg/kg/Tag) während 10 Tagen behandelt. Blutproben (200 µl) wurden unter leichter Anästhesie via Jugular-Vene genommen, und zwar je am letzten Tag der Vorbehandlung und einen Tag nach der letzten Verabreichung der Testsubstanz. Die Konzentration des Cholesterins im Plasma wurde mit einer kolorimetrischen Enzymmethode bestimmt. Die Plasmalipoproteine wurden durch Ausschlusschromatographie (Hennes et al., Science Tools, 36, 1992, 10-12) getrennt. Das Gesamtcholesterin wurde in jeder Fraktion mit einer fluorometrischen Enzymmmethode bestimmt (Gamble et al., J. Lipid Res., 19, 1978, 1068-1071), um die Menge des Cholesterins in den LDL- und HDL-Fraktionen zu berechnen. Für die Produkte der Beispiele 8a und 12 ist die Wirkung auf das Plasmacholesterin sowie das LDL- und HDL-Cholesterin, ausgedrückt in Prozent der Kontroll-Tiere, in der nachstehenden Tabelle wiedergegeben.

| Beispiel | 8a | 12 |
|---|---|---|
| Gesamtcholesterin | -30% | -25% |
| LDL-Cholesterin | -51% | -54% |
| HDL-Cholesterin | -18% | -23% |

Wie bereits erwähnt besitzen die Verbindungen der Formel I und ihre pharmazeutisch annehmbaren Säureadditionssalze ausserdem wertvolle antifungale Eigenschaften. Sie sind wirksam gegen eine Vielzahl von pathogenen Pilzen, die topische und systemische Infektionen hervorrufen, wie Candida albicans, Cryptococcus neoformans und Aspergillus fumigatus.

Antifungale Aktivität in vitro

Die Verbindungen wurden auf antifungale Aktivität gegen Candida albicans, Cryptococcus neoformans und Aspergillus fumigatus mittels einer Mikroverdünnungsmethode auf Mikrotiterplatten (96 Löcher pro Platte) getestet. Mit 1% Glukose und 0,25% di-Kaliumphosphat angereicherte Hefe wurde für die drei Pilzstämme verwendet. Die Pilz-

zellen wurden mit $3x10^4$ cfu (colony forming unit) in 1 ml Medium pro Loch angeimpft. Das Medium enthielt steigende Konzentrationen des Testsubstanz. Nach 24 bzw. 48 Stunden Inkubation bei 27°C wurde die Trübung in jedem Loch durch einen Mikrotiterplatten-Leser gemessen. Die Hemmung des Wachstums wurde im Vergleich zur Kontrolle (ohne Testsubstanz) berechnet. Die $IC_{50}$ ist die Konzentration der Testsubstanz, bei der das Wachstum um 50% gehemmt wird.

| Verbindung I Beispiel Nr. | $IC_{50}$ (mg/ml) bei: C. albicans | | C. neoformans 48 Stunden | A. fumigatus 48 Stunden |
|---|---|---|---|---|
| | nach: 24 Std. | 48 Std. | | |
| 25 | 0,32 | <0,32 | 1,00 | 0,82 |
| 26c | 0,49 | 5,70 | 2,70 | 8,40 |
| 29 | 0,71 | 21,00 | <0,32 | 0,71 |
| 36 | <0,32 | 1,10 | <0,32 | 2,40 |
| 43b | <0,32 | 5,00 | <0,32 | 6,40 |
| 48 | <0,32 | <0,32 | 0,47 | 18,00 |
| 54b | <0,32 | 6,50 | 0,69 | 6,10 |

Die Verbindungen der Formel I und ihre pharmazeutisch annehmbaren Säureadditionssalze können als Heilmittel, z.B. in Form pharmazeutischer Präparate zur enteralen, parenteralen oder topischen Applikation, Verwendung finden. Sie können beispielsweise peroral, z.B. in Form von Tabletten, Lacktabletten, Dragées, Hart- und Weichgelatinekapseln, Lösungen, Emulsionen oder Suspensionen, rektal, z.B. in Form von Suppositorien, parenteral, z.B. in Form von Injektionslösungen oder Infusionslösungen, oder topisch, z.B. in Form von Salben, Crèmen oder Oelen, verabreicht werden.

Die Herstellung der pharmazeutischen Präparate kann in jedem Fachmann geläufiger Weise dadurch erfolgen, dass man die beschriebenen Verbindungen der Formel I und ihre pharmazeutisch annehmbaren Säureadditionssalze, gegebenenfalls in Kombination mit anderen therapeutisch wertvollen Stoffen, zusammen mit geeigneten, nichttoxischen, inerten, therapeutisch verträglichen festen oder flüssigen Trägermaterialien und gegebenenfalls den üblichen pharmazeutischen Hilfsstoffen in eine galenische Darreichungsform bringt.

Als Trägermaterialien eignen sich sowohl anorganische als auch organische Trägermaterialien. So kann man für Tabletten, Lacktabletten, Dragées und Hartgelatinekapseln beispielsweise Lactose, Maisstärke oder Derivate davon, Talk, Stearinsäure oder deren Salze als Trägermaterialien verwenden. Für Weichgelatinekapseln eignen sich als Trägermaterialien beispielsweise pflanzliche Oele, Wachse, Fette und halbfeste und flüssige Polyole (je nach Beschaffenheit des Wirkstoffes sind jedoch bei Weichgelatinekapseln keine Träger erforderlich). Zur Herstellung von Lösungen und Sirupen eignen sich als Trägermaterialien beispielsweise Wasser, Polyole, Saccharose, Invertzucker und Glucose. Für Injektionslösungen eignen sich als Trägermaterialien beispielsweise Wasser, Alkohole, Polyole, Glycerin und pflanzliche Oele. Für Suppositorien eignen sich als Trägermaterialien beispielsweise natürliche oder gehärtete Oele, Wachse, Fette und halbflüssige oder flüssige Polyole. Für topische Präparate eignen sich als Trägermaterialien Glyceride, halbsynthetische und synthetische Glyceride, hydrierte Oele, flüssige Wachse, flüssige Paraffine, flüssige Fettalkohole, Sterole, Polyäthylenglykole und Cellulosederivate.

Als pharmazeutische Hilfsstoffe kommen die üblichen Stabilisierungs-, Konservierungs-, Netz- und Emulgiermittel, Mittel zur Verbesserung der Konsistenz, Mittel zur geschmacklichen Verbesserung, Salze zur Veränderung des osmotischen Druckes, Puffersubstanzen, Lösungsvermittler, Färbe- und Ueberzugsmittel und Antioxidantien in Frage.

Die Dosierung der Verbindungen der Formel I kann, abhängig von den zu bekämpfenden pathogenen Pilzen, dem Alter und dem individuellen Zustand des Patienten und von der Applikationsweise, innerhalb weiter Grenzen variieren und ist natürlich in jedem einzelnen Fall den individuellen Gegebenheiten anzupassen. Zur Verhütung und Bekämpfung von topischen und systemischen Infektionen durch pathogene Pilze kommt für den erwachsenen Patienten eine tägliche Dosis von etwa 0,01 g bis etwa 4 g, insbesondere etwa 0,05 g bis etwa 2 g in Betracht. Für die Cholesterinsenkung beträgt die Tagesdosis zweckmässigerweise zwischen 1 und 1200 mg, vorzugsweise 5 bis 100 mg, für den erwachsenen Patienten. Je nach Dosierung ist es dabei zweckmässig, die Tagesdosis in mehreren Dosierungseinheiten zu verabreichen.

Die pharmazeutischen Präparate enthalten zweckmässigerweise etwa 1-500 mg, vorzugsweise 2-200 mg einer Verbindung der Formel I.

Die nachfolgenden Beispiele dienen der näheren Erläuterung der vorliegenden Erfindung. Sie sollen deren Umfang jedoch in keiner Weise beschränken. Sämtliche Temperaturen sind in Celsiusgraden angegeben.

Beispiel 1

a) 75 ml Nitrobenzol werden in einem Eisbad gekühlt und nacheinander mit 17.3 g Aluminiumchlorid und 4-Brombenzoylchlorid in 25 ml Nitrobenzol bei maximal 6 °C versetzt. Das Gemisch wird 10 Min gerührt, wonach man 15.7 g 2,5-Difluoranisol so zugibt, dass die Temperatur 6°C nicht übersteigt. Die Lösung wird über Nacht auf Raumtemperatur aufwärmen gelassen, dann auf Eiswasser gegossen und mit Methylenchlorid extrahiert. Die organische Phase wird mit Wasser und 10% Natriumchlorid-Lösung gewaschen, über Natriumsulfat getrocknet und eingeengt. Nach Kristallisation aus Cyclohexan werden 28.4 g (4-Brom-phenyl)-(2,5-difluor-4-methoxy-phenyl)-methanon erhalten.

b) Eine Lösung von 22.9 g (4-Brom-phenyl)-(2,5-difluor-4-methoxy-phenyl)-methanon in 140 ml Essigsäure und 100 ml 62%-iger wässriger HBr-Lösung wird während 13 Std unter Rückfluss gekocht, anschliessend eingedampft, mit Toluol nachgedampft und in Essigester aufgenommen. Die anorganische Phase wird mit gesättigter Natriumhydrogencarbonat-Lösung und 10% iger Natriumchlorid-Lösung gewaschen und getrocknet. Nach Kristallisation aus Methylenchlorid/Äther/Pentan werden 20.8 g (4-Brom-phenyl)-(2,5-difluor-4-hydroxy-phenyl)-methanon erhalten.

c) Zu einer Lösung von 45.8 g (E)-1,4-Dibrom-2-buten, 20.8 g (4-Bromphenyl)-(2,5-difluor-4-hydroxy-phenyl)-methanon und 1.2 g Tetrabutylammoniumbromid in 150 ml Methylenchlorid gibt man 150 ml 10%ige Natronlauge. Das Gemisch wird während 4 Std bei Raumtemperatur gerührt, auf Wasser gegossen und mit Essigester extrahiert. Die organische Phase wird mit 10% iger Natriumchlorid-Lösung gewaschen, getrocknet, abfiltriert und eingedampft. Die Kristallmasse wird über Kieselgel mit Methylenchlorid als Eluens gereinigt, wobei 15.3 g (E)-[4-(4-Brom-but-2-enyloxy)-2,5-difluor-phenyl]-(4-brom-phenyl)-methanon erhalten werden.

d) Das oben erhaltene (E)-[4-(4-Brom-but-2-enyloxy)-2,5-difluor-phenyl]-(4-brom-phenyl)-methanon wird in 170 ml Äthanol gelöst und mit 17.2 ml N-Allyl-methyl-amin und 12 g Kaliumcarbonat während 3 Std gekocht, eingeengt, mit Wasser versetzt, mit Methylenchlorid extrahiert, mit 10% iger Natriumchlorid-Lösung gewachen, getrocknet, filtriert und eingeengt. Der Rückstand wird in Methylenchlorid gelöst, auf 0°C gekühlt und mit 7.2 ml 4.8M Salzsäure-Lösung in Äther versetzt. Kristallisation aus Methylenchlorid/Essigester ergibt 8.9 g (E)-[4-[4-(Allyl-methyl-amino)-but-2-enyloxy]-2,5-difluor-phenyl]-(4-brom-phenyl)-methanon•Hydrochlorid (1:1), Smp. 150°C.

Beispiel 2

Analog Beispiel 1 erhält man:

a) aus (E)-[4-(4-Brom-but-2-enyloxy)-2,5-difluor-phenyl]-(4-brom-phenyl)-methanon (Bsp 1c) und einer 33%-Lösung Dimethylamin in Äthanol, das (E)-(4-Brom-phenyl)-[2,5-difluor-4-(4-dimethylamino-but-2-enyloxy]-methanon•Hydrochlorid (1;1), Smp. 172 °C,

b) aus (4-Brom-phenyl)-(2,5-difluor-4-hydroxy-phenyl)-methanon (Bsp 1b) und 1,6-Dibromhexan, via 4-(6-Brom-hexyloxy)-2,5-difluor-phenyl]-(4-bromphenyl)-methanon, welches man mit N-Allyl-methyl-amin umsetzt, das [4-[6-(Allyl-methyl-amino)-hexyloxy]-2,5-difluor-phenyl]-(4-brom-phenyl)-methanon•Hydrochlorid (1:1), Smp. 134 °C,

c) aus 2,4-Difluorbenzoylchlorid und 2,5-Difluoranisol, via (2,4-Difluorphenyl)-(2,5-difluor-4-methoxy-phenyl)-methanon, welches man mit Bromwasserstoff entschützt und mit (E)-1,4-Dibrom-2-buten und N-Allyl-methyl-amin umsetzt, das (E)-[4-[4-(Allyl-methyl-amino)-but-2-enyloxy]-2,5-difluor-phenyl]-(2,4-difluor-phenyl)-methanon•Hydrochlorid (1:1), Smp. 141°C,

d) aus 2,4-Difluorbenzoylchlorid und 2,5-Difluoranisol, via (2,4-Difluorphenyl)-(2,5-difluor-4-methoxy-phenyl)-methanon, welches man mit Bromwasserstoff entschützt und mit (E)-1,4-Dibrom-2-buten und einer 33%-Lösung Dimethylamin in Äthanol umsetzt, das (E)-[2,5-Difluor-4-(4-dimethylamino-but-2-enyloxy)-phenyl]-(2,4-difluor-phenyl)-methanon• Hydrochlorid (1:1), Smp. 159 °C,

e) aus 2,4-Difluorbenzoylchlorid und 2,5-Difluoranisol, via (2,4-Difluorphenyl)-(2,5-difluor-4-methoxy-phenyl)-methanon, welches man mit Bromwasserstoff entschützt und mit 1,6-Dibromhexan und N-Allyl-methyl-amin umsetzt, das [4-[6-(Allyl-methyl-amino)-hexyloxy]-2,5-difluor-phenyl]-(2,4-difluor-phenyl)-methanon•Hydrochlorid (1:1),

MS: m/e 423 (M),

f) aus 2,4-Difluorbenzoylchlorid und 2,5-Difluoranisol, via (2,4-Difluorphenyl)-(2,5-difluor-4-methoxy-phenyl)-methanon, welches man mit Bromwasserstoff entschützt und mit 1,6-Dibromhexan und einer 33%-Lösung Dimethylamin in Äthanol umsetzt, das (2,4-Difluor-phenyl)-[4-(6-dimethylamino-hexyloxy)-2,5-difluor-phenyl] methanon •Hydrochlorid (1:1), MS: m/e 396 (M),

g) aus Cyclohexyl-4-hydroxyphenyl-methanon mit (E)-1,4-Dibrom-2-buten und einer 33%-Lösung Dimethylamin in Äthanol, das Cyclohexyl-p-[[(E)-4-(dimethylamino)-2-butenyl]oxy]phenyl-methanon, MS: m/e 382 (M),

h) aus (4-Brom-phenyl)-(2,3,5,6-tetrafluor-4-methoxy-phenyl)-methanon (Bsp. B), welches man mit Bromwasserstoff entschützt, mit (E)-1,4-Dibrom-2-buten und N-Allyl-methyl-amin umsetzt, das (E)-[4-[4-(Allyl-methylamino)-but-2-enyloxy] -2,3,5,6-tetrafluor-phenyl] -(4-brom-phenyl]-methanon•Hydrochlorid (1:1), MS: m/e 444 ($M-C_2H_5$, 1Br).

i) aus (4-Brom-phenyl)-(3-fluor-4-methoxy-2-methyl-phenyl)-methanon (Bsp. Aa), welches man mit Bromwasserstoff entschützt, mit (E)-1,4-Dibrom-2-buten und N-Allyl-methyl-amin umsetzt, das (E)-[4-[4-(Allylmethyl-amino)-but-2-enyloxy]-3-fluor-2-methyl-phenyl]-(4-brom-phenyl)-methanon•Hydrochlorid (1:1), MS: m/e 432 ($M+H^+$, 1Br),

j) aus (E)-[4-(4-Brom-but-2-enyloxy)-phenyl]-(4-brom-phenyl)-methanon und Methylaminoacetonitril•Hydrochlorid, mit Triethylamin in Ethanol, das (E)-[[4-[4-(4-Brom-benzoyl)-phenoxy]-but-2-enyl]-methyl-amino]-acetonitril, das in das Hydrochlorid überführt wird, MS: m/e 399 ($M+H^+$, 1Br),

k) aus (E)-[4-(4-Brom-but-2-enyloxy)-phenyl]-(4-brom-phenyl)-methanon und 3-Methylaminopropionitril, das (E)-3-[[4-[4-(4-Brom-benzoyl)-phenoxy]-but-2-enyl]-methyl-amino]-propionitril, das in das Fumarat überführt wird, MS: m/e 413 ($M+H^+$, 1Br),

l) aus (E)-[4-(4-Brom-but-2-enyloxy)-phenyl]-(4-brom-phenyl)-methanon und 4-Hydroxypiperidin in Ethanol, das (E)-(4-Brom-phenyl)-[4-[4-(4-hydroxypiperidin-1-yl)-but-2-enyloxy]-phenyl]-methanon, das in das Fumarat überführt wird, MS: m/e 429 ($M+H^+$, 1Br),

m) aus (E)-[4-(4-Brom-but-2-enyloxy)-phenyl]-(4-brom-phenyl)-methanon und 4-Dimethylamino-piperidin in $CH_2Cl_2$, das (E)-(4-Brom-phenyl)-[4-[4-(4-dimethylamino-piperidin-1-yl)-but-2-enyloxy]-phenyl]-methanon, das in das (E)-(4-Brom-phenyl)-[4-[4-(4-dimethylamino-piperidin-1-yl)-but-2-enyloxy]-phenyl]-methanon•Hydrochlorid überführt wird, MS: m/e 457 ($M+H^+$),

n) aus [4-(4-Brom-hexyloxy)-phenyl]-(4-brom-phenyl)-methanon und N-Methylhydroxylamin•Hydrochlorid, das (4-Brom-phenyl)-[4-[6-(hydroxymethyl-amino)-hexyloxy]-phenyl]-methanon, MS: m/e 406 ($M+H^+$, 1Br),

o) aus (E)-[4-(4-Brom-but-2-enyloxy)-phenyl]-(4-brom-phenyl)-methanon und N-Methylhydroxylamin•Hydrochlorid, das (E)-(4-Brom-phenyl)-[4- [4-(hydroxy-methyl-amino)-but-2-enyloxy]-phenyl]-methanon, MS: m/e 376 ($M+H^+$, 1Br),

p) aus [4-(6-Brom-hexyloxy)-2-fluor-phenyl]-(4-brom-phenyl)-methanon und N-Methylcyclopropylamin•Hydrochlorid, das (4-Brom-phenyl)- [4- [6-(cyclopropyl-methyl-amino)-hexyloxy]-2-fluor-phenyl]-methanon, das in das Hydrochlorid überführt wird, MS: m/e 447 (M, 1Br).

Beispiel 3

a) 1.0 g (4-Brom-phenyl)-(4-methoxy-2-methylsulfanyl-phenyl)-methanon (Bsp. C) werden in 6 ml Essigsäure und 3.5 ml 62% ige HBr suspendiert und über Nacht bei 125°C gerührt. Die Suspension wird auf ges. $NaHCO_3$-Lösung gegeben, die Phasen werden getrennt und die anorganische Phase wird mit Essigester extrahiert. Die organischen Phasen werden mit ges. $NaHCO_3$-Lösung sowie gesättigter Natriumchloridlösung gewaschen und getrocknet. Es werden 920 mg (4-Brom-phenyl)-(4-hydroxy-2-methylsulfanylphenyl)-methanon als braune Kristalle erhalten.

b) 450 mg (4-Brom-phenyl)-(4-hydroxy-2-methylsulfanyl-phenyl)-methanon werden in 7 ml Aceton aufgenommen

und mit 1.24 g $K_2CO_3$ und 530 µl 1,6-Dibromhexan versetzt. Die Suspension wird über Nacht unter Rückfluß erhitzt, abgekühlt, filtriert und eingeengt. Nach dem Entfernen des überschüßigen 1,6-Dibromhexans werden 710 mg [4-[6-(Brom)-hexyloxy]-2-methylsulfanyl-phenyl]-(4-brom-phenyl)-methanon als braunes Öl erhalten.

c) Das Produkt von b) wird in 7 ml DMA aufgenommen und mit 420 µl N-Allyl-methyl-amin über Nacht bei RT gerührt. Das Gemisch wird eingeengt und das zurückbleibende Öl in $CH_2Cl_2$ aufgenommen, mit ges.$NaHCO_3$-Lösung und ges. Natriumchloridlösung gewaschen und getrocknet. Das nach dem Abziehen und Reinigen an Kieselgel mit $CH_2Cl_2$:Methanol 95:5 erhaltene gelbe Öl (506 mg) [4-[6-(Allyl-methyl-amino)-hexyloxy]-2-methylsulfanyl-phenyl]-(4-brom-phenyl)-methanon wird in Ethanol aufgenommen und mit 111.6 mg Fumarsäure versetzt. Nach dem Rühren wird die Lösung eingeengt, in Essigester aufgenommen, eingeengt und anschließend lyophilisiert. Es werden 695 mg [4-[6-(Allyl-methyl-amino)-hexyloxy]-2-methylsulfanyl-phenyl]-(4-brom-phenyl)-methanon• Fumarat (1:1) als klebriges Öl erhalten, MS: m/e 476 ($M+H^+$, 1Br).

Beispiel 4

Analog Beispiel 3 erhält man

a) aus (4-Brom-phenyl)-(4-methoxy-2-methylsulfanyl-phenyl)-methanon (Bsp. C), via (E)-[4-[4-(Brom)-but-2-enyloxy]-2-methylsulfanyl-phenyl]-(4-brom-phenyl)-methanon, das (E)-[4-[4-(Allyl-methyl-amino)-but-2-enyloxy]-2-methylsulfanyl-phenyl]-(4-brom-phenyl)-methanon•Fumarat (1:1), MS: m/e 446 ($M+H^+$, 1Br),

b) aus (E)-[4-(4-Brom-but-2-enyloxy)-phenyl]-(4-brom-phenyl)-methanon und (2-Methoxy-ethyl)-methyl-amin, das (E)-(4-Brom-phenyl)-[4-[4-[(2-methoxyethyl)-methyl-amino]-but-2-enyloxy]-phenyl]-methanon•Fumarat (1:1), Smp. 92-98 °C,

c) aus N-[11-(3-Fluoro-4-hydroxy)-11-oxo-undecyl]acetamid] (Bsp. Ab), (E)-1,4-Dibrom-2-buten und N-Allyl-methyl-amin, das (E)-N-[11-[4-[4-(Allyl-methylamino)-but-2-enyloxy] -3-fluor-phenyl]-11-oxo-undecyl] -acetamid•Fumarat (1:1), Smp. 69-71° C,

d) aus 1-(2-Fluor-4-hydroxy-phenyl)-5-methyl-hex-4-en-1-on (Bsp. D), 1,6-Dibromhexan und Cyclopropylmethyl-methylamin, das 1-[4-[6-(Cyclopropylmethyl-methyl-amino)-hexyloxy]-2-fluor-phenyl]-5-methyl-hex-4-en-1-on, MS: m/e 390 ($M+H^+$),

e) aus 1-(2-Fluor-4-hydroxy-phenyl)-5-methyl-hex-4-en-1-on (Bsp. D), 1,6-Dibromhexan und Cyclopropyl-methyl-amin•Hydrochlorid, das 1-[4-[6-(Cyclopropyl-methyl-amino)-hexyloxy]-2-fluor-phenyl]-5-methyl-hex-4-en-1-on, MS: m/e 376 ($M+H^+$),

f) aus (4-Brom-phenyl)-(4-hydroxy-phenyl)-methanon, (E)-1,4-Dibrom-2-buten und 1-Methylamino-2-methylthio-äthan, das (E)-(4-Brom-phenyl)-[4-[4-[methyl-(2-methylsulfanyl-ethyl)-amino]-but-2-enyloxy]-phenyl]-methanon•Fumarat (1:1), MS: m/e 434 ($M+H^+$, 1Br).

Beispiel 5

Eine Lösung von 0.88 g (4-Brom-phenyl)-(2,4-dihydroxy-phenyl)-methanon und (E)-1,4-Dibrom-2-buten in 7.5 ml DMF und 0.56 g Lithiumcarbonat wird 48 Std bei 40°C gerührt. Nach Aufarbeitung mit Methylenchlorid/0.5 M Salzsäure und Reinigung über Kieselgel mit Hexan/Essigester (9:1) erhält man 0.14 g (E)-[4-(4-Brom-but-2-enyloxy)-2-hydroxy-phenyl]-(4-brom-phenyl)-methanon, welches analog Beispiel 3c) mit N-Allyl-methyl-amin das (E)-[4-(4-Allyl-methyl-amino-but-2-enyloxy)-2-hydroxy-phenyl]-(4-brom-phenyl)-methanon ergibt, das in das Hydrochlorid überführt wird, MS: m/e 416 ($M+H^+$, 1Br).

Beispiel 6

Zu einer Suspension von 44 mg 55% igem Natriumhydrid in 4 ml DMF, wird eine Lösung von 68 mg Imidazol in 4 ml DMF zugetropft. Nach dem Erwärmen des Gemisches auf 70° C, wird eine Lösung von 441mg (E)-[4-(4-Brom-but-2-enyloxy)-phenyl]-(4-brom-phenyl)-methanon in 12 ml DMF zugetropft. Dann wird das Gemisch noch 1 h bei 70° C gerührt und nach dem Abkühlen auf 5 ml Wasser gegossen und konzentriert. Der Rückstand wird mit Wasser versetzt und mit Methylenchlorid extrahiert. Die organischen Phasen werden mit gesättigter Natriumchloridlösung gewaschen, getrocknet und eingedampft. Der Rückstand wird mit Toluol/Aceton/Triethylamin (70 : 29: 1) auf Kieselgel gereinigt.

Das (E)-(4-Brom-phenyl)-[4-(4-imidazol-1-yl-but-2-enyloxy)-phenyl]-methanon (140 mg) wird als beige feste Base erhalten, die in das Hydrochlorid übergeführt wird, MS: m/e 397 (M, 1Br).

Beispiel 7

Analog Beispiel 6 erhält man

aus 4-(6-Bromhexyloxy)-phenyl]-(4-brom-phenyl)-methanon und Imidazol, das (4-Brom-phenyl)-[4-(6-imidazol-1-yl-hexyloxy)-phenyl]-methanon als weiße feste Base, die in das Hydrochlorid überführt wird, MS: m/e 427 ($M+H^+$, 1Br).

Beispiel 8

Analog Beispiel 3 erhält man:

a) aus (RS)-4-(1-(4-Brom-phenyl)-1-hydroxy-ethyl)-phenol (Bsp. Ea), (E)-1,4-Dibrom-2-buten und N-Allyl-methyl-amin, das (E)-(RS)-1-[4-[4-(Allyl-methylamino)-but-2-enyloxy]-phenyl]-(4-brom-phenyl)-ethanol, welches in das Fumarat überführt wird, MS: m/e 416 ($M+H^+$, 1Br).

b) aus (RS)-4-(1-(4-Brom-phenyl)-1-hydroxy-allyl)-phenol (Bsp. Eb), (E)-1,4-Dibrom-2-buten und N-Allyl-methyl-amin, das (E)-(RS)-1-[4-[4-(Allyl-methylamino)-but-2-enyloxy]-phenyl]-1-(4-brom-phenyl)-prop-2-en-1-ol, das in das Fumarat übergeführt wird, MS: m/e 428 ($M+H^+$, 1Br),

c) aus (RS)-4-(1-(4-Brom-phenyl)-1-hydroxy-cyclopropyl-methyl)-phenol (Bsp. Ec), (E)-1,4-Dibrom-2-buten und N-Allyl-methyl-amin, das (E)-(RS)-[4-[4-(Allyl-methyl-amino)-but-2-enyloxy]-phenyl]-(4-brom-phenyl)-cyclopropyl-methanol als Hydrobromid, Smp 143-144°C,

d) aus (RS)-4-(1-(4-Brom-phenyl)-2,2,2-trifluor-1-hydroxyl-ethyl)-phenol (Bsp. Ed), (E)-1,4-Dibrom-2-buten und N-Allyl-methyl-amin, das (E)-(RS)-1-[4-[4-(Allyl-methyl-amino)-but-2-enyloxy]-phenyl]-1-(4-brom-phenyl)-2,2,2-trifluor-ethanol, das in das Fumarat übergeführt wird, MS: m/e 470 ($M+H^+$, 1Br),

e) aus (RS)-4-(1-(4-Brom-phenyl)-2,2,2-trifluor-1-hydroxyl-ethyl)-phenol (Bsp. Ed), 1,6-Dibromhexan und N-Allyl-methyl-amin, das (RS)-1-[4-[6-(Allyl-methyl-amino)-hexyloxy]-phenyl]-1-(4-brom-phenyl)-2,2,2-trifluorethanol, das in das Fumarat übergeführt wird, MS: m/e 500 ($M+H^+$, 1Br).,

f) aus (RS)-4-(1-(4-Brom-phenyl)-vinyl)-phenol (Bsp. Ef), (E)-1,4-Dibrom-2-buten und N-Allyl-methyl amin, das (E)-Allyl-[4-[4-[1-(4-brom-phenyl)-vinyl]-phenoxy]-but-2-enyl]-methyl-amin, das in das Fumarat übergeführt wird, MS: m/e 398 ($M+H^+$, 1Br).

g) aus 4-(1-(4-Brom-phenyl)-cyclopropyl)-phenol (Bsp. Eg) und (E)-1,4-Dibrom-2-buten, via (E)-1-[4-(4-Brom-but-2-enyloxy)-phenyl]-1-(4-bromphenyl)-cyclopropan und Reaktion mit N-Allyl-methyl-amin, das (E)-Allyl-[4-[4-[1-(4-brom-phenyl)-cyclopropyl]-phenoxy]-but-2-enyl]-methyl-amin, das in das Fumarat übergeführt wird, MS: m/e 412 ($M+H^+$, 1Br).

Beispiel 9

Die zwei Enantiomere des (E)-(RS)-1-[4-[4-(Allyl-methyl-amino)-but-2-enyloxy]-phenyl]-(4-brom-phenyl)-ethanol (Bsp 8a) werden durch Super Critical Fluid Chromatography über mit einem Amylosederivat beschichteten Silicagel mit 30% Methanol und 0.5% Butylamin in $CO_2$ als Eluens getrennt. Man erhält

a) das (E)-(R oder S)-1-[4-[4-(Allyl-methyl-amino)-but-2-enyloxy]-phenyl]-(4-brom-phenyl)-ethanol und

b) das (E)-(S oder R)-1-[4-[4-(Allyl-methyl-amino)-but-2-enyloxy]-phenyl]-(4-brom-phenyl)-ethanol, MS: m/e 416 ($M+H^+$, 1Br).

Beispiel 10

Analog Beispiel 3 erhält man das Allyl-[6-[4-[1-(4-brom-phenyl)-vinyl]-3-fluor-phenoxy]-hexyl]-methyl-amin, das in das Fumarat überführt wird, MS: m/e 423 (M, 1Br).

Beispiel 11

Eine Lösung von 1.84 g (E)-N-[11-[4-[4-(Allyl-methyl-amino)-but-2-enyloxy]-3-fluor-phenyl]-11-oxo-undecyl]-acetamid (Bsp 4c) in 30 ml Äthanol/5 ml 18% iger wässriger Salzsäure wird während 7 Std gekocht, und nach Zusatz von 4 ml konzentrierter Salzsäure während 24 Std und nach Zusatz von weiteren 4 ml konzentrierter Salzsäure 30 Std gekocht. Nach dem Einengen wird in 10% iger Kaliumhydrogensulfatlösung/ Methylenchlorid aufgenommen, die wässrige Phase mit 10%iger Natriumhydroxid-Lösung basich gestellt und mit Methylenchlorid extrahiert. Die orgnische Phase wird getrocknet und eingeengt. Man erhält 1.48 g (E)-1-[4-[4-(Allyl-methyl-amino)-but-2-enyloxy]-3-fluor-phenyl]-11-amino-undecan-1-on, welches in das Fumarat überführt wird, MS: m/e 419 ($M+H^+$).

Beispiel 12

a) Eine Suspension aus 10g (E)-[4-(4-Brom-but-2-enyloxy)-phenyl]-(4-bromphenyl)-methanon, 7.47g N-Cyclopropyl-2,2,2-trifluor-acetamid, 6.7g Kaliumcarbonat und 0,55g Benzyltriethylammoniumbromid in 200ml Acetonitril wird 7h unter Rühren zum Sieden erhitzt. Nach dem Abkühlen auf Raumtemperatur wird die Suspension filtriert. Das Filtrat wird eingeengt; der Rückstand in einem Gemisch aus 500ml Methylenchlorid und 50ml Methanol gelöst und mit 50g eines stark sauren und stark basischen Ionenaustauschers gerührt, anschließend filtriert und mit Methylenchlorid gewaschen, wobei sich zwei Phasen bilden. Die organische Phase wird getrocknet und eingeengt. Es werden 11,5g N-[4-[4-(4-Brombenzoyl)-phenoxy]-but-2-enyl]-N-cyclopropyl-2,2,2-trifluoracetamid als gelblicher Feststoff erhalten, MS: m/e 412 ($M^+$-$CF_3$, 1Br).

b) Eine Lösung aus 11,5g N-[4-[4-(4-Brom-benzoyl)-phenoxy]-but-2-enyl)-N-cyclopropyl-2,2,2-trifluoracetamid in 150ml Methanol und 50ml Tetrahydrofuran wird unter Eiskühlung mit 5ml 20% wäßriger Kalilauge versetzt. Das Gemisch wird auf Raumtemperatur gebracht und eine Stunde gerührt, unter vermindertem Druck konzentriert und mit 100ml Wasser versetzt. Die Phasen werden getrennt, und die anorganische Phase wird mit Methylenchlorid extrahiert. Die organischen Auszüge werden mit gesättigter Natriumchloridlösung gewaschen, getrocknet und eingedampft. Nach der Reinigung des Rückstandes über Kieselgel mit Essigester-Hexan-Triethylamin (50/49/1), werden 7,0g (4-Brom-phenyl)-[4-(4-cyclopropylamino-but-2-enyloxy)-phenyl]-methanon erhalten, MS: m/e 386 ($M+H^+$, 1Br),

c) Das Produkt von b) wird in 100ml einer IN $NaH_2PO_4$-Lösung aufgenommen und mit 100ml Dioxan versetzt. Nach Zugabe von 10ml einer 37% wäßrigen Formaldehydlösung wird 3 Stunden auf 65°C erwärmt. Zur Aufarbeitung wird mit 10% wäßriger Natronlauge auf pH>11 gestellt und mit Ether extrahiert. Nach dem Eindampfen der etherischen Extrakte und Chromatographie des Rückstandes an Kieselgel mit Essigester-Hexan-Triethylamin (von 19/80/1 bis 29/70/1), werden 5.0g (E)-(4-Brom-phenyl)-[4-[4-(cyclopropyl-methyl-amino)-but-2-enyloxy]-phenyl]-methanon erhalten, das ins Hydrochlorid überführt wird, MS: m/e 400 ($M+H^+$, 1Br).

Beispiel 13

Analog Beispiel 12 erhält man:

a) aus [4-(6-Brom-hexyloxy)-phenyl]-(4-brom-phenyl)-methanon, das [6-[6-(Cyclopropyl-methyl-amino)-hexyloxy]-phenyl]-(4-brom-phenyl)-methanon, das in das Hydrochlorid überführt wird, MS: m/e 430, ($M+H^+$, 1Br),

b) aus (E)-4-[4-(4-Brom-but-2-enyloxy)-3-fluor-benzoyl]-benzonitril, das (E)-4-[4-[4-(Cyclopropyl-methyl-amino)-but-2-enyloxy]-3-fluor-benzoyl]-benzonitril, das mit Fumarsäure in das Fumarat oder mit 4 M Salzsäure in Äther in das Hydrochlorid überführt wird, MS: m/e 365 ($M+H^+$, 1Br).

Beispiel 14

Aus (4-Brom-phenyl)-[4-[6-[(3-hydroxy-propyl)-amino]-hexyloxy]-phenyl]-methanon (Bsp. Ff) und anschließender N-Methylierung (analog Bsp 12c) erhält man das (4-Brom-phenyl)-[4-[6-[(3-hydroxy-propyl)-methylamino]-hexyloxy]-phenyl]-methanon, das in das Fumarat überführt wird, MS: m/e 448 ($M+H^+$, 1Br).

Beispiel 15

a) 10g [4-(6-Brom-hexyloxy)-phenyl]-(4-brom-phenyl)-methanon werden in 200ml DMF gelöst und nach Zugabe von 14,7g Natriumazid wird 24 Stunden bei 90°C gerührt, abfiltriert und die Filtrate werden unter vermindertem Druck konzentriert. Der Rückstand wird in 200ml Essigester aufgenommen und mit 10% wäßriger Natriumhydrogencarbonatlösung gewaschen. Die organische Phase wird getrocknet und eingeengt. Es werden 9.2g [4-(6-Azido-hexyloxy)-phenyl]-(4-brom-phenyl)-methanon erhalten, MS: m/e 401 ($M+H^+$, 1Br).

b) Zu einer Lösung aus 8,7g [4-(6-Azido-hexyloxy)-phenyl]-(4-brom-phenyl)-methanon in 100 ml Tetrahydrofuran-Wasser (4:1) werden 7,1g Triphenylphosphin gegeben, und das Gemisch wird 5 Stunden bei Raumtemperatur gerührt und anschließend eingedampft. Der Rückstand wird in Methylenchlorid aufgenommen und mit etherischer Salzsäurelösung versetzt. Das ausgefallene Hydrochlorid wird abfiltriert und mit Ether gewaschen. Es werden 8,0g [4-(6-Amino-hexyloxy)-phenyl]-(4-brom-phenyl)-methanon• Hydrochlorid erhalten, MS: m/e 376, ($M+H^+$, 1Br).

c) 1,1g [4-(6-Amino-hexyloxy)-phenyl]-(4-brom-phenyl)-methanon werden in 30ml Ether suspendiert und bei 4°C werden 1,25ml Trifluoressigsäureanhydrid langsam zugetropft. Dann wird das Eisbad entfernt und das Reaktiongemisch 24 Stunden bei Raumtemperatur weitergerührt, dann auf 100ml Wasser gegossen und mit gesättigter Natriumhydrogencarbonat-lösung neutralisiert. Die wäßrige Phase wird abgetrennt und mit Essigester extrahiert. Die organischen Phasen werden mit gesättigter Natriumchloridlösung gewaschen, getrocknet und eingedampft. Der Rückstand wird in Methylenchlorid aufgenommen und abfiltriert. Das Filtrat wird eingeengt, in Methylenchlorid gelöst und mit Hexan versetzt. Nach dem Abfiltrieren der ausgefallenen Kristalle werden 0,8g N-[6-[4-(4-Brom-benzoyl)-phenoxy)]-hexyl]-2,2,2-trifluoracetamid erhalten, MS: m/e 471 ($M+H^+$, 1Br).

d) Zu einer Suspension von 0,08g 55% igem Natriumhydrid in 20ml DMF werden bei -20°C 0,65g N-[6-[4-(4-Brom-benzoyl)-phenoxy)]-hexyl]-2,2,2-trifluoracetamid gegeben. Das Gemisch wird 30 Minuten gerührt und die Reaktiontemperatur auf Raumtemperatur gebracht. Anschließend wird 0,24g Methyliodid zugegeben und 1 Stunde bei Raumtemperatur weitergerührt. Das Reaktiongemisch wird mit gesättiger Ammoniumchloridlösung versetzt, mit IN Salzsäurelösung auf pH ~4 gestellt und mit Methylenchlorid extrahiert. Die organischen Auszüge werden getrocknet und eingeengt. Nach der Chromatographie des Rückstandes an Kieselgel mit Essigester-Hexan (2:8) werden 0,45g N-[6-[4-(4-Brom-benzoyl)-phenoxy)]-hexyl]-N-methyl-2,2,2-trifluoracetamid erhalten, MS: m/e 485 ($M+H^+$, 1Br).

e) Analog Beispiel 12b) erhält man aus N-[6-[4-(4-Brom-benzoyl)-phenoxy)]-hexyl]-N-methyl-2,2,2-trifluoracetamid das (4-Brom-phenyl)-[4-(6-methylamino-hexyloxy)-phenyl]-methanon, MS: m/e 389 ($M+H^+$, 1Br).

f) Ein Gemisch aus 0,1g (4-Brom-phenyl)-[4-(6-methylamino-hexyloxy)-phenyl]-methanon, 0,13ml Diisopropylethylamin und 0,06ml Brommethylcyclopropan in 20 ml Dimethylacetamid wird 24 bei 50°C gerührt, eingeengt, mit Natriumhydrogencarbonatlösung versetzt und mit Methylenchlorid extrahiert. Die organischen Phasen werden mit gesättigter Natriumchlorid-lösung gewaschen, getrocknet und eingedampft. Der Rückstand wird über Kieselgel mit $CH_2Cl_2/MeOH/NH_4OH$ (94/5,4/0,6 bis 85/12,5/2,5) gereinigt. 0,68g (4-Brom-phenyl)-[4-[6-(cyclopropylmethyl-methyl-amino)-hexyloxy]-phenyl]-methanon werden erhalten, die in das Fumarat überführt werden, MS: m/e 443 ($M+H^+$, 1Br).

Beispiel 16

Ausgangsverbindung

Analog Beispiel 12a erhält man aus (E)-[4-(4-Brom-but-2-enyloxy)-phenyl]-(4-brom-phenyl)-methanon und 2,2,2-Trifluoracetamid das N-[4-[4-(4-Brom-benzoyl)-phenoxy]-but-2-enyl]-2,2,2-trifluoracetamid, MS: m/e 441 (M, 1Br)

Produkt

Analog Beispiel 15 erhält man:

a) aus N-[4-[4-(4-Brom-benzoyl)-phenoxy]-but-2-enyl]-2,2,2-trifluoracetamid via N- [4- [4-(4-Brom-benzoyl)-phenoxy] -but-2-enyl] -N-methyl-2,2,2-trifluoracetamid und (4-Brom-phenyl)-[4-(4-methylamino-but-2-enyloxy)-phenyl]-methanon, das (E)-(4-Brom-phenyl)-[4-[4-(cyclopropylmethyl-methyl-amino)-but-2-enyloxy]-phenyl]-methanon, das in das Fumarat übergeführt wird, MS: m/e 413 ($M+H^+$, 1Br),

b) aus (4-Brom-phenyl)-[4-(6-methylamino-hexyloxy)-phenyl]-methanon und 1-Bromaceton in Dimethylacetamid bei Raumtemperatur, das 1-[[6-[4-(4-Brom-benzoyl)-phenoxy]-hexyl]-methyl-amino]-propan-2-on, das in das Fumarat übergeführt wird, MS: m/e 446 ($M+H^+$, 1Br).

Beispiel 17

Eine Suspension aus 0,4g (E)-[[4-[4-(4-Brom-benzoyl)-phenoxy]-but-2-enyl]-methyl-amino]-acetonitril (Bsp 2j) und 0,04g Kaliumcarbonat in 3ml Dimethylsulfoxid wird auf 0°C abgekühlt und mit 1 ml 30% Wasserstoffperoxidlösung versetzt. Das Reaktiongemisch wird auf Raumtemperatur erwärmt und über Nacht gerührt. Zur Aufarbeitung wird mit 10 ml Wasser versetzt, und die ausgefallenen Kristalle werden abfiltriert und mit Wasser gewaschen. Es werden 0,38g (E)-2-[[4-[4-(4-Brom-benzoyl)-phenoxy]-but-2-enyl]-methyl-amino]-acetamid erhalten, MS: m/e 372 ($M^+$-$H_2NCO$, 1Br).

Beispiel 18

Analog Beispiel 17 erhält man:

aus (E)-4-[4-[4-(Cyclopropyl-methyl-amino)-but-2-enyloxy]-3-fluorbenzoyl]-benzonitril (Bsp 13b) das (E)-4-[4-[4-(Cyclopropyl-methyl-amino)-but-2-enyloxy]-3-fluor-benzoyl]-benzamid, das in das Fumarat übergeführt wird, MS: m/e 383 ($M+H^+$, 1Br).

Beispiel 19

Eine Lösung von 0.92 ml Hexamethyldisilazan in 2.5 ml THF wird bei 0°C mit 2.63 ml 1.6 M Butyl-Lithium in Hexan tropfenweise versetzt. Nach 15 Min wird auf -78 °C gekühlt und 0.6 g (Bsp. Fa) (E)-1-[4-[4-(Allyl-methylamino)-but-2-enyloxy]-3-fluor-phenyl]-ethanon (Ha) in 1.9 ml THF zugetropft. Nach 1 Std bei -78 ° C wird 0.5 ml Geranylbromid in 1 ml THF zugetropft. Man lässt auf Raumtemperatur aufwärmen, rührt 1 Std und giesst auf gesättigte Natriumbicarbonat-Lösung/Äther. Die organische Phase wird mit 10% iger Natriumchlorid-Lösung gewaschen, getrocknet und eingedampft. Nach Kieselgelchromatographie mit (97.5%) Methylenchlorid/Methanol wird der Rückstand in Äthanol mit 0.87 g Fumarsäure gelöst, eingedampft und aus Äther mit Pentan gefällt. Es wird 0.24 g (E)-1-[4-[(E)-4-(Allyl-methyl-amino)-but-2-enyloxy]-3-fluor-phenyl]-2-[(E)-3,7-dimethylocta-2,6-dienyl]-5,9-dimethyl-deca-4,8-dien-1-on•Fumarat (1:2), MS: m/e 549 (M) erhalten.

Beispiel 20

Analog Beispiel 19 erhält man:

aus (E)-1-[4-[4-(Allyl-methyl-amino)-but-2-enyloxy]-3-fluor-phenyl]-ethanon (Bsp. Fa) und 3,3-Dimethylallylbromid, das (E)-1-[4-[4-(Allyl-methylamino)-but-2-enyloxy]-3-fluor-phenyl]-5-methyl-2-(3-methyl-but-2-enyl)-hex-4-en-1-on•Fumarat (1:1), MS: m/e 413 (M).

Beispiel 21

Eine Lösung von 0.86 ml Diisopropylamin in 5 ml THF wird bei 0 ° C mit 3.5 ml 1.6 M Butyllithium in Hexan tropfenweise versetzt. Nach 15 Min wird auf -78 ° C gekühlt und 1.1 g (E)-1-[4-[4-(Allyl-methyl-amino)-but-2-enyloxy]-3-fluor-phenyl]-ethanon (Bsp. Fa) in 3.9 ml THF zugetropft. Nach 1 Std bei -78 ° C wird 0.7 ml 3-Methyl-2-butenal in 0.8 ml THF zugetropft. Nach 20 Min bei -78 °C werden 0.86 ml Essigsäure in 4.6 ml Äther zugetropft und auf gesättigte Natriumbicarbonat-Lösung/Methylenchlorid gegossen. Die organische Phase wird getrocknet und eingedampft. Nach Kieselgelchromatographie mit (97.5%) Methylenchlorid/Methanol wird der Rückstand in Äthanol mit 0.24 g Fumarsäure gelöst, eingedampft und aus Methylenchlorid mit Essigester/Äther gefällt. Es wird 0.64 g (E)-(RS)-1-[4-[4-(Allyl-methyl-amino)-but-2-enyloxy]-3-fluor-phenyl]-3-hydroxy-5-methyl-hex-4-en-1-on•Fumarat (1:1), MS: m/e 362 ($M+H^+$) erhalten.

Beispiel 22

Analog Beispiel 21 erhält man:

a) aus (E)-1-[4-[4-(Allyl-methyl-amino)-but-2-enyloxy]-2-fluor-phenyl]-ethanon (Bsp. Fb) und 3-Methyl-2-butenal, das (E)-(RS)-1-[4-[4-(Allyl-methylamino)-but-2-enyloxy]-2-fluor-phenyl]-3-hydroxy-5-methyl-hex-4-en-1-on, MS: m/e 362 ($M+H^+$),

b) aus (E)-1-[4-[4-(Allyl-methyl-amino)-but-2-enyloxy]-phenyl]-ethanon (Bsp. Fc) und 3-Methyl-2-butenal, das (E)-(RS)-1-[4-[4-(Allyl-methyl-amino)-but-2-enyloxy]-phenyl]-3-hydroxy-5-methyl-hex-4-en-1-on•Fumarat (1:1), MS: m/e 344 ($M+H^+$),

c) aus (E)-1-[4-[4-(Allyl-methyl-amino)-but-2-enyloxy]-3 fluor-phenyl]-ethanon (Bsp. Fa) und (E)-Citral, das (E)-(RS)-1-[4-[(E)-4-(Allyl-methyl-amino)-but-2-enyloxy]-3-fluor-phenyl]-3-hydroxy-5,9-dimethyl-deca-4,8-dien-1-on•Fumarat (1:1), MS: m/e 430 ($M+H^+$).

Beispiel 23

Aus (E)-1-[4-[4-(Allyl-methyl-amino)-but-2-enyloxy]-3-fluor-phenyl]-ethanon (Bsp. Fa) und 11-Brom-1-undecanal erhält man analog Beispiel 21 das (E)-(RS)-1-[4-[4-(Allyl-methyl-amino)-but-2-enyloxy]-3-fluor-phenyl]-13-brom-3-hydroxy-tridecan-1-on. 0.53 g des erhaltenen Bromids werden in 3.4 ml DMA gelöst, bei 0 °C mit 0.19 ml N-Allyl-methyl-amin versetzt und nach 20 Std bei Raumtemperatur eingedampft. Der Rückstand wird in Methylenchlorid/gesättigter Natriumbicarbonat-Lösung aufgenommen, die organische Phase getrocknet und abfiltriert. Nach dem Eindampfen wird über Kieselgel mit Methylenchlorid /Methanol (95:5 bis 6:1)gereinigt. Die 0.39 g (E)-(RS)-13-(Allyl-methyl-amino)-1-[4-[4-(allyl-methyl-amino)-but-2-enyloxy]-3-fluor-phenyl]-3-hydroxy-tridecan-1-on werden mit Fumarsäure in das (E)-(RS)-13-(Allyl-methyl-amino)-1-[4-[4-(allyl-methyl-amino)-but-2-enyloxy] -3-fluor-phenyl] -3-hydroxy-tridecan-1-on•Fumarat (1:2) überführt, MS: m/e 517 ($M+H^+$).

Beispiel 24

Aus 1-[6-[6-(Allyl-methyl-amino)-hexyloxy]-pyridin-3-yl]-ethanon und 3-Methyl-2-butenal wird analog Beispiel 21 direkt das dehydratisierte (E)-1-[6-[6-(Allyl-methyl-amino)-hexyloxy]-pyridin-3-yl]-5-methyl-hexa-2,4-dien-1-on•Fumarat (1:1) erhalten, MS: m/e 356 ($M^+$).

Beispiel 25

Eine Lösung von 4.1 g (E)-(RS)-1-[4-[4-(Allyl-methyl-amino)-but-2-enyloxy]-3-fluor-phenyl]-3-hydroxy-5-methyl-hex-4-en-1-on (Bsp 21) in 280 ml Toluol wird zu 2.6 g p-Toluolsulfonsäure gegeben und während 2.5 Std bei Raumtemperatur gerührt. Das Reaktionsgemisch wird eingeengt, in Methylenchlorid gelöst, mit gesättigter Natriumbicarbonat-Lösung extrahiert, getrocknet, eingeengt und über Kieselgel mit 97.5% Methylenchlorid/Methanol gereinigt. Das freie Amin wird mit 0.75 g Fumarsäure in Methylenchlorid/Methanol gelöst, eingedampft und aus Essigester/Methanol/Äther umkristallisiert. Man erhält 2.77 g (E)-1-[4-[(E)-4-(Allyl-methyl-amino)-but-2-enyloxy]-3-fluor-phenyl]-5-methyl-hexa-2,4-dien-1-on•Fumarat (1:1), MS: m/e 344 ($M+H^+$).

Beispiel 26

Analog Beispiel 25 erhält man:

a) aus (E)-(RS)-13-(Allyl-methyl-amino)-1-[4-[4-(allyl-methyl-amino)-but-2-enyloxy]-3-fluor-phenyl]-3-hydroxy-tridecan-1-on (Bsp 23), das (E)-13-(Allylmethyl-amino)-1-[4-[(E)-4-(allyl-methyl-amino)-but-2-enyloxy]-3-fluorphenyl]-tridec-2-en-1-on•Fumarat (1:2), MS: m/e 499 ($M+H^+$),

b) aus (E)-(RS)-1-[4-[4-(Allyl-methyl-amino)-but-2-enyloxy]-2-fluor-phenyl]-3-hydroxy-5-methyl-hex-4-en-1-on (Bsp 22a), das (E)-1-[4-[(E)-4-(Allyl-methylamino)-but-2-enyloxy]-2-fluor-phenyl]-5-methyl-hexa-2,4-dien-1-on•Fumarat (1:1), MS: m/e 344 ($M+H^+$),

c) aus (E)-(RS)-1-[4-[4-(Allyl-methyl-amino)-but-2-enyloxy]-phenyl]-3-hydroxy-5-methyl-hex-4-en-1-on (Bsp 22b), das (E)-1-[4-[(E)-4-(Allyl-methyl-amino)-but-2-enyloxy]-phenyl]-5-methyl-hexa-2,4-dien-1-on•Fumarat (1:1), Smp. 120-123 ° C,

d) aus (E)-(RS)-1-[4-[(E)-4-(Allyl-methyl-amino)-but-2-enyloxy]-3-fluorphenyl]-3-hydroxy-5,9-dimethyl-deca-4,8-dien-1-on (Bsp 22c), das (2E,4E)-1-[4-[(E)-4-(Allyl-methyl-amino)-but-2-enyloxy]-3-fluor-phenyl]-5,9-dimethyl-deca-2,4,8-trien-1-on•Fumarat (1:1), MS: m/e 412 ($M+H^+$).

Beispiel 27

27.1. Ausgangsverbindung

Eine Lösung von 2.4 g 4-Brom-2-fluoracetanilid in 30 ml absolutem THF wird auf- 78 °C gekühlt und innert 20 Min mit 13.8 ml 1.6 M Butyllithium in Hexan versetzt. Nach 15 Min werden 2.6 g 4-Brom-N-methoxy-N-methylbenzamid (hergestellt aus 4-Brom-benzoylchlorid und N,O-Dimethyl-hydoxylamin-hydrochlorid mit N-Methylmorpholin) in 5 ml THF zugetropft. Nach 20 Std Rühren bei -78 ° C wird die Reaktionslösung auf kalte 10% ige Kaliumhydrogensulfat-Lösung/Äther gegossen, die organische Phase wird mit 10% ige Natriumchloridlösung gewaschen und getrocknet. Nach Kristallisation aus Methylenchlorid/Äther werden 1.2 g N-[4-(4-Brombenzoyl)-(2-fluor-phenyl)-acetamid erhalten, MS: m/e 335 (M, 1Br).

27.2. Produkt

5.0 g N-[4-(4-Brom-benzoyl)-(2-fluor-phenyl)-acetamid (Bsp 27.1) werden in THF mit 9.6 g (E)-1,4-Dibrombuten gelöst und bei -22 °C mit 1.3 g 55 % igem Natriumhydrid versetzt. Nach 30 Min wird über Nacht auf RT aufwärmen gelassen. Die Reaktion wird durch Zusatz von Essigester und Wasser beendet und das Reaktionsgemisch mit 10% iger Kaliumhydrogensulfat-Lösung/Essigester aufgearbeitet. Die organische Phase wird mit 10 % iger Natriumchlorid Lösung gewaschen, getrocknet und eingeengt. Nach Chromatographie über Kieselgel mit Methylenchlorid/Essigester (99:1 bis 98:2) werden 4.8 g (E)-N-(4-Brom-but-2-enyl)-N-[4-(4-bromo-benzoyl)-2-fluorphenyl]-acetamid erhalten, welches in 35 ml N,N-Dimethylacetamid gelöst bei Raumtemperatur mit 2.0 ml N-Allyl-methyl-amin versetzt wird. Nach 3 Std wird eingedampft, in Methylenchlorid aufgenommen und mit ges. Natriumbicarbonat-Lösung /10% iger Natriumchloridösung gewaschen, getrocknet und eingeengt. Nach Reinigung über Kieselgel mit Methylenchlorid/Methanol (0.2% bis 5%) wird in Methylenchlorid/Äther mit 4.8 M Salzsäure-Lösung in Äther versetzt und mit Essigester/Äther gefällt. Man erhält 3.5 g (E)-N-[4-(Allyl-methyl-amino)-but-2-enyl]-N-[4-(4-brom-benzoyl)-2-fluor-phenyl]-acetamid•Hydrochlorid (1:1), MS: m/e 458 (M, 1Br).

Beispiel 28

Analog Beispiel 27 erhält man:
aus N-[4-(4-Brom-benzoyl)-(2-fluor-phenyl)-acetamid (Bsp 27.1) mit 1,6-Dibromhexan, N- [6-(Allyl-methyl-amino)-hexyl] -N- [4-(4-bromo-benzoyl)-2-fluor-phenyl]-acetamid•Hydrobromid (1:1), MS: m/e 488 (M, 1Br).

Beispiel 29

2.0 g (E)-N-[4-(Allyl-methyl-amino)-but-2-enyl)-N-[4-(4-brom-benzoyl)-2-fluor-phenyl]-acetamid (Bsp 27) werden zu einer Lösung von 0.4 g KOH in Äthanol gegeben und während 5 Std unter Rückfluss gekocht. Nach Eindampfen wird der Rückstand in einer 10% igen Natriumchloridlösung in Essigester aufgenommen und die organische Phase getrocknet und eingeengt. Nach Reinigung über Kieselgel mit Methylenchlorid/Methanol (0.5% bis 2%) wird in Methylenchlorid gelöst und bei 0 °C mit einem Equivalent Salzsäure in Äther versetzt. Fällen mit Essigester/Äther ergibt 1.2 g (E)-[4-[4-(Allyl-methyl-amino)-but-2-enylamino]-2-fluor-phenyl]-(4-brom-phenyl)-methanon•Hydrochlorid (1:1), MS: m/e 416 (M, 1Br).

Beispiel 30

Analog Beispiel 29 erhält man:
aus N- [6-(Allyl-methyl-amino)-hexyl] -N- [4-(4-bromo-benzoyl)-2-fluorphenyl]-acetamid (Bsp 28), das [4-[6-(Allyl-methyl-amino)-hexylamino]-3-fluor-phenyl]-(4-brom-phenyl)-methanon•Hydrochlorid (1:1), MS: m/e 446 (M, 1Br).

Beispiel 31

31.1. Ausgangsverbindungen

A.a) Zu einer Lösung von 5.0 g (1RS,2RS)-1,2-Cyclopropandicarbonsäurediäthylester in 9 ml Methanol wird 13.4

ml 2 M Kaliumhydroxid in Methanol zugetropft. Nach 2.5 Std wird mit 8% iger Phosphorsäure angesäuert und mit gesättigter Natriumchlorid-Lösung/Methylenchlorid extrahiert, getrocknet und eingeengt, wobei man 5.1 g (1RS, 2RS)-1,2-Cyclopropandicarbonsäure-monomethylester erhält.

A.b) Eine Lösung von 9.3 g (1RS,2RS)-1,2-Cyclopropandicarbonsäuremonomethylester, 4.0 ml N-Cyclopropanamin und 11.6 g N-(3-Dimethylaminopropyl)-N'-äthylcarbodiimide-hydrochlorid in 210 ml Methylenchlorid wird bei 0 °C mit 0.7 g Dimethylaminopyridin versetzt und anschliessend 2 Std bei Raumtemperatur gerührt. Die Reaktionslösung wird mit Methylenchlorid/10%iger Kaliumhydrogensulfat-Lösung aufgearbeitet. Die organische Phase wird mit gesättigter Natriumbicarbonat-Lösung gewaschen, getrocknet und eingeengt, wobei man 10.6 g (1RS, 2RS)-2-Cyclopropylcarbamoyl-cyclopropancarbonsäure-methylester erhält.

A.c) Eine Lösung von 4.9 g (1RS,2RS)-2-Cyclopropylcarbamoyl-cyclopropancarbonsäure-methylester und 10.8 ml Methyliodid in 120 ml 1,2-Dimethoxyäthan wird bei 0 °C mit 1.2 g 55% igem Natriumhydrid behandelt und während 22 Std bei 0 °C gerührt. Nach Zugabe von Wasser wird eingedampft und mit 10% iger Kaliumhydrogensulfatlösung/Äther /Äther extrahiert, mit gesättigter Natriumchloridlösung gewaschen und die organische Phase getrocknet.

A.d) Der rohe (1RS,2RS)-2-(Cyclopropyl-methyl-carbamoyl)-cyclopropancarbonsäure-methylester wird in 9 ml THF gelöst und in eine siedende Suspension von 1.4g Lithiumaluminiumhydrid in 40 ml THF getropft. Das Reaktionsgemisch wird weitere 24 Std gekocht, auf 0 °C gekühlt und mit 9 ml Wasser versetzt, getrocknet, abfiltriert und eingeengt. Das Öl wird in Methylenchlorid gelöst, getrocknet und eingeengt, wobei man 4.2 g (1RS,2RS)-[2-[(Cyclopropyl-methyl-amino)-methyl]-cyclopropyl]-methanol erhält, MS: m/e 156 ($M+H^+$).

B) Analog Beispiel 31.1.A) erhält man:

B.a) aus (1RS,2RS)-1,2-Cyclopropandicarbonsäure-diäthylester, via (1RS,2RS)-1,2-Cyclopropandicarbonsäure-monomethylester und (1RS,2RS)-2-Allyl- methylcarbamoyl-cyclopropancarbonsäure-methylester, das (1RS, 2RS)-[2-[(Allyl-methyl-amino)-methyl]-cyclopropyl]-methanol, MS: m/e 156 ($M+H^+$),

B.b) aus (1RS,2RS)-1,2-Cyclopropandicarbonsäure-diäthylester, via (1RS,2RS)-1,2-Cyclopropandicarbonsäure-monomethylester und (1RS,2RS)-2-Äthyl-methylcarbamoyl-cyclopropancarbonsäure-methylester, das (1RS, 2RS)-[2-[(Äthyl-methyl-amino)-methyl]-cyclopropyl]-methanol,

B.c) aus (1RS,2RS)-1,2-Cyclopropandicarbonsäure-diäthylester, via (1RS,2RS)-1,2-Cyclopropandicarbonsäure-monomethylester, (1RS,2RS)-2-Cyclopropylmethylcarbamoyl-cyclopropancarbonsäure-methylester und (1RS, 2RS)-2-(Cyclopropylmethyl-methyl-carbamoyl)-cyclopropancarbonsäure-methylester, das (1RS,2RS)-[2-[(Cyclopropylmethyl-methyl-amino)-methyl] -cyclopropyl] -methanol,

B.d) aus (1RS,2RS)-1,2-Cyclopropandicarbonsäure-diäthylester, via (1RS,2RS)-1,2-Cyclopropandicarbonsäure-monomethylester, (1RS,2RS)-2-Cyclopropylcarbamoyl-cyclopropancarbonsäure-methylester und (1RS,2RS)-2-(Allyl-cyclopropyl-carbamoyl)-cyclopropancarbonsäure-methylester, das (1RS,2RS)-[2- [(Allyl-cyclopropyl-amino)-methyl] -cyclopropyl] -methanol.

31.2. Produkt

Eine Lösung von 6.2 g Triphenylphosphin, 6.5 g (4-Brom-phenyl)-(4-hydroxy-phenyl)-methanon und 3.64 g (1RS,2RS)-[2-[(Cyclopropyl-methylamino)-methyl]-cyclopropyl]-methanol (Bsp 31.1.A.d) in 190 ml THF wird bei Raumtemperatur während 1 Std mit 4.03 ml Diäthylazodicarboxylat in 18 ml THF behandelt. Nach 3 Std Nachrühren wird eingeengt, in Äther aufgenommen und mit gesättigter Natriumbicarbonat-Lösung und 10% iger Natriumchlorid-Lösung gewaschen, getrocknet und eingeengt. Der Rückstand wird in Äther gelöst und mit Hexan gefällt. Die Mutterlauge wird eingeengt und das rohe (1RS,2RS)-(4-Brom-phenyl)-[4-[2-[(cyclopropylmethyl-amino)-methyl]-cyclopropylmethoxy]-phenyl]-methanon in Äthanol gelöst, mit 2.7 g Fumarsäure versetzt und auskristallisiert. Man erhält 4.1 g (1RS,2RS)-(4-Brom-phenyl)-[4-[2-[(cyclopropyl-methyl-amino)-methyl]-cyclopropylmethoxy]-phenyl]-methanon•Fumarat (1:1), Smp. von 136-137 °C.

Kristallisation von (1RS,2RS)-(4-Brom-phenyl)-[4-[2-[(cyclopropylmethyl-amino)-methyl]-cyclopropylmethoxy]-phenyl]-methanon aus Methylenchlorid mit 4 M Salzsäure in Äther ergibt (1RS,2RS)-(4-Bromphenyl)- [4- [2-[(cyclopropyl-methyl-amino)-methyl] -cyclopropylmethoxy]-phenyl]-methanon•Hydrochlorid (1:1), Smp. von 93-95 °C.

Beispiel 32

Analog Beispiel 31.2 erhält man:

a) aus (1RS,2RS)-[2-[(Cylcopropyl-methyl-amino)-methyl]-cyclopropyl]methanol (Bsp 31.1.A.d) und (4-Brom-phenyl)-(3-fluor-4-hydroxy-phenyl)-methanon, das (1RS,2RS)-(4-Brom-phenyl)-[4-[2-[(cyclopropyl-methylamino)-methyl]-cyclopropylmethoxy]-3-fluor-phenyl]-methanon•Fumarat (1:1), Smp. 152-154 °C,

b) aus (1RS,2RS)-[2-[(Cylcopropyl-methyl-amino)-methyl]-cyclopropyl]-methanol (Bsp 31.1.A.d) und (3-Fluor-4-hydroxy-benzoyl)-benzonitril , das (1RS,2RS)-4-[4-[2-[(Cyclopropyl-methyl-amino)-methyl]-cyclopropylmethoxy]-3-fluor-benzoyl]-benzonitril •Fumarat (1:1), Smp. 116-117 °C,

c) aus (1RS,2RS)-[2-[(Cyclopropylmethyl-methyl-amino)-methyl]-cyclopropyl]-methanol (Bsp 31.1.Bc) und (4-Brom-phenyl)-(4-hydroxyphenyl)-methanon, das (1RS,2RS)-(4-Brom-phenyl)-[4-[2-[(cyclopropylmethyl-methyl-amino)-methyl]-cyclopropylmethoxy]-phenyl]-methanon•Fumarat (1:1), Smp. 128-131 °C,

d) aus (1RS,2RS)-[2-[(Cylcopropyl-methyl-amino)-methyl]-cyclopropyl]-methanol (Bsp 31.1.A.d) und (4-Brom-phenyl)-(2-fluor-4-hydroxy-phenyl)-methanon, das (1RS,2RS)-(4-Brom-phenyl)-[4-[2-[(cyclopropyl-methylamino)-methyl] -cyclopropylmethoxy] -2-fluor-phenyl]-methanon •Fumarat (1:1), Smp. 133-135 °C,

e) aus (1RS,2RS)-[2-[(Allyl-cyclopropyl-amino)-methyl]-cyclopropyl]-methanol (Bsp 31.1.Bd) und (4-Brom-phenyl)-(4-hydroxy-phenyl)-methanon, das (1RS,2RS)-[4-[2-[(Allyl-cyclopropyl-amino)-methyl]-cyclopropylmethoxy]-phenyl]-(4-brom-phenyl)-methanon•Hydrochlorid (1:1), Smp. 120-122°C,

f) aus (1RS,2RS)-[2-[(Cylcopropyl-methyl-amino)-methyl]-cyclopropyl]-methanol (Bsp 31.1.A) und 1-(4-Hydroxy-phenyl)-5-methyl-hexan-1-on, das (1RS,2RS)-1-[4-[2-[(Cyclopropyl-methyl-amino)-methyl]-cyclopropylmethoxy]-phenyl]-5-methyl-hexan-1-on•Hydrochlorid (1:1), Smp. 110-112 °C,

g) aus (1RS,2RS)-[2-[(Äthyl-methyl-amino)-methyl]-cyclopropyl]-methanol (Bsp 31.1.Bb) und (4-Brom-phenyl)-(4-hydroxy-phenyl)-methanon, das (1RS,2RS)-(4-Brom-phenyl)-[4-[2-[(ethyl-methyl-amino)-methyl]-cyclopropylmethoxy]-phenyl]-methanon•Fumarat (1:1), Smp. 136-138 °C,

h) aus (1RS,2RS)-[2-[(Allyl-methyl-amino)-methyl]-cyclopropyl]-methanol (Bsp 31.1.Ba) und (4-Brom-phenyl)-(4-hydroxy-phenyl)-methanon, das (1RS,2RS)-[4-[2-[(Allyl-methyl-amino)-methyl]-cyclopropylmethoxy]-phenyl]-(4-brom-phenyl)-methanon, welches in das Hydrochlorid überführt wird, Smp. 112 °C (unter Zersetzung).

Beispiel 33

Eine Lösung von 2.6 g [4-[6-(Allyl-methyl-amino)-hexyloxy]-2-fluorphenyl]-(4-brom-phenyl)-methanon in 115 ml Dimethylacetamid wird mit 7.2 ml 8.03M Methylamin in Ethanol während 3 Std bei 120 °C gekocht, eingeengt und über Kieselgel mit Methylenchlorid/ Methanol (2.5%-10%) chromatographiert. Das erhaltene Öl wird in Methylenchlorid gelöst und über Nacht mit 0.5 g Fumarsäure gerührt, wobei man 1.5 g [4-[6-(Allylmethyl-amino)-hexyloxy]-2-methyl-amino-phenyl]-(4-brom-phenyl)-methanon•Fumarat, Smp. von 72 °C erhält.

Beispiel 34

Analog Beispiel 33 erhält man:

a) aus [4-[6-(Allyl-methyl-amino)-hexyloxy]-2-fluor-phenyl]-(4-brom-phenyl)-methanon mit Dimethylamin, [4-[6-(Allyl-methyl-amino)-hexyloxy]-2-dimethylamino-phenyl]-(4-brom-phenyl)-methanon•Hydrochlorid (1:2), MS: m/e 473 ($M+H^+$, 1Br),

b) aus [4-[6-(Allyl-methyl-amino)-hexyloxy]-2-fluor-phenyl]-(4-brom-phenyl)-methanon mit 1,2,4-Triazol, [4-[6-(Allyl-methyl-amino)-hexyloxy]-2-1H-[1,2,4]triazol-1-yl-phenyl]-(4-brom-phenyl)-methanon•Hydrochlorid (1:1), MS: m/e 496 (M, 1Br),

c) aus 1-[4-[6-(Allyl-methyl-amino)-hexyloxy]-2-fluor-phenyl]-5-methyl-hex-5-en-1-on (Bsp. F.e) mit Methylamin in Ethanol, das 1-[4-[6-(Allyl-methylamino)-hexyloxy]-2-methylamino-pheny]-4-methyl-hex-5-en-1-on, MS: m/e 487

$(M+H^+)$,

d) aus (E)-1-[4-[4-(Allyl-methyl-amino)-but-2-enyloxy]-2-fluor-phenyl]-5-methyl-hex-4-en-1-on (Bsp. Fd) mit Natrium-thiomethanolat in THF, (E)-1-[4-[4-(Allyl-methyl-amino)-but-2-enyloxy]-2-methylsulfanyl-phenyl]-5-methyl-hex-4-en-1-on•Fumarat (1:1), MS: m/e 374 $(M^+)$.

Beispiel 35

Analog Beispiel 33 wird 8.97 g [4-[6-(Allyl-methyl-amino)-hexyloxy]-2-fluor-phenyl]-(4-brom-phenyl)-methanon in 450 ml THF mit 37 ml 5.4M Natrium-methanolat in Methanol 14 Std bei Raumtemperatur und 1 Std unter Rückfluss gerührt. Die Lösung wird eingedampft und in Methylenchlorid/10%iger Natriumchlorid-Lösung aufgenommen. Die organische Phase wird getrocknet, in Äther gelöst und mit 2.08 g Fumarsäure über Nacht gerührt. Man erhält 8.17 g [4-[6-(Allyl-methyl-amino)-hexyloxy]-2-methoxy-phenyl]-(4-brom-phenyl)-methanon•Fumarat (Smp. 108-113 °C).

Das erhaltene Fumarat wird in Methylenchlorid/gesättigter Natriumbicarbonat-Lösung aufgenommen, die organische Phase wird getrocknet und eingeengt. 3.09 g des so erhaltenen [4-[6-(Allyl-methyl-amino)-hexyloxy]-2-methoxy-phenyl]-(4-brom-phenyl)-methanon werden in 13 ml Essigsäure/7.7 ml 62%iger HBr-Lösung 2 Std bei 90 °C gekocht. Das Reaktionsgemisch wird eingeengt und mit Methylenchlorid/gesättigter Natriumbicarbonat-Lösung in die freie Base umgewandelt. Der Rückstand wird mit 0.74 g Fumarsäure versetzt und aus Äthanol/Äther ausgeölt. Es werden 2.05 g [4-[6-(Allyl-methyl-amino)-hexyloxy]-2-hydroxy-phenyl]-(4-brom-phenyl)-methanon ·Fumarat erhalten, MS: m/e 446 $(M+H^+$, 1Br).

Beispiel 36

Analog Beispiel 33 werden 17.55 g [4-[6-(Allyl-methyl-amino)-hexyloxy]-2-fluor-phenyl]-(4-brom-phenyl)-methanon mit 50.7 ml 4-Methoxybenzylamin und 6.5 g Kaliumcarbonat in 600 ml Toluol 23 Std unter Rückfluss gekocht. Nach Abfiltrieren, Eindampfen und Reinigung über Kieselgel mit Methylenchlorid/Methanol (2.5% bis 10%) werden 17.43 g [4-[6-(Allyl-methylamino)-hexyloxy]-2-(4-methoxy-benzylamino)-phenyl]-(4-brom-phenyl)-methanon erhalten. Eine Lösung von diesem Material in 200 ml Trifluoressigsäure wird während 45 Std bei Raumtemperatur gerührt, eingedampft und mit Methylenchlorid/gesättigter Natriumbicarbonat-Lösung in die frei Base überführt. Nach Reinigung über Kieselgel mit Methylenchlorid/ Methanol (9:1) werden 13.23 g [2-Amino-4-[6-(allyl-methyl-amino)-hexyloxy]-phenyl]-(4-brom-phenyl)-methanon erhalten.

9.1 g der freien Base werden in Methylenchlorid/Äther gelöst, mit 2.25 g Fumarsäure und durch Rühren über Nacht in 7.28 g [2-Amino-4-[6-(allylmethyl-amino)-hexyloxy]-phenyl]-(4-brom-phenyl)-methanon•Fumarat (1:1) überführt, Smp. von 78 °C (unter Zersetzung).

Beispiel 37

Eine Lösung von 0.9 g [2-Amino-4-[6-(allyl-methyl-amino)-hexyloxy]-phenyl]-(4-brom-phenyl)-methanon (Bsp. 36) wird in 20 ml Methylenchlorid bei 0°C mit 0.17 ml Methansulfonylchlorid in 0.8 ml Methylenchlorid und mit 24,4 mg Dimethylaminopyridin versetzt. Die Reaktion wird über Nacht auf Raumtemperatur aufwärmen gelassen, eingeengt und in Methylenchlorid/ gesättigter Natriumbicarbonat-Lösung aufgenommen. Die organische Phase wird getrocknet und eingeengt. Reinigung über Kieselgel mit Methylenchlorid/Methanol (95:5) als Eluens ergibt 0.57 g N-[5-[6-(Allylmethyl-amino)-hexyloxy]-2-(4-brom-benzoyl)-phenyl]-methansulfonamid, welches ins Fumarat überführt wird, MS: m/e 523 $(M+H^+$, 1 Br).

Beispiel 38

2.8 g Cer(III)chlorid werden getrocknet, dann in 45 ml THF aufgenommen und während 1 Std bei Raumtemperatur gerührt. Bei -78 °C werden dann 7.2 ml 1.6M Methyllithium in Äther zugegeben und nach einer weiteren Std 4.0 g [4-[6-(Allyl-methyl-amino)-hexyloxy]-2-fluor-phenyl]-(4-brom-phenyl)-methanon in 18 ml THF zugetropft. Nach 2.5 Std bei -78 °C und 1 Std bei 0 °C wird mit gesättigter Ammoniumchlorid-Lösung/Methylenchlorid aufgearbeitet. Die organische Phase wird getrocknet und eingeengt. Der Rückstand wird mit 0.9 g Fumarsäure in Äthanol gelöst, wobei man nach dem Eindampfen 5.1 g (RS)-1-[4-[6-(Allyl-methyl-amino)-hexyloxy]-2-fluor-phenyl]-1-(4-brom-phenyl)-ethanol•Fumarat (1:1) erhält, MS: m/e 463 (M, 1Br).

Beispiel 39

Analog Beispiel 38 erhält man:

a) aus (E)-[4-[4-(Allyl-methyl-amino)-but-2-enyloxy]-phenyl]-5-methyl-hex-4-en-1-on, das (E)-(RS)-2-[4-[4-(Allyl-methyl-amino)-but-2-enyloxy]-phenyl]-6-methyl-hept-5-en-2-ol •Fumarat (1:1), MS: m/e 344 ($M+H^+$),

b) aus (E)-[4-[4-(Allyl-methyl-amino)-but-2-enyloxy]-3-fluor-phenyl]-(4-bromphenyl)-methanon und Vinylmagnesiumchlorid, das (E)-(RS)-1-[4-[4-(Allylmethyl-amino)-but-2-enyloxy]-3-fluor-phenyl]-(4-brom-phenyl)-prop-2-en-1-ol•Fumarat (1:1), MS: m/e 446 ($M+H^+$, 1Br),

c) aus [4-[(Allyl-methyl-amino)-methyl]-biphenyl-4-yl]-(4-bromo-phenyl)-methanon und Methylmagnesiumchlorid-Lösung, das (RS)-1-[4-[(Allylmethyl-amino)-methyl]-biphenyl-4-yl]-1-(4-bromo-phenyl)-ethanol, das in das Fumarat übergeführt wird, MS: m/e 436 ($M+H^+$, 1Br).

Beispiel 40

Zu 4.8 ml (1.7 M in THF) Vinylmagnesiumchlorid-Lösung werden bei 0°C 1.0g [4-[6-(Allyl-methyl-amino)-hexyloxy]-2-hydroxy-phenyl]-(4-bromophenyl)-methanon (Bsp 35) in 9 ml THF/Äther (1:1) während 45 Min getropft. Die Reaktionslösung wird über Nacht auf Raumtemperatur aufwärmen gelassen, mit 3 ml Essigsäure/Wasser (1:1) versetzt und mit gesättigter Natriumbicarbonat-Lösung/Methylenchlorid aufgearbeitet. Nach dem Trocknen der organischen Phase wird eingeengt und über Kieselgel mit Methylenchlorid/Methanol (95:5) gereinigt. Man erhält 0.64 g (RS)-5-[6-(Allyl-methyl-amino)-hexyloxy]-2-[1-(4-brom-phenyl)-1-hydroxy-allyl]-phenol, MS: m/e 474 ($M+H^+$, 1Br).

Beispiel 41

Analog Beispiel 40 erhält man:
aus [2-Amino-4-[6-(allyl-methyl-amino)-hexyloxy]-phenyl]-(4-bromphenyl)-methanon (Bsp 36), das (RS)-1-[4-[6-(Allyl-methyl-amino)-hexyloxy]-2-amino-phenyl]-1-(4-brom-phenyl)-prop-2-en-1-ol, MS: m/e 473 ($M+H^+$, 1Br).

Beispiel 42

42.1. Ausgangsverbindungen

A) 3.58 g (E)-4-Brom-2-buten-1-ol werden in 100 ml Aceton gelöst und mit 9.84 g $K_2CO_3$ und 3.4 ml N-Allyl-methyl-amin versetzt. Die Suspension wird 40h bei RT gerührt, filtriert und das nach dem Einengen erhaltene Rohprodukt an Kieselgel mit Methylenchlorid:Methanol (Gradient 9:1 - 3:1) gereinigt. Es werden 1.52 g (E)-4-(Allyl-methyl-amino)-but-2-en-1-ol als farblose Flüssigkeit erhalten, MS: m/e 141 (M).

B) 1 ml 1-Brom-6-hexanol werden in DMA aufgenommen, mit 1.47 ml N-Allyl-methyl-amin versetzt und 16 h bei RT gerührt. Das Reaktionsgemisch wird eingeengt und über Nacht lyophilisiert. Es werden 1.9 g 6-(Allylmethyl-amino)-hexan-1-ol•Hydrobromid als Rohprodukt erhalten, MS: m/e 171 (M).

C) 10 g 6-Chlor-pyridin-2-carbonsäure werden in 50 ml Thionylchlorid gelöst und 3.5 h unter Rückfluß erhitzt. Die Lösung wird abgekühlt und unter vermindertem Druck vom überschüßigen Thionylchlorid befreit. Das Rohprodukt wird in 60 ml Methylenchlorid aufgenommen und mit 7.22g N,O-Dimethylhydroxylamin•Hydrochlorid versetzt. Unter Eiskühlung werden 25 ml $NEt_3$ in 30 ml $CH_2Cl_2$ hinzugegeben und die Lösung wird 1.5 h bei RT gerührt, die Suspension wird filtriert und das Filtrat mit verdünnter Natronlauge und gesättigter Natriumchloridlösung gewaschen und getrocknet. Nach dem Einengen der Lösung werden 13.12 g 6-Chlor-pyridin-2-carbonsäure-methoxy-methyl-amid als Rohprodukt erhalten. 6.7 g von diesem Amid in 40 ml THF werden bei -78°C zu einer Lösung aus 40 ml n-BuLi (1.6 M in Hexan) und 15 g 1,4-Dibrombenzol in 140 ml THF getropft. Die Lösung wird 2h bei -78°C, 1h bei 0°C gerührt und dann mit 60 ml 2M HCl versetzt. Die Phasen werden getrennt, die anorganische Phase wird mit 50 ml Ether extrahiert, die organischen Phasen werden mit ges. $NaHCO_3$-Lösung und gesättigter Natriumchloridlösung gewaschen und getrocknet. Das Rohprodukt wird über Kieselgel mit Essigester:Hexan (6:1) gereinigt und aus Essigester/ Hexan kristallisiert. Es werden 6.75 g (4-Brom-phenyl)-(6-chlor-pyridin-3-yl)-methanon, Smp von 127.4°C erhalten, MS: m/e 295 (M, 1Br).

D) Analog Bsp. 42.1.C) erhält man:

a) aus 6-Chlor-pyridin-2-carbonsäure, via 6-Chlor-pyridin-2-carbonsäure-methoxy-methyl-amid mit Methylmagnesiumbromid in THF, das 1-(6-Chlor-pyridin-3-yl)-ethanon, MS: m/e 155 (M),

b) aus 5-Chlor-pyridin-2-carbonsäure, via 5-Chlor-pyridin-2-carbonsäure-methoxy-methyl-amid mit 1,4-Dibrombenzol/n-Butyllithium, das (4-Bromphenyl)-(5-chlor-pyridinyl-2-yl)-methanon, Smp. 117.5-119.5°C, MS: m/e 296 (M, 1Br).

42.2. Produkt

1.11 g (4-Brom-phenyl)-(6-chlor-pyridin-3-yl)-methanon (Bsp 42.1.C), 696 mg 6-(Allyl-methyl-amino)-hexan-1-ol (Bsp 42.1.B), 880 mg KOH, 552 mg $K_2CO_3$ und 200 mg Dicyclohexano-[18]krone-6 werden unter Argon in 50 ml Toluol gelöst und über Nacht auf 80°C erhitzt. Die Suspension wird erneut mit 323 mg 6-(Allyl-methyl-amino)-hexan-1-ol versetzt und weitere 5h erhitzt. Das Reaktionsgemisch wird mit $H_2O$ versetzt und mit $CH_2Cl_2$ extrahiert. Die organischen Phasen werden mit ges. $NaHCO_3$-Lösung und ges. Natriumchloridlösung gewaschen und getrocknet. Das erhaltene Rohprodukt wird in $CH_2Cl_2$:MeOH (95:5) chromatographiert. Es werden 1.02 g gelbbraunes Öl [6-[6-(Allyl-methyl-amino)-hexyloxy]-pyridin-3-yl]-(4-bromphenyl)-methanon erhalten, die in 15 ml Ethanol gelöst und mit 261 mg Fumarsäure in 5 ml Ethanol versetzt werden. Die Lösung wird 1h bei RT gerührt, eingeengt und das Öl mehrfach abgedampft und lyophilisiert. Es werden 1.2 g 6-[6-(Allyl-methyl-amino)-hexyloxy]-pyridin-3-yl]-(4-bromphenyl)-methanon•Fumarat (1:1) als gelbes Öl erhalten, MS: m/e 430 (M).

Beispiel 43

Analog Beispiel 42.2 erhält man:

a) aus (4-Brom-phenyl)-(6-chlor-pyridin-3-yl)-methanon (Bsp 42.1.C) und (E)-4-(Allyl-methyl-amino)-but-2-en-1-ol (Bsp 42.1.A) in Gegenwart von KOH und $K_2CO_3$ inToluol, das (E)-[6-[4-(Allyl-methyl-amino)-but-2-enyloxy]-pyridin-3-yl]-(4-brom-phenyl)-methanon•Fumarat (1:1) erhalten, MS: m/e 401 ($M^+$, 1Br),

b) aus (4-Brom-phenyl)-(5-chlor-pyridinyl-2-yl)-methanon (Bsp 42.1.Db) und (Allyl-methyl-amino)-hexan-1-ol (Bsp 42.1.B) in Gegenwart von KOH, $K_2CO_3$ und Dicyclohexano-[18]krone-6 in Toluol, das [5-[6-(Allyl-methyl-amino)-hexyloxy]-pyridin-2-yl]-(4-brom-phenyl)-methanon•Fumarat (1:1), MS: m/e 431 ($M+H^+$, 1Br).

Beispiel 44

a) Aus 22.6 g 4-Brombiphenyl wird mit 2.36 g Magnesium in 75 ml THF die entsprechende Grignard-Verbindung hergestellt. Zu dieser Lösung tropft man eine Lösung von 8.0g 5-Methoxy-2H-3,4-dihydropyrrol in 25ml THF. Das Gemisch wird 5 Std. am Rückfluss gekocht, dann auf eine gesättigte Lösung von Ammoniumchlorid gegossen, mit Essigester extrahiert, getrocknet und eingeengt. Der Rückstand wird aus Isopropanol umkristallisiert, wodurch 1.93g 5-Biphenylyl-2H-3,4-dihydropyrrol isoliert werden, Smp. 230 °C.

b) 1.85g 5-Biphenylyl-2H-3,4-dihydropyrrol wird in 50ml Methanol gelöst und mit 0.38g Natriumborhydrid versetzt. Es wird eine Stunde bei Raumtemperatur gerührt, dann eingeengt, mit Wasser versetzt, mit Essigester extrahiert, getrocknet und eingeengt. Man isoliert so 1.48g 2-Biphenylylpyrrolidin, Smp. 50-53 °C.

c) 1.40g 2-Biphenylyl-pyrrolidin werden in 40ml Methanol gelöst und mit 1.5ml einer 36%igen wässrigen Formaldehyd-Lösung versetzt. Das Gemisch wird mit 0.38g Natriumborhydrid versetzt und 15 Min im Eisbad gerührt, eingeengt, mit Essigester extrahiert, getrocknet und eingeengt. Der Rückstand wird in wenig Aether gelöst und mit einer ges. Lösung von HCl-Gas in Aether versetzt. Der farblose Niederschlag wird abgenutscht und getrocknet. Man isoliert 1.57g N-Methyl-2-biphenylyl-pyrrolidin-hydrochlorid.

d) 350mg des N-Methyl-2-biphenylyl-pyrrolidin-hydrochlorids werden in 10ml Schwefelkohlenstoff aufgeschlämmt und mit 341mg Aluminiumchlorid versetzt. Zu dieser klebrigen Masse wird eine Lösung von 842 mg 4-Brom-benzoylchlorid in 3ml Schwefelkohlenstoff zugetropft. Das Gemisch wird 2 Stunden am Rückfluss gehalten und eingedampft. Der Rückstand wird in Toluol, dann in Essigester verrührt, wobei man 90 mg (±)(4-Bromphenyl)-[4'-(1-methylpyrrolidin-2-yl)-biphenyl-4-yl]-methanon•Hydrochlorid, Smp. 225 °C erhält.

Beispiel 45

a) Aus 16g 4-Bromtoluol und 2.23g Magnesium in 150ml THF wird die entsprechende Grignard-Verbindung hergestellt. Diese wird tropfenweise bei Raumtemperatur zu einer Lösung von 10g 2-Bromthiophen, 3g Palladiumacetat und 1.27g Triphenylphosphin in 150 ml THF gegeben. Das Gemisch wird 3.5 Std unter Argon am Rückfluss gekocht, dann auf Eiswasser gegossen und mit Essigester extrahiert. Chromatographie an Kieselgel (Toluol) ergibt 9.06g 2-p-Tolyl-thiophen.

b) Aus 1.0 g 2-p-Tolyl-thiophen, 840mg Aluminiumchlorid und 1.46g 4-Bromobenzoylchlorid in 20ml Schwefelkohlenstoff erhält man in einer Friedel-Crafts-Reaktion nach Chromatographie an Kieselgel (Methylenchlorid) 1.02g (4-Bromphenyl)-(5-p-tolyl-thiophen-2-yl)-methanon, Smp. 172-174°C.

c) 0.5g (4-Bromphenyl)-(5-p-tolyl-thiophen-2-yl)-methanon und 262 mg N-Bromsuccinimid in 20ml Tetrachlorkohlenstoff werden nach Zugabe einer Spatelspitze Azaisobutyronitril 19 Std. am Rückfluss gehalten, eingeengt und an Kieselgel chromatographiert (Methylenchlorid/Hexan). Man isoliert 517 mg [5-(4-Bromomethyl-phenyl)-thiophen- 2-yl]-4-bromophenyl-methanon, Smp. 184 °C (Zersetzung).

d) 435mg [5-(4-Brommethyl-phenyl)-thiophen- 2-yl]-4-bromophenylmethanon werden in 25ml Aceton gelöst, 300 mg Kaliumcarbonat und 0.15ml N-Allyl-methyl-amin zugesetzt und das Gemisch wird unter Argon 4 Std. am Rückfluss gehalten, mit Eiswasser versetzt, mit Essigester extrahiert, getrocknet und an Kieselgel (Essigester/Hexan) chromatographiert. Man isoliert 219 mg 5-(4-[(Allyl-methylamino)-methyl]-phenyl)-thiophen-2-yl)-(4-bromphenyl)-methanon, Smp. 131-133 °C.

Beispiel 46

Analog zu Beispiel 45 erhält man:

a) aus [5-(4-Brommethyl-phenyl)-thiophen- 2-yl]-4-brom-phenyl-methanon mit Dimethylamin in Äthanol, das 5-(4-[Dimethylamino)-methyl]-phenyl)-thiophen-2-yl)-(4-brom-phenyl)-methanon, Smp. von 149-152°,

b) aus 2-p-Tolyl-thiophen und 2,4-Difluor-benzoylchlorid, via (2,4-Difluorphenyl)-5-p-tolyl-thiophen-2-yl)-methanon und [5-(4-Brom-methyl-phenyl)-thiophen-2-yl]-4-(2,4-difluor-phenyl)-methanon mit N-Allyl-methyl-amin, das 5-(4-[(Allyl-methylamino)-methyl]-phenyl)-thiophen-2-yl)-(4-(2,4-difluorophenyl))-methanon, Smp. von 82-84 °C,

c) aus 2-p-Tolyl-thiophen und 2,4-Difluor-benzoylchlorid, via (2,4-Difluorphenyl)-5-p-tolyl-thiophen-2-yl)-methanon und [5-(4-Brommethyl-phenyl)-thiophen-2-yl]-4-(2,4-difluor-phenyl)-methanon mit Kaliumcarbonat und Dimethylaminlösung in Aethanol, das (2-Dimethylamino-4-fluorphenyl)-[5-(4-dimethylaminomethyl-phenyl)-thiophen-2-yl]-methanon, MS: m/e 382 (M)

Beispiel 47

47.1. Ausgangsverbindung

Analog Beispiel 45 erhält man aus 4-Brom-toluol und 3-Brom-anisol, via 3-Methoxy-4'-methyl-biphenyl, (4-Brom-phenyl)-(3-methoxy-4'-methylbiphenyl-4-yl)-methanon und(4'-Brommethyl-3-methoxy-biphenyl-4-yl)-(4-brom-phenyl)-methanon mit Dimethylamin in Äthanol, das (4-Bromphenyl)-(4'-dimethylaminomethyl-3-methoxy-biphenyl-4-yl)-methanon, Smp. des Hydrochlorids 252-255°C.

47.2. Produkt

700 mg (4-Brom-phenyl)-(4'-dimethylaminomethyl)-3-methoxy-biphenyl-4-yl)-methanon werden in 15ml einer 62 proz. HBr Lösung 18 Std. am Rückfluss gehalten, mit Eiswasser versetzt, mit Essigester extrahiert und an Kieselgel chromatographiert (Methylenchlorid/Methanol). Man isoliert 205mg (4-Bromophenyl)-(4'-dimethylaminomethyl-3-hydroxy-biphenyl-4-yl)-methanon, Smp. 85-88 °C.

Beispiel 48

Eine Lösung von 211 mg (RS)-[4'-[(Allyl-methyl-amino)-methyl]-biphenyl-4-yl]-(4-bromo-phenyl)-methanol wird in 3 ml Methylenchlorid bei -78 °C mit 0.065 ml Diäthylamino-schwefeltrifluorid behandelt. Nach 3 Std bei Raumtemperatur wird noch einmal auf -78 °C gekühlt und 0.065 ml Diäthylamino-schwefeltrifluorid zugegeben. Nach 16 Std bei Raumtemperatur wird auf eiskalte gesättigte Natriumhydrogencarbonat-Lösung gegossen und mit Methylenchlorid extrahiert. Nach Chromatographie über Kieselgel mit Methylenchlorid/Methanol (2%-5%), Auflösen des Rückstandes in Äthanol und Umsetzung mit 108 mg Fumarsäure erhält man (RS)-Allyl-[4'-[(4-brom-phenyl)-fluor-methyl]-biphenyl-4-ylmethyl]-methylamin•Fumarat (1:1), MS: m/e 423 (M, 1Br).

Beispiel 49

49.1. Ausgangsverbindung

a) Ein Gemisch aus 7g (4-Hydroxy)-piperidin-1-carbonsäure-tert-butyl ester und 210 ml 1,6-Dibromhexan wird zuerst mit 3.5g Tetrabutylammoniumhydrogensulfat und dann mit 210ml 50% wäßriger Natronlauge versetzt. Nach 5 tägigem Rühren bei Raumtemperatur wird das Reaktiongemisch mit Methylenchlorid verdünnt, die organische Phase abgetrennt und die wäßrige Phase mit Methylenchlorid extrahiert. Die vereinigten organischen Phasen werden mit gesättigter Natriumchloridlösung gewaschen, getrocknet und eingeengt. Das braune Öl wird auf Kieselgel mit Essigster-Hexan chromatigraphiert . Es werden 10,9g 4-(6-Brom-hexyloxy)-piperidin-1-carbonsäure-tert-butylester erhalten.

b) Der erhaltene Ester wird mit 2,9ml N-Allyl-methyl-amin, 4.1g Kaliumcarbonat und 30 ml Aceton versetzt. Das Gemisch wird 2 Tage bei Raumtemperatur gerührt, abfiltriert und eingedampft. Anschließend wird die Boc-gruppe mit 34ml Trifluoressigsäure in 100ml Methylenchlorid abgespalten. Nach Einengen, azeotropen Destillation der Trifluoressigsäure mit Toluol und Trocknen werden 14g Allyl-methyl-[6-(piperidin-4-yloxy)-hexyl]-amin erhalten. MS: m/e 255 ($M+H^+$).

49.2. Produkt

3g Allyl-methyl-[6-(piperidin-4-yloxy)-hexyl]-amin (Bsp. 49.1) werden in 50ml Methylenchlorid gelöst und mit 5,3ml Hünig-Base (Di-isopropyl-äthylamin) und 1,9g 4-Brom-phenylsulfonylchlorid versetzt. Das Reaktionsgemisch wird 3 Stunden bei Raumtemperatur gerührt, mit wäßriger Natriumhydrogencarbonat-Lösung versetzt und mit Methylenchlorid extrahiert. Die organischen Phasen werden mit gesättigter Natriumchloridlösung gewaschen, getrocknet und eingeengt. Das braune Öl wird an Kieselgel mit Essigester/Methanol (9:1) gereinigt. Es werden 2,6g Allyl-[6-[1-(4-brom-phenylsulfonyl)-piperidin-4-yloxy]-hexyl]-methyl-amin erhalten, die in das Fumarat überführt werden, MS: m/e 473 ($M+H^+$, 1Br).

Beispiel 50

Analog Beispiel 49.2 erhält man

a) aus Allyl-methyl-[6-(piperidin-4-yloxy)-hexyl]-amin und 4-Brombenzoylchlorid, das [4-[6-(Allyl-methyl-amino)-hexyloxy]-piperidin-1-yl]-1-(4-bromphenyl) methanon, das mit 4 M Salzsäure-Lösung in Äther in das Hydrochlorid überführt wird, MS: m/e 437 ($M+H^+$, 1Br).

b) aus Allyl-methyl-[6-(piperidin-4-yloxy)-hexyl]-amin und 4-Bromphenacylbromid, das 2-[4-[6-(Allyl-methyl-amino)-hexyloxy]-piperidin-1-yl]-1-(4-brom-phenyl)-ethanon, das mit 4 M Salzsäure-Lösung in Äther in das 2-[4-[6-(Allyl-methyl-amino)-hexyloxy]-piperidin-1-yl]-1-(4-brom-phenyl)-ethanon•Hydrochlorid (1:2) überführt wird, MS: m/e 451 ($M+H^+$, 1Br).

Beispiel 51

Ein Gemisch aus 3,0g [4-[(E)-4-(Allyl-methyl-amino)-but-2-enyloxy]-phenyl] -(4-brom-phenyl)-methanon•Hydrobromid, 0.77g O-Methylhydroxylamin•Hydrochlorid und 1,07g Natriumacetat in 100 ml Ethanol wird 3 Tage unter Rückfluß erhitzt. Das Reaktionsgemisch wird eingeengt, mit 100 ml gesättigter wäßriger NaHCO3-Lösung versetzt

und mit Essigester extrahiert. Die organischen Auszüge werden mit gesättigter Natriumchlorid-lösung gewaschen, getrocknet und eingedampft. Der Rückstand wird über Kieselgel mit Essigester-Hexan-Triethylamin (39: 60 : 1) gereinigt. Es werden 2,22g (E)- und/oder (Z)-[4-[(E)-4-(Allyl-methyl-amino)-but-2-enyloxy]-phenyl]-(4-brom-phenyl)-methanon O-methyl-oxim erhalten, die mit mit 103 ml 0.05M Fumarsäurelösung versetzt werden. Nach Rühren wird die Lösung lyophilisiert. Es werden (E)- und/oder (Z)-[4-[(E)-4-(Allyl-methylamino)-but-2-enyloxy]-phenyl]-(4-brom-phenyl)-methanon-O-methyloxim•Fumarat (1:1) als Öl erhalten. MS: m/e 429 ($M+H^+$, 1Br).

Beispiel 52

Analog Beispiel 51 erhält man:

a) aus [4-[(E)-4-(Allyl-methyl-amino)-but-2-enyloxy]-phenyl]-(4-brom-phenyl)-methanon•Hydrobromid und Hydroxylamin•Hydrochlorid, das (E)-und/oder (Z)-[4-[(E)-4-(Allyl-methyl-amino)-but-2-enyloxy]-phenyl]-(4-bromphenyl)-methanon-oxim, MS: m/e 414 ($M^+$, 1Br),

b) aus [4-[(E)-4-(Allyl-methyl-amino)-but-2-enyloxy]-phenyl]-(4-brom-phenyl)-methanon•Hydrobromid und O-tert-Butylhydroxylamin•Hydrochlorid, das (E)- und/oder (Z)-[4-[(E)-4-Allyl-methyl-amino)-but-2-enyloxy]-phenyl-(4-brom-phenyl)-methanon-O-tert-butyl-oxim, das in das Fumarat überführt wird, MS: m/e 397 [M- $OC(CH_3)_3$, 1 Br],

c) aus [4-[(E)-4-(Allyl-methyl-amino)-but-2-enyloxy]-phenyl]-(4-brom-phenyl)-methanon•Hydrobromid und O-Allylhydroxylamin•Hydrochlorid, das (E)-und/oder (Z)-[4-[(E)-4-(Allyl-methyl-amino)-but-2-enyloxy]-phenyl]-(4-brom-phenyl)-methanon-O-allyl-oxim, das ins Fumarat überführt wird, MS: m/e 455 ($M+H^+$, 1 Br),

d) aus [4-[6-(Allyl-methyl-amino)-hexyloxy]-2-fluor-phenyl]-(4-brom-phenyl)-methanon•Hydrobromid und Hydroxylamin•Hydrochlorid, das (E)-und/oder (Z)-[4-[6-(Allyl-methyl-amino)-hexyloxy]-2-fluor-phenyl]-(4-bromphenyl)-methanon-oxim, das ins Fumarat überführt wird, MS: m/e 463 ($M+H^+$, 1 Br).

Beispiel 53

a) Eine Lösung von 10.7 g 1,4-Dibrombenzol in 80 ml absolutem THF wird auf -78 °C gekühlt und innert 30 Min mit 25.6 ml 1.6 M Butyllithium in Hexan versetzt. Nach 30 Min werden 6.08 g (4E,8E)-(RS)-4,8-Dimethyl-10-(tetrahydro-pyran-2-yloxy)-deca-4,8-dienal in 20 ml THF zugetropft. Nach 1 Std bei - 78 ° C wird das Bad entfernt, bis die Suspension sich auflöst, bei -78 °C werden anschliessend 7.2 ml Essigsäure in 10 ml Äther zugetropft. Die Lösung wird auf gesättigte Ammoniumchlorid-Lösung/Essigester gegossen. Die organische Phase wird mit gesättigter Natriumbicarbonat-Lösung und 10% iger Natriumchlorid-Lösung gewaschen, getrocknet und eingedampft. Nach Kieselgelchromatographie mit Hexan/Essigester 95:5 werden 4.3 g (4E,8E)-1-(4-Brom-phenyl)-4,8-dimethyl-10-(tetrahydro-pyran-2-yloxy]-deca-4,8-dien-1-ol erhalten.

b) Eine Lösung von 3.4 g (4E,8E)-1-(4-Brom-phenyl)-4,8-dimethyl-10-(tetrahydro-pyran-2-yloxy]-deca-4,8-dien-1-ol in 60 ml Methylenchlorid werden nacheinander mit 0.29 g Natriumcarbonat und 7.5 g Mangan(IV)-oxid behandelt. Nach 3 Std Rühren wird abfiltriert und noch einmal in Methylenchlorid mit 0.29 g Natriumcarbonat und 7.5 g Mangan(IV)-oxid versetzt. Nach Filtration und Kieselgelchromatographie mit Hexan/Essigester (9:1) werden 1.98 g (4E,8E)-(RS)-1-(4-Brom-phenyl)-4,8-dimethyl-10-(tetrahydro-pyran-2-yloxy]-deca-4,8-dien-1-on erhalten.

c) Eine Lösung von 1.98 g (4E,8E)-(RS)-1-(4-Brom-phenyl)-4,8-dimethyl-10-(tetrahydro-pyran-2-yloxy]-deca-4,8-dien-1-on in 4 ml Methylenchlorid wird unter Argon bei - 50 °C mit 2.22 g Triphenylphosphindibromid versetzt. Nach 10 Min wird auf 0 °C aufwärmen gelassen, dann bei 0 °C gerührt und eingedampft. Der Rückstand wird in 16 ml Dimethylacetamid gelöst und bei 0 °C mit 0.87 ml N-Allyl-methyl-amin tropfenweise versetzt. Dann wird das Lösungsmittel abgezogen, der Rückstand in Methylenchlorid/gesättigter Natriumbicarbonat aufgenommen, die organische Phase getrocknet, eingeengt und über Kieselgel mit Methylenchlorid/Methanol (2.5% bis 5%) gereinigt. Reines (4E,8E)-10-(Allyl-methyl-amino)-1-(4-brom-phenyl)-4,8-dimethyl-deca-4,8-dien-1-on wird mit 0.17 g Fumarsäure in Äthanol gelöst, eingeengt und aus Essigester mit Pentan ausgeölt. Man erhält 0.78 g (4E,8E)-10-(Allyl-methyl-amino)-1-(4-brom-phenyl)-4,8-dimethyl-deca-4,8-dien-1-on•Fumarat (1:1), MS: m/e 404 ($M+H^+$, 1Br).

Beispiel 54

Analog Beispiel 53 erhält man:

a) aus (4E,8E)-(RS)-4,8-Dimethyl-10-(tetrahydro-pyran-2-yloxy)-deca-4,8-dienal, via (4E,8E)-1-(4-Brom-phenyl)-4,8-dimethyl-10-(tetrahydro-pyran-2-yloxy]-deca-4,8-dien-1-ol und (4E,8E)-(RS)-1-(4-Brom-phenyl)-4,8-dimethyl-10-(tetrahydro-pyran-2-yloxy]-deca-4,8-dien-1-on mit Dimethylamin, das (4E,8E)-1-(4-Brom-phenyl)-10-dimethylamino-4,8-dimethyl-deca-4,8-dien-1-on•Fumarat (1:1), MS: m/e 377 (M, 1Br).

b) aus (4E,8E)-(RS)-4,8-Dimethyl-10-(tetrahydro-pyran-2-yloxy)-deca-4,8-dienal mit Magnesium 4-methyl-pent-3-enyl bromid, via (9E,13E)-2,9,13-Trimethyl-15-(tetrahydro-pyran-2-yloxy)-pentadeca-2,9,13-trien-6-ol und (9E,13E)-(RS)-2,9,13-Trimethyl-15-(tetrahydro-pyran-2-yloxy)-pentadeca-2,9,13-trien-6-on, das (9E,13E)-15-(Allyl-methyl-amino)-2,9,13-trimethylpentadeca-2,9,13-trien-6-on•Fumarat (1:1), MS: m/e 331 (M).

c) aus (2E,6E)-(RS)-2,6-Dimethyl-8-(tetrahydro-pyran-2-yloxy)-octa-2,6-dienal mit Magnesium 4-methyl-pent-3-enyl bromid, via (7E,11E)-2,7,11-Trimethyl-13-(tetrahydro-pyran-2-yloxy)-trideca-2,7,11-trien-6-ol und (7E,11E)-(RS)-2,7,11-Trimethyl-13-(tetrahydro-pyran-2-yloxy)-trideca-2,7,11-trien-6-on, das (7E,11E)-13-(Allyl-methyl-amino)-2,7,11-trimethyl-trideca-2,7,11-trien-6-on•Fumarat (1:1), MS: m/e 303 (M).

d) aus (2E,6E)-(RS)-2,6-Dimethyl-8-(tetrahydro-pyran-2-yloxy)-octa-2,6-dienal mit Magnesium 4-methyl-pent-3-enyl bromid, via (7E,llE)-2,7,11-Trimethyl-13-(tetrahydro-pyran-2-yloxy)-trideca-2,7,11-trien-6-ol und (7E,11E)-(RS)-2,7,11-Trimethyl-13-(tetrahydro-pyran-2-yloxy)-trideca-2,7,11-trien-6-on, das (7E,11E)-13-(Allyl-methyl-amino)-2,7,11-trimethyl-trideca-2,7,11-trien-6-on, das nach Behandlung mit 4M Salzsäure in Äther ein Gemisch aus (7E,11E)-und (7Z,11E)-13-(Allyl-methyl-amino)-2,7,11-trimethyl-trideca-2,7,11-trien-6-on•Hydrochlorid (1:1) erhalten, MS: m/e 303 (M).

e) aus (2E,6E)-(RS)-2,6-Dimethyl-8-(tetrahydro-pyran-2-yloxy)-octa-2,6-dienal, via (2E,6E)-(RS)-1-(4-Brom-phenyl)-2,6-dimethyl-8-(tetrahydro-pyran-2-yloxy)-octa-2,6-dien-1-ol und (2E,6E)-(RS)-1-(4-Bromo-phenyl)-2,6-dimethyl-8-(tetrahydro-pyran-2-yloxy)-octa-2,6-dien-1-on, das (2E,6E)-8-(Allyl-methylamino)-1-(4-bromo-phenyl)-2,6-dimethyl-octa-2,6-dien-1-on•Fumarat (1:1), MS: m/e 376 ($M+H^+$, 1Br).

f) aus (2E,6E)-(RS)-2,6-Dimethyl-8-(tetrahydro-pyran-2-yloxy)-octa-2,6-dienal mit Magnesium pent-4-enyl bromid, via (7E,11E)-(RS)-7,11-Dimethyl-13-(tetrahydro-pyran-2-yloxy)-trideca-1,7,11-trien-6-ol und (7E, 11E)-(RS)-7,11-Dimethyl-13-(tetrahydro-pyran-2-yloxy)-trideca-1,7,11-trien-6-on, das (7E,11E)-13-(Allyl-methyl-amino)-7,11-dimethyl-trideca-1,7,11-trien-6-on•Hydrochlorid (1:1), MS: m/e 289 (M).

Beispiel 55

Aus (2E,6E)-(RS)-2,6-Dimethyl-8-(tetrahydro-pyran-2-yloxy)-octa-2,6-dienal erhält man via (2E,6E)-1-(4-Brom-phenyl)-2,6-dimethyl-8-(tetrahydropyran-2-yloxy)-octa-2,6-dien-1-ol (ohne Oxidation), das (2E,6E)-(RS)-8-(Allylmethyl-amino)-1-(4-brom-phenyl)-2,6-dimethyl-octa-2,6-dien-1-ol•Fumarat (1:1), MS: m/e 376 (M-H, 1Br).

Beispiel 56

Eine Lösung von 188 mg (2E,6E)-8-(Allyl-methyl-amino)-1-(4-bromphenyl)-2,6-dimethyl-octa-2,6-dien-1-on in 4 ml Benzol wird mit einer Lösung von 210 mg Natriumhydrogencarbonat, 61 mg Tricaprylylmethylammoniumchlorid und 245 mg Natriumdithionit in 4ml Wasser versetzt und 20 Min bei 80 °C gekocht. Es werden nocheinmal 245 mg Natriumdithionit zugegeben und nach weiteren 20 Min bei 80 °C, mit Wasser/Äther aufgearbeitet. Der Rückstand wird über Kieselgel mit Methylenchlorid/ Methanol (2%) gereinigt, in Äthanol mit 22 mg Fumarsäure versetzt und eingeengt. Man erhält 102 mg (E)-(RS)-8-(Allyl-methyl-amino)-1-(4-bromphenyl)-2,6-dimethyl-oct-6-en-1-on•Fumarat (1:1), MS: m/e 376 (M-H, 1Br).

Beispiel 57

57.1. Ausgangsverbindungen

A.

a) Eine Lösung von 105 g 80% ige Natriumchlorit und 100 g Natriumdihydrogenphosphat in 1l Wasser wird zu 50 g (4E,8E)-(RS)-4,8-Dimethyl-10-(tetrahydro-pyran-2-yloxy)-deca-4,8-dienal in 1.51 tert-Butanol und 1.5 l 2-Methyl-2-buten innert 30 Min zugegeben. Nach 2 1/2 Std bei Raumtemperatur wird eingeengt und der Rückstand in Methylenchlorid aufgenommen, mit Eiswasser und 10% iger Kaliumhydrogensulfat-Lösung gewaschen und getrocknet. Man erhält 46.5 g (4E,8E)-(RS)-4,8-Dimethyl-10-(tetrahydro-pyran-2-yloxy])-deca-4,8-diensäure. Die erhaltene Säure wird in 590 ml Methylenchlorid gelöst und bei 0 °C mit 14.9ml N-Allyl-methyl-amin, 32.0 g N-(3-Dimethylaminopropyl)-N'-äthylcarbodiimide-hydrochlorid und 1.88 g Dimethylaminopyridin versetzt. Nach 3 Std bei Raumtemperatur und Aufarbeiten mit Methylenchlorid/10%iger Kaliumhydrogensulfat und anschliessend gesättigter Natriumhydogencarbonat-Lösung wird die organische Phase getrocknet und eingeengt. Man erhält 52.3 g rohes (4E,8E)-(RS)-4,8-Dimethyl-10-(tetrahydro-pyran-2-yloxy)-deca-4,8-diensäure allyl-methylamid.

b) Das Allyl-methylamid wird in 15 ml Methanol gelöst und zu einer Suspension von 80 g eines sauren Ionenaustauschers in 550 ml Methanol getropft. Nach 5 Min Rühren wird abfiltriert, eingeengt und über Kieselgel mit Methylenchlorid/Methanol /Methanol (9:1) gereinigt. Es werden 29.1 g (4E,8E)-10-Hydroxy-4,8-Dimethyl-deca-4,8-diensäure-allyl-methylamid erhalten. 23,6 g des rohen Amids werden in 20 ml THF gelöst und so zu 3.5 g Lithiumaluminiumhydrid in 165 ml THF getropft, dass die Temperatur nicht über 28 °C steigt. Das Reaktionsgemisch wird nach 2 Std mit 10 ml Wasser versetzt, getrocknet, abfiltriert und eingeengt. Das Öl wird in 10% iger Kaliumhydrogensulfat/Äther aufgenommen, die Wasserphase mit gesättigter Natriumcarbonat-Lösung auf pH 10 gestellt und mit Methylenchlorid extrahiert. Nach Trocknung und Einengen der organischen Phase erhält man 6.9 g (4E,8E)-10-(Allylmethyl-amino)-3,7-dimethyl-deca-2,6-dien-1-ol, MS: m/e 234 (M-OH).

c) Der erhaltene Alkohol wird in 130 ml Toluol gelöst und mit 22g Mangan(IV)-oxid versetzt. Dann wird abfiltriert und noch zweimal in Toluol mit Mangan(IV)-oxid versetzt. Nach Filtration erhält man 5.5 g (2E,6E)-10-(Allylmethyl-amino)-3,7-dimethyl-deca-2,6-dienal.

B) Analog Beispiel 57.1.A) erhält man
aus (2E,6E)-(RS)-2,6-Dimethyl-8-(tetrahydro-pyran-2-yloxy)-octa-2,6-dienal via (2E,6E)-(RS)-2,6-Dimethyl-8-(tetrahydro-pyran-2-yloxy)-octa-2,6-diensäure allyl-methylamid und (2E,6E)-8-(Allyl-methyl-amino)-3,7-dimethyl-octa-2,6-dien-1-ol, das (2E,6E)-8-(Allyl-methyl-amino)-3,7-dimethyl-octa-2,6-dienal, welches direkt eingesetzt wird.

57.2. Produkt

Eine Lösung von 6,4 g 1,4-Dibrombenzol in 50 ml absolutem THF wird auf - 78 °C gekühlt und innert 30 Min mit 15.3 ml 1.6 M Butyllithium in Hexan versetzt. Nach 1 Std werden 3.0 g (2E,6E)-10-(Allyl-methyl-amino)-3,7-dimethyl-deca-2,6-dienal (Bsp 57.1.A) in 12 ml THF zugetropft. Nach 1 1/2 Std bei - 78 ° C wird das Bad entfernt, bis die Suspension sich auflöst.Bei -78 °C werden anschliessend 4.5 ml Essigsäure in 6 ml Äther zugetropft . Dann wird auf gesättigte Ammoniumchlorid-Lösung/Essigester gegossen. Die organische Phase wird mit gesättigter Natriumbicarbonat-Lösung und 10%iger Natriumchlorid-Lösung gewaschen, getrocknet und eingedampft. Nach Kieselgelchromatographie mit Methylenchlorid/Methanol (95:5) werden 1.14 g (2E,6E)-(RS)-10-(Allyl-methyl-amino)-1-(4-bromo-phenyl)-3,7-dimethyl-deca-2,6-dien-1-ol erhalten. Dieses wird in Äthanol mit 0.29 g Fumarsäure gelöst, eingedampft und mit Essigester/Äther zu 0.91g (2E,6E)-(RS)-10-(Allyl-methyl-amino)-1-(4-brom-phenyl)-3,7-dimethyl-deca-2,6-dien-1-ol•Fumarat (1:1) umgesetzt, MS: m/e 406 ($M+H^+$, 1Br).

Beispiel 58

Analog Beispiel 57.2. erhält man:
aus (2E,6E)-8-(Allylmethyl-amin)-3,7-dimethyl-octa-2,6-dienal (Bsp 57.1.B) und Butyllithium/1,4-Dibrombenzol, das (2E,6E)-(RS)-8-(Allyl-methylamino)-1-(4-brom-phenyl)-3,7-dimethyl-octa-2,6-dien-1-ol•Fumarat (1:1), MS: m/e 378 ($M+H^+$, 1Br).

Beispiel 59

Eine Lösung von 162 mg (2E,6E)-(RS)-10-(Allyl-methyl-amino)-1-(4-brom-phenyl)-3,7-dimethyl-deca-2,6-dien-1-ol (Bsp 57.2) in 6 ml Methylenchlorid wird nacheinander mit 28 mg Natriumcarbonat und 730 mg Mangan (IV)-oxid behandelt. Nach 2 Std Rühren wird abfiltriert, eingeengt und in Äthanol mit 28 mg Fumarsäure gelöst, eingedampft und mit Essigester/ Äther gefällt. Es werden 87 mg (2E,6E)-10-(Allyl-methyl-amino)-1-(4-brom-phenyl)-3,7-dimethyl-deca-2,6-dien-1-on•Fumarate (1:1) , MS: m/e 404 ($M+H^+$, 1Br) erhalten.

Beispiel 60

Analog Beispiel 59 erhält man:
aus (2E,6E)-(RS)-8-(Allyl-methyl-amino)-1-(4-brom-phenyl)-3,7-dimethyl-octa-2,6-dien-1-ol (Bsp 58), das (2E,6E)-8-(Allyl-methyl-amino)-1-(4-brom-phenyl)-3,7-dimethyl-octa-2,6-dien-1-on•Fumarat (1:1) , MS: m/e 376 ($M+H^+$, 1Br).

Beispiel 61

Eine Lösung von 230 mg [2-Amino-4-[6-(allyl-methyl-amino)-hexyloxy]-phenyl]-(4-brom-phenyl)-methanon (Bsp 36) in 0.5 ml Ameisensäure und 2 ml Formamid wird während 5 Min bei 165 °C gekocht, am Kugelrohr bei 170°C/ 0.1 Torr eingeengt und mit Methylenchlorid/gesättigter Natriumbicarbonat-Lösung in das freie Amin überführt. Nach der Reinigung über Kieselgel mit Methylenchlorid/Methanol (2.5%) als Eluens werden 30 mg N-[5-[6-(Allyl-methyl-amino)-hexyloxy]-2-(4-brom-benzoyl)-phenyl]-formamid erhalten, MS: m/e 473 ($M+H^+$, 1Br).

Pharmazeutische Anwendungsformen folgender Zusammensetzung können in an sich bekannter Weise hergestellt werden:

Beispiel A

Tabletten mit 5 mg (E)-(RS)-1-[4-[4-(Allyl-methyl-amino)-but-2-enyloxylphenyl]-(4-brom-phenyl)-ethanol als Wirkstoff

| Zusammensetzung: | 1 Tablette enthält: |
|---|---|
| Wirkstoff | 5,0 mg |
| Milchzucker | 148,0 mg |
| Kartoffelstärke | 65,0 mg |
| Magnesiumstearat | 2,0 mg |
| | 220,0 mg |

Beispiel B

Dragées mit 5 mg (E)-(RS)-1-[4-[4-(Allyl-methyl-amino)-but-2-enyloxylphenyl]-(4-brom-phenyl)-ethanol

Die Tabletten von Bsp. A werden nach bekanntem Verfahren mit einer Hülle überzogen, die im wesentlichen aus Zucker und Talkum besteht. Die fertigen Dragées werden mit Hilfe von Bienenwachs poliert.

| | |
|---|---|
| Dragéegewicht: | 300 mg |

Beispiel C

Suppositorien mit 5 mg (E)-(RS)-1-[4-[4-(Allyl-methyl-amino)-but-2-enyloxy]-phenyl]-(4-brom-phenyl)-ethanol als Wirkstoff

| Zusammensetzung: | 1 Zäpfchen enthält: |
|---|---|
| Wirkstoff | 5,0 mg |

(fortgesetzt)

| Zusammensetzung: | 1 Zäpfchen enthält: |
|---|---|
| Zäpfchenmasse (z.B. Witepsol W 45® ) | 1695,0 mg<br>1700,0 mg |

Beispiel D

Kapseln mit 5 mg (E)-(RS)-1-[4-[4-(Allyl-methyl-amino)-but-2-enyloxylphenyl]-(4-brom-phenyl)-ethanol als Wirkstoff

| Zusammensetzung: | 1 Kapsel enthält: |
|---|---|
| Wirkstoff | 5,0 mg |
| Lactose | 82,0 mg |
| Stärke | 82,0 mg |
| Magnesiumstearat | 1,0 mg |
| | 170,0 mg |

**Patentansprüche**

**1.** Tertiäre Amine der Formel

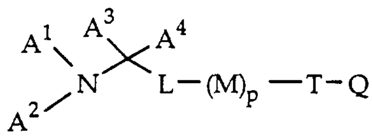

I

worin

| | |
|---|---|
| $A^1$ | Alkyl oder Alkenyl und |
| $A^2$ | Cycloalkyl, Cycloalkyl-alkyl oder eine gegebenenfalls durch eine Gruppe $R^1$, $CONH_2$ oder CN substituierte Alkyl- oder Alkenylgruppe und, falls $A^1$ Alkyl ist, $A^2$ auch OH sein kann, |
| $A^3$ und $A^4$ | Wasserstoff oder Alkyl sind oder |
| $A^1$ zusammen mit $A^2$ oder mit $A^3$ | eine gegebenenfalls durch $R^1$ substituierte Alkylen-, Alkenylen- oder Alkadienylengruppe $A^1$-$A^2$ bzw. $A^1$-$A^3$ mit bis zu 5 C-Atomen bildet, wobei in einer Gruppe $A^1$-$A^2$ oder $A^1$-$A^3$ bis zu 2 C-Atome durch ein (oder zwei) N-Atom(e) und/oder durch eine Gruppe N-Alkyl ersetzt sein können, |
| $R^1$ | an einem gesättigten C-Atom von $A^2$, $A^1$-$A^2$ oder $A^1$-$A^3$ gebundenes OH, Oxo, Alkyl (O oder S) oder Dialkylamino ist, wobei ein durch $R^1$ substituiertes C-Atom bzw. ein in $A^1$, $A^2$, $A^1$-$A^2$ oder $A^1$-$A^3$ enthaltenes ungesättigtes C-Atom in einer anderen als der $\alpha$-Stellung zu $N(A^1A^2)$ gebunden sein sollen, |
| p = 1 und L | Phenylen oder direkt oder über O, NH oder N(Alkyl oder Alkanoyl) an M gebundenes Alkylen oder Alkenylen mit insgesamt bis zu 11 C-Atomen und zumindest 4 bzw. 3 C-Atomen zwischen den zwei freien Valenzen oder Cycloalkylen-alkylen oder |
| p = 0 und L an T | gebundenes $C_{6-11}$-Alkenylen oder $C_{6-11}$-Alkadienylen, |
| M | Thienylen, Pyridylen, 1,4-Phenylen, durch einen oder mehreren Substituenten aus der Gruppe von Alkyl, Halogen, $N(R^2,R^{21})$, $CONH_2$, CN, $NO_2$, $CF_3$, OH, Alkyl (O oder S), 1,2,4-Triazol-1-yl oder Tetrazol-1-yl substituiertes 1,4-Phenylen oder eine Gruppe der Formel |

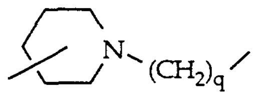 ($M^1$)

q — 1 oder 0,

$R^2$ und $R^{21}$ — H, Alkyl, Alkenyl, Alkanoyl oder $SO_2$-Alkyl,

T — CO, $CH(R^3)$, $C(R^4,R^5)$ oder $C{=}NOR^6$ und, falls M eine Gruppe $M^1$ und q = 0 ist, T auch $SO_2$ sein kann,

$R^3$ — OH, F, Alkoxy oder Alkanoyloxy,

$R^4$ OH und $R^5$ — Alkyl, Alkenyl, Alkinyl, Cycloalkyl oder $CF_3$ oder

$R^4$ zusammen mit $R^5$ — die Gruppe $CH_2$, $CH_2O$ oder $CH_2CH_2$,

$R^6$ — H, Alkyl oder Alkenyl,

Q — Cycloalkyl, $C(R^7,R^8)$, durch einen oder mehreren Substituenten aus der Gruppe von Alkyl, Halogen, $N(R^9,R^{10})$, $CONH_2$, CN, $NO_2$, $CF_3$, 1,2,4-Triazol-1-yl und Tetrazol-1-yl substituiertes Phenyl oder eine geradkettige Alkyl-, Alkenyl-, Alkadienyl- oder Alkatrienylgruppe Q' mit 0 bis 3 Methylsubstituenten und insgesamt 6 bis 13 C-Atomen ist, wobei eine solche Gruppe Q' durch OH und/oder durch $N(R^9,R^{10})$ substituiert sein kann,

$R^7$ und $R^8$ — $C_{5-11}$-Alkyl, $C_{5-11}$-Alkenyl oder $C_{5-11}$-Alkadienyl und

$R^9$ und $R^{10}$ — H, Alkyl, Alkenyl oder Alkanoyl sind,

mit den Massnahmen, dass a) in einer Verbindung der Formel I, in der T eine Gruppe CO oder CHOH, L Phenylen oder eine direkt oder über O oder N-Alkyl an M gebundene Alkylen- oder Alkenylengruppe, M 1,4-Phenylen oder durch Alkyl, Alkoxy, Halogen, CN, $NO_2$ oder $CF_3$ monosubstituiertes 1,4-Phenylen und Q substituiertes Phenyl, eine Alkenylgruppe oder eine gegebenenfalls durch OH substituierte Alkylgruppe ist, $A^2$ nicht Alkyl oder Alkenyl sein soll oder $A^1$ zusammen mit $A^2$ nicht Alkylen sein soll,

b) in einer Verbindung der Formel I, in der $A^1$ zusammen mit $A^2$ Alkylen oder durch $R^1$ substituiertes Alkylen, $A^2$ Hydroxyalkyl oder $A^1$ und $A^2$ je eine Alkylgruppe sind, M nicht Pyridylen sein soll und
c) in einer Verbindung der Formel I, in der T eine Gruppe $C(OH, R^{51})$, worin $R^{51}$ Alkyl, Alkenyl, Alkinyl oder Cycloalkyl, M 1,4-Phenylen oder substituiertes 1,4-Phenylen und L eine über ein O-Atom an M gebundene Alkylengruppe ist, letztere zumindest 5 C-Atomen zwischen den 2 freien Valenzen und insgesamt bis zu 11 C-Atomen enthalten soll,

und Säureadditionssalze davon.

2. Verbindungen nach Anspruch 1, worin $A^2$ Cycloalkyl oder Cycloalkyl-alkyl und T CO ist.

3. Verbindungen nach Anspruch 1 oder 2 der Formel

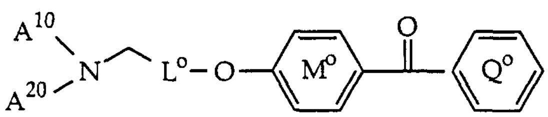 Ia

worin $A^{10}$ Alkyl, $A^{20}$ Cycloalkyl oder Cycloalkyl-alkyl, $L^o$ Alkylen oder Alkenylen mit insgesamt bis zu 11 C-Atomen und zumindest 4 bzw. 3 C-Atomen zwischen den zwei freien Valenzen oder Cycloalkylen-alkylen, $M^o$ gegebenenfalls halogeniertes 1,4-Phenylen und $Q^o$ durch Halogen oder CN substituiertes Phenyl, insbesondere worin $A^{10}$ Methyl, $A^{20}$ Cyclopropyl oder Cyclopropylmethyl, $L^o$ n-Pentylen, n-Propenylen oder Cyclopropylenmethylen, $M^o$ unsubstituiertes oder fluoriertes 1,4-Phenylen und $Q^o$ durch Br oder CN substituiertes Phenyl ist, speziell

(4-Brom-phenyl)-[4-[6-(cyclopropyl-methyl-amino)-hexyloxy]-2-fluorphenyl] -methanon,
(E)-(4-Brom-phenyl)- [4- [4-(cyclopropyl-methyl-amino)-but-2-enyloxy]-phenyl]-methanon,
[6-[6-(Cyclopropyl-methyl-amino)-hexyloxy]-phenyl]-(4-brom-phenyl)-methanon,
(E)-4-[4-[4-(Cyclopropyl-methyl-amino)-but-2-enyloxy]-3-fluor-benzoyl]-benzonitril,
(4-Brom-phenyl)-[4-[6-(cyclopropylmethyl-methyl-amino)-hexyloxy]-phenyl]-methanon,
(1RS,2RS)-(4-Brom-phenyl)-[4-[2-[(cyclopropyl-methyl-amino)-methyl]-cyclopropylmethoxy]-phenyl]-methanon,
(1RS,2RS)-(4-Brom-phenyl)-[4-[2-[(cyclopropyl-methyl-amino)-methyl]-cyclopropylmethoxy]-3-fluor-phenyl]-methanon.

**4.** Verbindungen nach Anspruch 1 oder 2 aus der Gruppe der folgenden:

1-[4-[6-(Cyclopropylmethyl-methyl-amino)-hexyloxy]-2-fluor-phenyl]-5-methyl-hex-4-en-1-on,
1-[4-[6-(Cyclopropyl-methyl-amino)-hexyloxy]-2-fluor-phenyl]-5-methyl-hex-4-en-1-on,
(E)-(4-Brom-phenyl)-[4-[4-(cyclopropylmethyl-methyl-amino)-but-2-enyloxy]-phenyl]-methanon,
(E)-4-[4-[4-(Cyclopropyl-methyl-amino)-but-2-enyloxy]-3-fluor-benzoyl]-benzamid,
(1RS,2RS)-4-[4-[2-[(Cyclopropyl-methyl-amino)-methyl]-cyclopropylmethoxy] -3-fluor-benzoyl] -benzonitril,
(1RS,2RS)-(4-Brom-phenyl)-[4-[2-[(cyclopropylmethyl-methyl-amino)-methyl]-cyclopropylmethoxy]-phenyl] -methanon,
(1RS,2RS)-(4-Brom-phenyl)-[4-[2-[(cyclopropyl-methyl-amino)-methyl]-cyclopropylmethoxy] -2-fluor-phenyl]-methanon,
(1RS,2RS)-[4-[2-[(Allyl-cyclopropyl-amino)-methyl]-cyclopropylmethoxy]-phenyl] -(4-brom-phenyl)-methanon,
(1RS,2RS)-1-[4-[2-[(Cyclopropyl-methyl-amino)-methyl]-cyclopropyl-methoxy]-phenyl]-5-methyl-hexan-1-on.

**5.** Verbindungen nach Anspruch 1, worin T eine Gruppe CHOH, CHF, $C(R^4,R^5)$ oder $C=NOR^6$ ist und $R^4$, $R^5$ und $R^6$ die gleiche Bedeutung wie in Anspruch 1 haben, insbesondere worin T eine Gruppe C(OH,Alkyl), C(OH, Alkenyl), $C=CH_2$ oder C=NO-Alkyl ist.

**6.** Verbindungen nach Anspruch 1 oder 5 der Formel

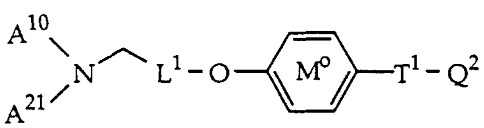 Ib

worin $A^{10}$ Alkyl, $A^{21}$ Alkenyl, $L^1$ Alkylen oder Alkenylen mit insgesamt bis zu 11 C-Atomen und zumindest 4 bzw. 3 C-Atomen zwischen den zwei freien Valenzen, $M^o$ gegebenenfalls halogeniertes 1,4-Phenylen, $T^1$ eine Gruppe C(OH, Alkyl), C(OH,Alkenyl), $C=CH_2$ oder C=NO-Alkyl und $Q^2$ Halophenyl oder Alkenyl mit 0 bis 3 Methylsubstituenten und insgesamt 6 bis 13 C-Atomen, insbesondere worin $A^{10}$ Methyl, $A^{21}$ Allyl, $L^1$ n-Pentylen oder n-Propenylen, $M^o$ unsubstituiertes oder fluoriertes 1,4-Phenylen und $Q^2$ Bromphenyl oder 4-Methylpent-3-enyl, speziell

(E)-(RS)-1-[4-[4-(Allyl-methyl-amino)-but-2-enyloxy]-phenyl]-(4-bromphenyl)-ethanol,
(E)-(RS)-1-[4-[4-(Allyl-methyl-amino)-but-2-enyloxy]-phenyl]-1-(4-bromphenyl)-prop-2-en-1-ol,
(E)-Allyl-[4-[4-[1-(4-brom-phenyl)-vinyl]-phenoxy]-but-2-enyl]-methylamin,
(RS)-1- [4- [6-(Allyl-methyl-amino)-hexyloxy] -2-fluor-phenyl] -1-(4-bromphenyl)-ethanol,
(E)-(RS)-2-[4-[4-(Allyl-methyl-amino)-but-2-enyloxy]-phenyl]-6-methyl-hept-5-en-2-ol.

**7.** Verbindungen nach Anspruch 1 oder 5 aus der Gruppe der folgenden:

(E)-(RS)-[4-[4-(Allyl-methyl-amino)-but-2-enyloxy]-phenyl]-(4-bromphenyl)-cyclopropyl-methanol,
(E)-(RS)-1-[4-[4-(Allyl-methyl-amino)-but-2-enyloxy]-phenyl]-1-(4-bromphenyl)-2,2,2-trifluor-ethanol,
(RS)-1-[4-[6-(Allyl-methyl-amino)-hexyloxy]-phenyl]-1-(4-brom-phenyl)-2,2,2-trifluor-ethanol,
(E)-Allyl-[4-[4-[1-(4-brom-phenyl)-cyclopropyl]-phenoxy]-but-2-enyl]-methyl-amin,
(E)-(R oder S)-1-[4-[4-(Allyl-methyl-amino)-but-2-enyloxy]-phenyl]-(4-brom-phenyl)-ethanol,
(E)-(S oder R)-1-[4-[4-(Allyl-methyl-amino)-but-2-enyloxy]-phenyl]-(4-brom-phenyl)-ethanol,

Allyl-[6-[4-[1-(4-brom-phenyl)-vinyl]-3-fluor-phenoxy]-hexyl]-methylamin,
(E)-(RS)-1-[4-[4-(Allyl-methyl-amino)-but-2-enyloxy]-3-fluor-phenyl]-(4-brom-phenyl)-prop-2-en-1-ol,
(RS)-1-[4-[(Allyl-methyl-amino)-methyl]-biphenyl-4-yl]-1-(4-bromophenyl)-ethanol,
(RS)-5-[6-(Allyl-methyl-amino)-hexyloxy]-2-[1-(4-brom-phenyl)-1-hydroxy-allyl]-phenol,
(RS)-1-[4-[6-(Allyl-methyl-amino)-hexyloxy]-2-amino-phenyl]-1-(4-bromphenyl)-prop-2-en-1-ol,
(RS)-Allyl-[4'-[(4-brom-phenyl)-fluor-methyl]-biphenyl-4-ylmethyl]-methyl-amin,
(E)- und/oder (Z)-[4-[(E)-4-(Allyl-methyl-amino)-but-2-enyloxy]-phenyl]-(4-brom-phenyl)-methanon-O-methyl-oxim,
(E)- und/oder (Z)-[4-[(E)-4-(Allyl-methyl-amino)-but-2-enyloxy]-phenyl]-(4-brom-phenyl)-methanon-oxim,
(E)- und/oder (Z)-[4-[(E)-4-Allyl-methyl-amino)-but-2-enyloxy]-phenyl-(4-brom-phenyl)-methanon-O-tert-butyl-oxim,
(E)- und/oder (Z)-[4-[(E)-4-(Allyl-methyl-amino)-but-2-enyloxy]-phenyl]-(4-brom-phenyl)-methanon-O-allyl-oxim,
(E)- und/oder (Z)-[4-[6-(Allyl-methyl-amino)-hexyloxy]-2-fluor-phenyl]-(4-brom-phenyl)-methanon-oxim.

**8.** Verbindungen nach Anspruch 1, worin M gegebenenfalls durch Alkyl, Halogen, $NH_2$, mono- oder di-alkyliertes Amino, Alkanoylamino, Alkylsulfonylamino, OH, Alkyl(O oder S) oder 1,2,4-Triazol-1-yl substituiertes 1,4-Phenylen, insbesondere worin M durch $NH_2$, mono- oder di-alkyliertes Amino, OH, S-Alkyl oder zwei Halogenatomen substituiertes 1,4-Phenylen ist.

**9.** Verbindungen nach Anspruch 1 oder 8 der Formel

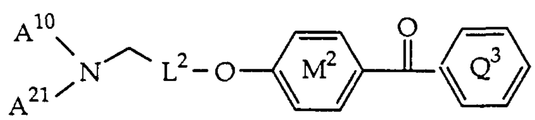
Ic

worin $A^{10}$ Alkyl, $A^{21}$ Alkenyl, $L^2$ Alkylen mit bis zu 11 C-Atomen und zumindest 4 C-Atomen zwischen den zwei freien Valenzen, $M^2$ durch $NH_2$, mono- oder dialkyliertes Amino, OH, S-Alkyl oder zwei Halogenatomen substituiertes 1,4-Phenylen und $Q^3$ halogeniertes Phenyl, insbesondere worin $A^{10}$ Methyl, $A^{21}$ Allyl, $L^2$ n-Pentylen, $M^2$ durch $NH_2$, $NHCH_3$, $N(CH_3)_2$, OH, $SCH_3$ oder durch zwei F-Atomen substituiertes 1,4-Phenylen und $Q^3$ Bromphenyl ist, speziell

(E)-[4-[4-(Allyl-methyl-amino)-but-2-enyloxy]-2,5-difluor-phenyl]-(4-brom-phenyl)-methanon,
[4- [6-(Allyl-methyl-amino)-hexyloxy] -2-methylsulfanyl-phenyl] -(4-bromphenyl)-methanon,
[4-[6-(Allyl-methyl-amino)-hexyloxy]-2-methylamino-phenyl]-(4-bromphenyl)-methanon,
[4-[6-(Allyl-methyl-amino)-hexyloxy]-2-dimethylamino-phenyl]-(4-bromphenyl)-methanon,
[4- [6-(Allyl-methyl-amino)-hexyloxy] -2-hydroxy-phenyl] -(4-bromophenyl)-methanon,
[2-Amino-4-[6-(allyl-methyl-amino)-hexyloxy]-phenyl]-(4-brom-phenyl)-methanon.

**10.** Verbindungen nach Anspruch 1 oder 8 aus der Gruppe der folgenden:

(E)-(4-Brom-phenyl)-[2,5-difluor-4-(4-dimethylamino-but-2-enyloxy]-methanon,
[4-[6-(Allyl-methyl-amino)-hexyloxy]-2,5-difluor-phenyl]-(4-bromphenyl)-methanon,
(E)-[4-[4-(Allyl-methyl-amino)-but-2-enyloxy]-2,5-difluor-phenyl]-(2,4-difluor-phenyl)-methanon,
(E)-[2,5-Difluor-4-(4-dimethylamino-but-2-enyloxy)-phenyl]-(2,4-difluorphenyl)-methanon,
[4-[6-(Allyl-methyl-amino)-hexyloxy]-2,5-difluor-phenyl]-(2,4-difluorphenyl)-methanon,
(2,4-Difluor-phenyl)-[4-(6-dimethylamino-hexyloxy)-2,5-difluorphenyl]methanon,
(E)-[4-[4-(Allyl-methyl-amino)-but-2-enyloxy]-2,3,5,6-tetrafluor-phenyl]-(4-brom-phenyl]-methanon,
(E)-[4-[4-(Allyl-methyl-amino)-but-2-enyloxy]-3-fluor-2-methyl-phenyl]-(4-brom-phenyl)-methanon,
(E)-[4-[4-(Allyl-methyl-amino)-but-2-enyloxy]-2-methylsulfanyl-phenyl]-(4-brom-phenyl)-methanon,
(E)-N-[11-[4-[4-(Allyl-methyl-amino)-but-2-enyloxy]-3-fluor-phenyl]-11-oxo-undecyl] -acetamid,
(E)-[4-(4-Allyl-methyl-amino-but-2-enyloxy)-2-hydroxy-phenyl]-(4-bromphenyl)-methanon,
(E)-1-[4- [4-(Allyl-methyl-amino)-but-2-enyloxy] -3-fluor-phenyl]-11-amino-undecan- 1-on,
(E)-1-[4-[(E)-4-(Allyl-methyl-amino)-but-2-enyloxy]-3-fluor-phenyl]-2-[(E)-3,7-dimethyl-octa-2,6-dienyl]-5,9-dimethyl-deca-4,8-dien-1-on,

(E)-1-[4-[4-(Allyl-methyl-amino)-but-2-enyloxy]-3-fluor-phenyl]-5-methyl-2-(3-methyl-but-2-enyl)-hex-4-en-1-on,
(E)-(RS)-1-[4-[4-(Allyl-methyl-amino)-but-2-enyloxy]-3-fluor-phenyl]-3-hydroxy-5-methyl-hex-4-en-1-on,
(E)-(RS)-1-[4-[4-(Allyl-methyl-amino)-but-2-enyloxy]-2-fluor-phenyl]-3-hydroxy-5-methyl-hex-4-en-1-on,
(E)-(RS)-1- [4-[4-(Allyl-methyl-amino)-but-2-enyloxy]-phenyl]-3-hydroxy-5-methyl-hex-4-en-1-on,
(E)-(RS)-1-[4-[(E)-4-(Allyl-methyl-amino)-but-2-enyloxy]-3-fluor-phenyl]-3-hydroxy-5,9-dimethyl-deca-4,8-dien-1-on,
(E)-(RS)-13-(Allyl-methyl-amino)-1-[4-[4-(allyl-methyl-amino)-but-2-enyloxy]-3-fluor-phenyl]-3-hydroxy-tridecan-1-on,
(E)-1-[4-[(E)-4-(Allyl-methyl-amino)-but-2-enyloxy]-3-fluor-phenyl]-5-methyl-hexa-2,4-dien-1-on,
(E)-13-(Allyl-methyl-amino)-1-[4-[(E)-4-(allyl-methyl-amino)-but-2-enyloxy]-3-fluor-phenyl]-tridec-2-en-1-on,
(E)-1-[4-[(E)-4-(Allyl-methyl-amino)-but-2-enyloxy]-2-fluor-phenyl]-5-methyl-hexa-2,4-dien-1-on,
(E)-1-[4-[(E)-4-(Allyl-methyl-amino)-but-2-enyloxy]-phenyl]-5-methyl-hexa-2,4-dien-1-on,
(2E,4E)-1-[4-[(E)-4-(Allyl-methyl-amino)-but-2-enyloxy]-3-fluor-phenyl]-5,9-dimethyl-deca-2,4,8-trien-1-on,
[4-[6-(Allyl-methyl-amino)-hexyloxy]-2-1H-[1,2,4] triazol-1-yl-phenyl]-(4-brom-phenyl)-methanon,
1-[4-[6-(Allyl-methyl-amino)-hexyloxy]-2-methylamino-pheny]-4-methyl-hex-5-en-1-on,
(E)-1-[4-[4-(Allyl-methyl-amino)-but-2-enyloxy]-2-methylsulfanylphenyl]-5-methyl-hex-4-en-1-on,
N-[5-[6-(Allyl-methyl-amino)-hexyloxy]-2-(4-brom-benzoyl)-phenyl]-methansulfonamid,
(4-Bromphenyl)-(4'-dimethylaminomethyl-3-hydroxy-biphenyl-4-yl)-methanon,
N-[5-[6-(Allyl-methyl-amino)-hexyloxy]-2-(4-brom-benzoyl)-phenyl]-formamid.

**11.** Verbindungen nach Anspruch 1, worin L eine über NH oder N-Alkanoyl an M gebundene Alkylen- oder Alkenylengruppe mit insgesamt bis zu 11 C-Atomen und zumindest 4 bzw. 3 C-Atomen zwischen den zwei freien Valenzen.

**12.** Verbindungen nach Anspruch 1 oder 11 der Formel

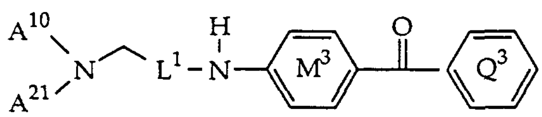

Id

worin $A^{10}$ Alkyl, $A^{21}$ Alkenyl, $L^1$ Alkylen oder Alkenylen mit insgesamt bis zu 11 C-Atomen und zumindest 4 bzw. 3 C-Atomen zwischen den zwei freien Valenzen, $M^3$ halogeniertes 1,4-Phenylen und $Q^3$ halogeniertes Phenyl, insbesondere worin $A^{10}$ Methyl, $A^{21}$ Allyl, $L^1$ n-Pentylen oder n-Propenylen, $M^3$ Fluorophenylen und $Q^3$ Bromphenyl, speziell

(E)-[4-[4-(Allyl-methyl-amino)-but-2-enylamino]-3-fluor-phenyl]-(4-brom-phenyl)-methanon,
[4-[6-(Allyl-methyl-amino)-hexylamino]-3-fluor-phenyl]-(4-bromphenyl)-methanon.

**13.** Verbindungen nach Anspruch 1 oder 11 aus der Gruppe der folgenden:

(E)-N-[4-(Allyl-methyl-amino)-but-2-enyl]-N-[4-(4-brom-benzoyl)-3-fluorphenyl]-acetamid,
N-[6-(Allyl-methyl-amino)-hexyl]-N-[4-(4-bromo-benzoyl)-2-fluorphenyl]-acetamid.

**14.** Verbindungen nach Anspruch 1, worin M eine Gruppe der Formel

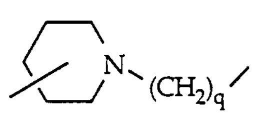

$(M^1)$

und q 1 oder 0, insbesondere worin M zusammen mit T die Piperidin-1-yl-sulfonylgruppe bildet.

**15.** Verbindungen nach Anspruch 1 oder 14 der Formel

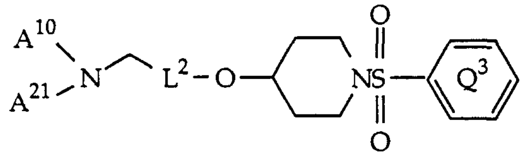

Ie

worin $A^{10}$ Alkyl, $A^{21}$ Alkenyl, $L^2$ Alkylen mit bis zu 11 C-Atomen und zumindest 4 C-Atomen zwischen den zwei freien Valenzen und $Q^3$ halogeniertes Phenyl, insbesondere worin $A^{10}$ Methyl, $A^{21}$ Allyl, $L^2$ n-Pentylen und $Q^3$ Bromphenyl ist, speziell

Allyl-[6-[1-(4-brom-phenylsulfonyl)-piperidin-4-yloxy]-hexyl]-methylamin.

**16.** Verbindungen nach Anspruch 1 oder 14 aus der Gruppe von

[4-[6-(Allyl-methyl-amino)-hexyloxy]-piperidin-1-yl]-1-(4-brom-phenyl) methanon,
2-[4-[6-(Allyl-methyl-amino)-hexyloxy]-piperidin-1-yl]-1-(4-bromphenyl)-ethanon.

**17.** Verbindungen nach Anspruch 1, worin $A^1$ Alkyl und $A^2$ OH oder gegebenenfalls durch eine Gruppe $R^1$, $CONH_2$ oder CN substituiertes Alkyl ist oder

$A^1$ zusammen mit $A^2$ oder mit $A^3$ eine gegebenenfalls durch $R^1$ substituiertes Alkylen-, Alkenylen- oder Alkadienylengruppe $A^1$-$A^2$ bzw. $A^1$-$A^3$ mit bis zu 5 C-Atomen bildet,
wobei in einer Gruppe $A^1$-$A^2$ oder $A^1$-$A^3$ ein C-Atom durch ein N-Atom ersetzt sein kann und
$R^1$ an einem gesättigten C-Atom von $A^2$, $A^1$-$A^2$ oder $A^1$-$A^3$ gebundenes OH, Oxo, Alkyl(O oder S) oder Dialkylamino ist,
wobei ein durch $R^1$ substituiertes C-Atom bzw. ein in $A^2$, $A^1$-$A^2$ oder $A^1$-$A^3$ enthaltenes ungesättigtes C-Atom in einer anderen als der $\alpha$-Stellung zu $N(A^1A^2)$ gebunden sein sollen.

**18.** Verbindungen nach Anspruch 1 oder 17, worin $A^1$ zusammen mit $A^2$ durch OH substituiertes Alkylen mit bis zu 5 C-Atomen ist.

**19.** Verbindungen nach Anspruch 1, 17 oder 18 der Formel

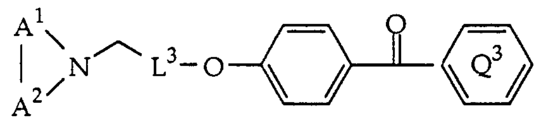

If

worin $A^1$ zusammen mit $A^2$ eine durch OH substituierte Alkylengruppe mit bis zu 5 C-Atomen, $L^3$ Alkenylen mit insgesamt bis zu 11 C-Atomen und zumindest 3 C-Atomen zwischen den zwei freien Valenzen und $Q^3$ halogeniertes Phenyl, insbesondere worin $A^1$ zusammen mit $A^2$ 4-Hydroxy-piperidin-1-yl, $L^3$ n-Propenylen und $Q^3$ Bromphenyl ist, speziell

(E)-(4-Brom-phenyl)-[4-[4-(4-hydroxy-piperidin-1-yl)-but-2-enyloxy]-phenyl] -methanon.

**20.** Verbindungen nach Anspruch 1 oder 17 aus der Gruppe der folgenden:

Cyclohexyl-p-[[(E)-4-(dimethylamino)-2-butenyl]oxy]phenyl-keton,
(E)-[[4-[4-(4-Brom-benzoyl)-phenoxy]-but-2-enyl]-methyl-amino]-acetonitril,
(E)-3-[[4-[4-(4-Brom-benzoyl)-phenoxy]-but-2-enyl]-methyl-amino]-propionitril,
(E)-(4-Brom-phenyl)-[4-[4-(4-dimethylamino-piperidin-1-yl)-but-2-enyloxy] -phenyl] -methanon,
(4-Brom-phenyl)-[4-[6-(hydroxy-methyl-amino)-hexyloxy]-phenyl]-methanon,
(E)-(4-Brom-phenyl)-[4-[4-(hydroxy-methyl-amino)-but-2-enyloxy]-phenyl] -methanon,
(E)-(4-Brom-phenyl)-[4-[4-[(2-methoxy-ethyl)-methyl-amino]-but-2-enyloxy] -phenyl] -methanon,
(E)-(4-Brom-phenyl)-[4-[4-[methyl-(2-methylsulfanyl-ethyl)-amino]-but-2-enyloxy] -phenyl] -methanon,
(E)-(4-Brom-phenyl)-[4-(4-imidazol-1-yl-but-2-enyloxy)-phenyl]-methanon,
(4-Brom-phenyl)-[4-(6-imidazol-1-yl-hexyloxy)-phenyl]-methanon,

(4-Brom-phenyl)-[4-[6-[(3-hydroxy-propyl)-methyl-amino]-hexyloxy]-phenyl] -methanon,
1-[[6-[4-(4-Brom-benzoyl)-phenoxy] -hexyl] -methyl-amino] -propan-2-on,
(E)-2-[[4- [4-(4-Brom-benzoyl)-phenoxy] -but-2-enyl] -methyl-amino]-acetamid,
(±)(4-Brom-phenyl)-[4'-(1-methylpyrrolidin-2-yl)-biphenyl-4-yl]-methanon.

**21.** Verbindungen nach Anspruch 1, worin p = 0 und L an T gebundenes $C_{6-11}$-Alkenylen oder $C_{6-11}$-Alkadienylen ist.

**22.** Verbindungen nach Anspruch 1 oder 21 der Formel

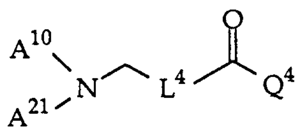 Ig

worin $A^{10}$ Alkyl, $A^{21}$ Alkenyl, $L^4$ $C_{6-11}$-Alkadienylen und $Q^4$ eine Alkenylgruppe mit 0 bis 3 Methylsubstituenten und insgesamt 6 bis 13 C-Atomen, insbesondere worin $A^{10}$ Methyl, $A^{21}$ Allyl, $L^4$ Dimethyloctadienylen und $Q^4$ 4-Methyl-3-pentenyl ist, speziell
(9E,13E)-15-(Allyl-methyl-amino)-2,9,13-trimethyl-pentadeca-2,9,13-trien-6-on.

**23.** Verbindungen nach Anspruch 1 oder 21 aus der Gruppe der folgenden:

(4E,8E)-10-(Allyl-methyl-amino)-1-(4-brom-phenyl)-4,8-dimethyl-deca-4,8-dien-1-on,
(4E,8E)-1-(4-Brom-phenyl)-10-dimethylamino-4,8-dimethyl-deca-4,8-dien-1-on,
(7E,11E)-13-(Allyl-methyl-amino)-2,7,11-trimethyl-trideca-2,7,11-trien-6-on,
(7E,11E)- und (7Z,11E)-13-(Allyl-methyl-amino)-2,7,11-trimethyl-trideca-2,7,11-trien-6-on,
(2E,6E)-8-(Allyl-methyl-amino)-1-(4-brom-phenyl)-2,6-dimethyl-octa-2,6-dien-1-on,
(7E,11E)-13-(Allyl-methyl-amino)-7,11-dimethyl-trideca-1,7,11-trien-6-on,
(2E,6E)-(RS)-8-(Allyl-methyl-amino)-1-(4-brom-phenyl)-2,6-dimethyl-octa-2,6-dien-1-ol,
(E)-(RS)-8-(Allyl-methyl-amino)-1-(4-brom-phenyl)-2,6-dimethyl-oct-6-en-1-on,
(2E,6E)-(RS)-10-(Allyl-methyl-amino)-1-(4-brom-phenyl)-3,7-dimethyl-deca-2,6-dien-1-ol,
(2E,6E)-(RS)-8-(Allyl-methyl-amino)-1-(4-brom-phenyl)-3,7-dimethyl-octa-2,6-dien-1-ol,
(2E,6E)-10-(Allyl-methyl-amino)-1-(4-brom-phenyl)-3,7-dimethyl-deca-2,6-dien-1-on,
(2E,6E)-8-(Allyl-methyl-amino)-1-(4-brom-phenyl)-3,7-dimethyl-octa-2,6-dien-1-on.

**24.** Verbindungen nach Anspruch 1, worin M Thienylen oder Pyridylen, speziell aus der Gruppe der folgenden:

(E)-1-[6-[6-(Allyl-methyl-amino)-hexyloxy]-pyridin-3-yl]-5-methyl-hexa-2,4-dien-1-on,
6-[6-(Allyl-methyl-amino)-hexyloxy]-pyridin-3-yl]-(4-brom-phenyl)-methanon,
(E)-[6-[4-(Allyl-methyl-amino)-but-2-enyloxy]-pyridin-3-yl]-(4-bromphenyl)-methanon,
[5- [6-(Allyl-methyl-amino)-hexyloxy] -pyridin-2-yl] -(4-brom-phenyl)-methanon,
5-(4-[(Allyl-methylamino)-methyl]-phenyl)-thiophen-2-yl)-(4-bromphenyl)-methanon,
5-(4-[Dimethylamino)-methyl]-phenyl)-thiophen-2-yl)-(4-brom-phenyl)-methanon,
5-(4-[(Allyl-methylamino)-methyl]-phenyl)-thiophen-2-yl)-(4-(2,4-difluorphenyl))-methanon,
(2-Dimethylamino-4-fluor-phenyl)-[5-(4-dimethylaminomethyl-phenyl)-thiophen-2-yl] -methanon.

**25.** Verbindungen nach Anspruch 1, worin L über ein O-Atom an M gebundenes Cycloalkylen-alkylen ist, speziell

(1RS,2RS)-(4-Brom-phenyl)-[4-[2-[(ethyl-methyl-amino)-methyl]-cyclopropylmethoxy]-phenyl] -methanon,
(1RS,2RS)-[4-[2-[(Allyl-methyl-amino)-methyl]-cyclopropylmethoxy]-phenyl]-(4-brom-phenyl)-methanon.

**26.** Verbindungen nach einem der Ansprüche 1-25 zur Verwendung als therapeutische Wirkstoffe.

**27.** Verfahren zur Herstellung der Verbindungen nach Anspruch 1, dadurch gekennzeichnet, dass man

a) ein Bromid der Formel

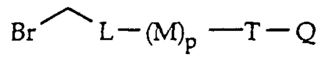 II

mit einem Amin $HN(A^1,A^2)$ umsetzt,

b) ein Amin der Formel

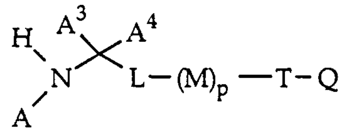 III

worin A die gleiche Bedeutung wie $A^2$ im Anspruch 1 hat, methyliert,

c) ein Amin der Formel III, worin A die gleiche Bedeutung wie $A^1$ im Anspruch 1 hat, mit einem Halogenid der Formel Hal-$A^o$ umsetzt, worin Hal Halogen und $A^o$ Cycloalkyl-alkyl oder durch eine Gruppe $R^1$, $CONH_2$ oder CN substituiertes Alkyl oder Alkenyl ist,

d) ein Aethanon der Formel

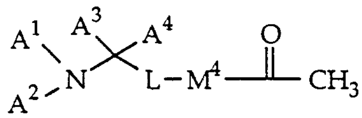 IV

worin $M^4$ für 1,4-Phenylen, das wie in Anspruch 1 angegeben substituiert sein kann, oder für Thienylen oder Pyridylen steht,
mit einem Halogenid der Formel

$$Hal\text{-}R^7$$

zu einem Keton der Formel I umsetzt, worin T zusammen mit Q eine Gruppe $C(O)C(R^7)_2$ ist und $R^7$ die gleiche Bedeutung wie in Anspruch 1 hat,

e) ein Aethanon der Formel IV mit einem Aldehyd der Formel

HC(O)Q"

worin Q" eine geradkettige Alkyl-, Alkenyl- oder Alkadienylgruppe mit 0 bis 3 Methylsubstituenten und insgesamt 4 bis 11 C-Atomen ist,
umsetzt,

f) ein β-Hydroxyketon der Formel

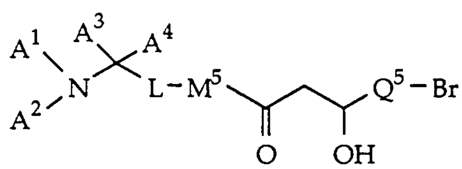 V

worin $M^5$ für 1,4-Phenylen, das wie in Anspruch 1 angegeben substituiert sein kann, steht und $Q^5$ eine der obigen einbindigen Gruppe Q" entsprechende zweibindige Gruppe ist,
mit einem Amin $HN(R^9,R^{10})$ umsetzt,

g) ein Aminoalkohol der Formel

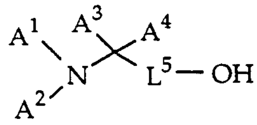

VI

worin $L^5$ Alkylen oder Alkenylen mit insgesamt bis zu 11 C-Atomen und zumindest 4 bzw. 3 C-Atomen zwischen den zwei freien Valenzen oder Cycloalkylen-alkylen ist,
mit einer Verbindung der Formel

$$HO\text{-}M^6\text{-}T\text{-}Q$$

umsetzt,
worin $M^6$ für 1,4-Phenylen, das wie in Anspruch 1 angegeben substituiert sein kann, oder für Thienylen steht,

h) ein Aminoalkohol der Formel VI mit einem Chlorid der Formel

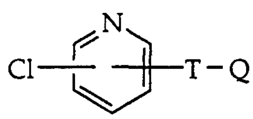

VII

umsetzt,

i) ein Säureadditionssalz eines Amins der Formel

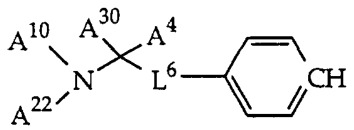

VIII

worin $A^{10}$ Alkyl, $A^{22}$ Cycloalkyl, Cycloalkyl-alkyl oder eine gegebenenfalls durch eine Gruppe $R^{10}$ oder CN substituierte Alkylgruppe,

$A^{30}$ und $A^4$ Wasserstoff oder Alkyl sind oder
$A^{10}$ zusammen mit $A^{22}$ oder mit $A^{30}$ eine gegebenenfalls durch $R^{11}$ substituierte Alkylengruppe bildet, wobei in einer solchen Alkylengruppe ein C-Atom durch N-Alkyl ersetzt sein kann, $R^{11}$ Oxo, Alkyl (O oder S) oder Dialkylamino und
$L^6$ Phenylen oder direkt oder über O oder N-Alkyl an den Phenylring gebundenes Alkylen mit insgesamt bis zu 11 C-Atomen und zumindest 4 C-Atomen zwischen den zwei freien Valenzen ist,

mit einem Säurechlorid der Formel

$$ClC(O)\text{-}Q^6$$

umsetzt,
worin $Q^6$ Cycloalkyl, $C(R^{70},R^{80})$, durch einen oder mehreren Substituenten aus der Gruppe von Alkyl, Halogen, Dialkylamino, CN, $NO_2$, $CF_3$, 1,2,4-Triazol-1-yl und Tetrazol-1-yl substituiertes Phenyl oder eine geradkettige Alkylgruppe mit 0 bis 3 Methylsubstituenten und insgesamt 6 bis 13 C-Atomen ist und $R^{70}$ und $R^{80}$ für $C_{5-11}$-Alkyl stehen,

j) ein Diamin der Formel

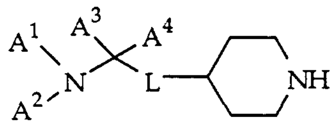

IX

mit einem Halogenid der Formel

$ClS(O)_2$-Q, ClC(O)-Q oder $BrCH_2C(O)$-Q

umsetzt,

k) ein Aldehyd der Formel

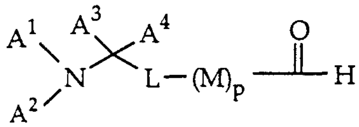

X

mit einem die Gruppe Q einführenden Mittel umsetzt, wobei Q Cycloalkyl, $C(R^7,R^8)$, durch einen oder mehreren Substituenten aus der Gruppe von Alkyl, Halogen, $N(R^{90},R^{100})$, CN, $NO_2$, $CF_3$, 1,2,4-Triazol-1-yl und Tetrazol-1-yl substituiertes Phenyl oder eine geradkettige Alkyl-, Alkenyl-, Alkadienyl-oder Alkatrienylgruppe $Q^{10}$ mit 0 bis 3 Methylsubstituenten und insgesamt 6 bis 13 C-Atomen ist, wobei eine solche Gruppe $Q^{10}$ durch $N(R^{90}, R^{100})$ substituiert sein kann und $R^{90}$ und $R^{100}$ Alkyl oder Alkenyl ist,

l) gewünschtenfalls eine in einer Verbindung der Formel I enthaltene reaktionsfähige Gruppe funktionell abwandelt und

m) gewünschtenfalls ein Amin der Formel I in ein physiologisch verträgliches Säureadditionssalz oder ein Säureadditionssalz einer Verbindung der Formel I in das Amin der Formel I überführt.

**28.** Antimykotisch wirksame und Cholesterin senkende Mittel, enthaltend eine Verbindung nach einem der Ansprüche 1-25 als Wirkstoff gegebenenfalls neben einem therapeutisch inerten Träger.

**29.** Verwendung der Verbindungen nach einem der Ansprüche 1-25 für die Herstellung von antimykotisch wirksamen und Cholesterin senkenden Mittel.

## Claims

**1.** Tertiary amines of the formula

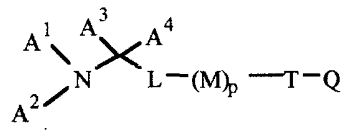

I

wherein

| | |
|---|---|
| $A^1$ | is alkyl or alkenyl and |
| $A^2$ | is cycloalkyl, cycloalkyl-alkyl or an alkyl or alkenyl group optionally substituted by a group $R^1$, $CONH_2$ or CN and, where $A^1$ is alkyl, $A^2$ can also be OH, |
| $A^3$ and $A^4$ | are hydrogen or alkyl or |
| $A^1$ and $A^2$ or $A^3$ | together form an alkylene, alkenylene or alkadienylene group $A^1$-$A^2$ or $A^1$-$A^3$ with up to 5 C atoms optionally substituted by $R^1$, whereby in a group $A^1$-$A^2$ or $A^1$-$A^3$ up to 2 C atoms can be replaced by one (or two) N atom(s) and/or by a N-alkyl group, |
| $R^1$ | is OH, oxo, alkyl (O or S) or dialkylamino bonded to a saturated C atom of $A^2$, $A^1$-$A^2$ or $A^1$-$A^3$, whereby a C atom substituted by $R^1$ or an unsaturated C atom present in $A^1$, $A^2$, $A^1$-$A^2$ or $A^1$-$A^3$ must be bonded in a position other than the $\alpha$-position to $N(A^1A^2)$, |
| p = 1 and L | is phenylene, or alkylene or alkenylene with a total of up to 11 C atoms and at least 4 or, respectively, 3 C atoms between the two free valencies and which is bonded to M directly or via O, NH or N(alkyl or alkanoyl), or cycloalkylene-alkylene or |
| p = 0 and L | is $C_{6-11}$-alkenylene or $C_{6-11}$-alkadienylene bonded to T, |
| M | is thienylene, pyridylene, 1,4-phenylene, 1,4-phenylene substituted by one or more substituents from the group of alkyl, halogen, $N(R^2R^{21})$, $CONH_2$, CN, $NO_2$, $CF_3$, OH, alkyl (O or S), 1,2,4-triazol-1-yl or tetrazol-1-yl or a group of the formula |

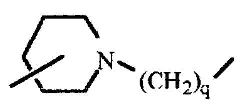

$(M^1)$

| | |
|---|---|
| q | is 1 or 0, |
| $R^2$ and $R^{21}$ | are H, alkyl, alkenyl, alkanoyl or $SO_2$-alkyl, |
| T | is CO, $CH(R^3)$, $C(R^4,R^5)$ or $C=NOR^6$ and, where M is a group $M^1$ and q = 0, T can also be $SO_2$, |
| $R^3$ | is OH, F, alkoxy or alkanoyloxy, |
| $R^4$ is OH and $R^5$ | is alkyl, alkenyl, alkynyl, cycloalkyl or $CF_3$ or |
| $R^4$ and $R^5$ | together are the group $CH_2$, $CH_2O$ or $CH_2CH_2$, |
| $R^6$ | is H, alkyl or alkenyl, |
| Q | is cycloalkyl, $C(R^7,R^8)$, phenyl substituted by one or more substituents from the group of alkyl, halogen, $N(R^9,R^{10})$, $CONH_2$, CN, $NO_2$, $CF_3$, 1,2,4-triazol-1-yl and tetrazol-1-yl or a straight-chain alkyl, alkenyl, alkadienyl or alkatrienyl group Q' with 0 to 3 methyl substituents and a total of 6 to 13 C atoms, whereby such a group Q' can be substituted by OH and/or by $N(R^9,R^{10})$, |
| $R^7$ and $R^8$ | are $C_{5-11}$-alkyl, $C_{5-11}$-alkenyl or $C_{5-11}$-alkadienyl and |
| $R^9$ and $R^{10}$ | are H, alkyl, alkenyl or alkanoyl, |

with the provisos that a) $A^2$ must not be alkyl or alkenyl or $A^1$ and $A^2$ together must not be alkylene in a compound of formula I in which T is a group CO or CHOH, L is phenylene or an alkylene or aLkenylene group bonded to M directly or via O or N-alkyl, M is 1,4-phenylene or 1,4-phenylene monosubstituted by alkyl, alkoxy, halogen, CN, $NO_2$ or $CF_3$ and Q is substituted phenyl, an alkenyl group or an alkyl group optionally substituted by OH,

b) M must not be pyridylene in a compound of formula I in which $A^1$ and $A^2$ together signify alkylene or alkylene substituted by $R^1$, $A^2$ is hydroxyalkyl or $A^1$ and $A^2$ are each an alkyl group and

c) in a compound of formula I in which T is a group $C(OH, R^{51})$, wherein $R^{51}$ is alkyl, alkenyl, alkynyl or cycloalkyl, M is 1,4-phenylene or substituted 1,4-phenylene and L is

an alkylene group bonded to M via a O atom, the latter must contain at least 5 C atoms between the 2 free valencies and a total of up to 11 C atoms,

and acid addition salts thereof.

2. Compounds according to claim 1, wherein $A^2$ is cycloalkyl or cycloalkylakyl and T is CO.

3. Compounds according to claim 1 or 2 of the formula

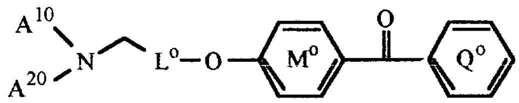

Ia

wherein $A^{10}$ is alkyl, $A^{20}$ is cycloalkyl or cycloalkyl-alkyl, $L^o$ is alkylene or alkenylene with a total of up to 11 C atoms and at least 4 or, respectively, 3 C atoms between the two free valencies or cycloalkylene-alkylene, $M^o$ is optionally halogenated 1,4-phenylene and $Q^o$ is phenyl substituted by halogen or CN, especially wherein $A^{10}$ is methyl, $A^{20}$ is cyclopropyl or cyclopropylmethyl, $L^o$ is n-pentylene, n-propenylene or cyclopropylenemethylene, $M^o$ is unsubstituted or fluorinated 1,4-phenylene and $Q^o$ is phenyl substituted by Br or CN, especially

(4-bromo-phenyl)-[4-(6-(cyclopropyl-methyl-amino)-hexyloxy]-2-fluorophenyl]-methanone,
(E)-(4-bromo-phenyl)-[4-[4-(cyclopropyl-methyl-amino)-but-2-enyloxy]-phenyl] -methanone,
[6-[6-(cyclopropyl-methyl-amino)-hexyloxy]-phenyl]-(4-bromo-phenyl)-methanone,
(E)-4-[4-[4-(cyclopropyl-methyl-amino)-but-2-enyloxy] -3-fluoro-benzoyl]-benzonitrile,
(4-bromo-phenyl)-[4-[6-(cyclopropylmethyl-methyl-amino)-hexyloxy]-phenyl]-methanone,
(1RS,2RS)-(4-bromo-phenyl)-[4-[2-[(cyclopropyl-methyl-amino)-methyl]-cyclopropylmethoxy] -phenyl] -methanone,
(1RS,2RS)-(4-bromo-phenyl) - [4- [2- [ (cyclopropyl-methyl-amino) -methyl]-cyclopropylmethoxy]-3-fluoro-phenyl]-methanone.

4. Compounds according to claim 1 or 2 from the following group:

1-[4- [6-(Cyclopropylmethyl-methyl-amino)-hexyloxy] -2-fluoro-phenyl] -5-methyl-hex-4-en-1-one,
1-[4-[6-(cyclopropyl-methyl-amino) -hexyloxy] -2-fluoro-phenyl]-5-methyl-hex-4-en-1-one,
(E)-(4-bromo-phenyl)-[4-[4-(cyclopropylmethyl-methyl-amino)-but-2-enyloxy]-phenyl] -methanone,
(E)-4-[4- [4-(cyclopropyl-methyl-amino)-but-2-enyloxy] -3-fluoro-benzoyl]benzamide,
(1RS,2RS)-4-[4-[2-[ (cyclopropyl-methyl-amino) -methyl] -cyclopropylmethoxy] -3-fluoro-benzoyl]-benzonitrile,
(1RS,2RS)-(4-bromo-phenyl)-[4- [2- [ (cyclopropylmethyl-methyl-amino) -methyl]-cyclopropylmethoxy] -phenyl] -methanone,
(1RS,2RS)-(4-bromo-phenyl)-[4-[2-[(cyclopropyl-methyl-amino) -methyl]-cyclopropylmethoxy]-2-fluoro-phenyl]-methanone,
(1RS,2RS)-[4-[2-[ (allyl-cyclopropyl-amino)-methyl]-cyclopropylmethoxy]-phenyl] -(4-bromo-phenyl) -methanone,
(1RS,2RS)-1-[4-[2-[(cyclopropyl-methyl-amino)-methyl]-cyclopropylmethoxy]-phenyl] -5-methyl-hexan-1-one.

5. Compounds according to claim 1, wherein T is a group CHOH, CHF, $C(R^4,R^5)$ or $C\text{-}NOR^6$ and $R^4$, $R^5$ and $R^6$ have the same significance as in claim 1, wherein T is a group C(OH, alkyl), C(OH, alkenyl), $C{=}CH_2$ or C=NO-alkyl.

6. Compounds according to claim 1 or 5 of the formula

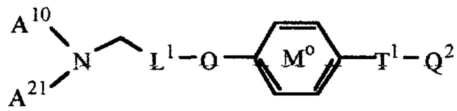 Ib

wherein $A^{10}$ is alkyl, $A^{21}$ is alkenyl, $L^1$ is alkylene or alkenylene with a total of up to 11C atoms and at least 4 or, respectively, 3 C atoms between the two free valencies, $M^o$ is optionally halogenated 1,4-phenylene, $T^1$ is a group C(OH, alkyl), C(OH, alkenyl), $C=CH_2$ or C=NO-alkyl and $Q^2$ is halophenyl or alkenyl with 0 to 3 methyl substituents and a total of 6 to 13 C atoms, especially wherein $A^{10}$ is methyl, $A^{21}$ is allyl, $L^1$ is n-pentylene or n-propenylene, $M^o$ is unsubstituted or fluorinated 1,4-phenylene and $Q^2$ is bromophenyl or 4-methylpent-3-enyl, especially

(E)-(RS)-1-[4-[4-(allyl-methyl-amino)-but-2-enyloxy]-phenyl] -(4-bromophenyl) ethanol,
(E)-(RS)-1-[4-[4-(allyl-methyl-amino)-but-2-enyloxy] -phenyl] -1-(4-bromophenyl)-prop-2-en-1 -ol,
(E)-allyl-[4-[4-[1-(4-bromo-phenyl)-vinyl]-phenoxy]-but-2-enyl]-methyl-amine,
(RS)-1-[4-[6-(allyl-methyl-amino)-hexyloxy] -2-fluoro-phenyl] -1-(4-bromophenyl) -ethanol,
(E) (RS)-2-[4-[4-(allyl-methyl amino)-but-2-enyloxy]-phenyl]-6-methyl-hept-5-en-2-ol.

**7.** Compounds according to claim 1 or 5 from the following group:

(E)-(RS)-[4-[4-(Allyl-methyl-amino)-but-2-enyloxy]-phenyl]-(4-bromo-phenyl)-cyclopropyl-methanol,
(E)-(RS)-1-[4- [4-(allyl-methyl-amino) -but-2-enyloxy]-phenyl]-1-(4-bromophenyl)-2,2,2-trifluoro-ethanol,
(RS)-1-[4-[6-(allyl-methyl-amino)-hexyloxy]-phenyl]-1-(4-bromo-phenyl)-2,2,2-trifluoro-ethanol,
(E)-allyl-[4-[4-[1-(4-bromo-phenyl)-cyclopropyl] -phenoxy] -but-2-enyl] -methylamine,
(E)-(R or S)-1-[4-[4-(allyl-methyl-amino)-but-2-enyloxy]-phenyl]-(4-bromophenyl)-ethanol,
(E)-(S or R)-1-[4-[4-(allyl-methyl-amino)-but-2-enyloxy]-phenyl]-(4-bromophenyl) -ethanol,
allyl- [6- [4- [1-(4-bromo-phenyl) -vinyl] -3-fluoro-phenoxy] -hexyl] -methyl-amine,
(E)-(RS)-1-[4-[4-(allyl-methyl-amino)-but-2-enyloxy]-3-fluoro-phenyl]-(4-bromo-phenyl)-prop-2-en-1-ol,
(RS)-1-[4-[(allyl-methyl-amino)-methyl] -biphenyl-4-yl]-1-(4-bromo-phenyl)-ethanol,
(RS)-5-[6- (allyl-methyl-amino)-hexyloxy] -2- [1-(4-bromo-phenyl)-1-hydroxyallyl]-phenol,
(RS)-1-[4-[6-(allyl-methyl-amino)-hexyloxy] -2-amino-phenyl]-1-(4-bromophenyl)-prop-2-en-1-ol,
(RS)-allyl-[4'-[(4-bromo-phenyl)-fluoro-methyl]-biphenyl-4-ylmethyl]-methylamine,
(E)- and/or (Z)-[4-[(E)-4-(allyl-methyl-amino)-but-2-enyloxy]-phenyl]-(4-bromo-phenyl)-methanone O-methyl oxime,
(E)- and/or (Z)-[4-[(E)-4-(allyl-methyl-amino) -but-2-enyloxy] -phenyl] -(4-bromo-phenyl)-methanone oxime,
(E)-and/or (Z)-[4-[(E)-4-allyl-methyl-amino)-but-2-enyloxy]-phenyl-(4-bromophenyl)-methanone O-tert-butyl oxime,
(E)- and/or (Z)-[4-[(E)-4-(allyl-methyl-amino)-but-2-enyloxy]-phenyl]-(4-bromo-phenyl)-methanone O-allyl oxime,
(E)- and/or (Z)-[4-[6-(allyl-methyl-amino)-hexyloxy]-2-fluoro-phenyl]-(4-bromo-phenyl)-methanone oxime.

**8.** Compounds according to claim 1, wherein M is 1,4-phenylene optionally substituted by alkyl, halogen, $NH_2$, mono or di-alkylated amino, alkanoylamino, alkylsulphonylamino, OH, alkyl(O or S) or 1,2,4-triazol-1-yl, especially wherein M is 1,4-phenylene substituted by $NH_2$, mono- or di-alkylated amino, OH, S-alkyl or two halogen atoms.

**9.** Compounds according to claim 1 or 8 of the formula

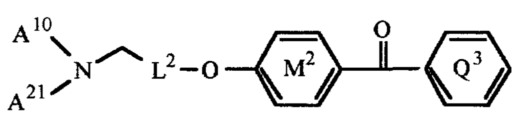 Ic

wherein $A^{10}$ is alkyl, $A^{21}$ is alkenyl, $L^2$ is alkylene with up to 11 C atoms and at least 4 C atoms between the

two free valencies, $M^2$ is 1,4-phenylene substituted by $NH_2$, mono- or dialkylated amino, OH, S-alkyl or two halogen atoms and $Q^3$ is halogenated phenyl, especially wherein $A^{10}$ is methyl, $A^{21}$ is allyl, $L^2$ is n-pentylene, $M^2$ is 1,4-phenylene substituted by $NH_2$, $NHCH_3$, $N(CH_3)_2$, OH, $SCH_3$ or by two F atoms and $Q^3$ is bromophenyl, especially

(E) - [4- [4-(allyl-methyl-amino) -but-2-enyloxy]-2,5-difluoro-phenyl]-(4-bromo-phenyl)-methanone,
[4-[6-(allyl-methyl-amino)-hexyloxy]-2-methylsulphanyl-phenyl]-(4-bromophenyl) -methanone,
[4-[6-(allyl-methyl-amino)-hexyloxy]-2-methylamino-phenyl]-(4-bromo-phenyl)-methanone,
[4-[6-(allyl-methyl-amino)-hexyloxy]-2-dimethylamino-phenyl]-(4-bromophenyl)-methanone,
[4-[6-(allyl-methyl-amino)-hexyloxy]-2-hydroxy-phenyl]-(4-bromo-phenyl)-methanone,
[2-amino-4-[6-(allyl-methyl-amino)-hexyloxy]-phenyl]-(4-bromo-phenyl)-methanone.

**10.** Compounds according to claim 1 or 8 from the following group:

(E)-(4-Bromo-phenyl)-[2,5-difluoro-4-(4-dimethylamino-but-2-enyloxy]-methanone,
[4- [6-(allyl-methyl-amino) -hexyloxy] -2,5-difluoro-phenyl] -(4-bromo-phenyl)-methanone,
(E)-[4-[4-(allyl-methyl-amino)-but-2-enyloxy]-2,5-difluoro-phenyl]-(2,4-difluoro-phenyl) -methanone,
(E)- [2,5-difluoro-4-(4-dimethylamino-but-2-enyloxy)-phenyl]-(2,4-difluorophenyl)-methanone,
[4- [6-(allyl-methyl-amino)-hexyloxy]-2,5-difluoro-phenyl] -(2,4-difluoro-phenyl)-methanone,
(2,4-difluoro-phenyl)-[4-(6-dimethylamino-hexyloxy)-2,5-difluoro-phenyl]-methanone,
(E)-[4- [4-(allyl-methyl-amino)-but-2-enyloxy]-2,3,5,6-tetrafluoro-phenyl] -(4-bromo-phenyl] -methanone,
(E)-[4-[4-(allyl-methyl-amino)-but-2-enyloxy] -3-fluoro-2-methyl-phenyl]-(4-bromo-phenyl) -methanone,
(E)-[4- [4-(allyl-methyl-amino)-but-2-enyloxy]-2-methylsulphanyl-phenyl]-(4-bromo-phenyl) -methanone,
(E) -N-[11-[4-[4-(allyl-methyl-amino) -but-2-enyloxy] -3-fluoro-phenyl]-11-oxo-undecyl]-acetamide,
(E)-[4-(4-allyl-methyl-amino-but-2-enyloxy)-2-hydroxy-phenyl]-(4-bromophenyl) -methanone,
(E)-1-[4- [4-(allyl-methyl-amino) -but-2-enyloxy] -3-fluoro-phenyl]-11-amino-undecan-1-one,
(E)-1-[4-[(E)-4-(allyl-methyl-amino) -but-2-enyloxy] -3-fluoro-phenyl] -2- [ (E) -3,7-dimethyl-octa-2,6-dienyl]-5,9-dimethyl-deca-4,8-dien-1-one,
(E)-1-[4-[4-(allyl-methyl-amino)-but-2-enyloxy] -3-fluoro-phenyl] -5-methyl-2-(3-methyl-but-2-enyl)-hex-4-en-1-one,
(E)-(RS)-1-[4-[4-(allyl-methyl-amino)-but-2-enyloxy]-3-fluoro-phenyl]-3-hydroxy-5-methyl-hex-4-en-1-one,
(E)-(RS)-1-[4-[4-(allyl-methyl-amino)-but-2-enyloxy]-2-fluoro-phenyl] -3-hydroxy-5-methyl-hex-4-en-1-one,
(E) (RS)-1-[4- [4-(allyl-methyl-amino) -but-2-enyloxy]-phenyl] -3-hydroxy-5-methyl-hex-4-en-1-one,
(E)-(RS)-1-[4-[(E)-4-(allyl-methyl-amino)-but-2-enyloxy] -3-fluoro-phenyl] -3-hydroxy-5,9-dimethyl-deca-4,8-dien-1-one,
(E) -(RS) - 13-(allyl-methyl-amino)-1- [4- [4-(allyl-methyl-amino) -but-2-enyloxy] -3-fluoro-phenyl] -3-hydroxy-tridecan-1-one,
(E)-1-[4-[(E)-4-(allyl-methyl-amino)-but-2-enyloxy] -3-fluoro-phenyl]-5-methyl-hexa-2,4-dien-1-one,
(E)-13-(allyl-methyl-amino)-1- [4- [ (E) -4-(allyl-methyl-amino) -but-2-enyloxy] -3-fluoro-phenyl]-tridec-2-en-1-one,
(E)-1-[4- [(E)-4-(allyl-methyl-amino)-but-2-enyloxy] -2-fluoro-phenyl]-5-methyl-hexa-2,4-dien-1-one,
(E)-1-[4-[(E)-4-(allyl-methyl-amino)-but-2-enyloxy] -phenyl]-5-methyl-hexa-2,4-dien-1-one,
(2E,4E)-1-[4-[(E)-4-(allyl-methyl-amino)-but-2-enyloxy]-3-fluoro-phenyl]-5,9-dimethyl-deca-2,4,8-trien-1-one,
[4- [6-(allyl-methyl-amino)-hexyloxy] -2-1 H- [1,2,4]triazol-1-yl-phenyl]-(4-bromo-phenyl)-methanone,
1-[4-[6-(allyl-methyl-amino)-hexyloxy] -2-methylamino-pheny]-4-methyl-hex-5-en-1-one,
(E)-1-[4-[4-(allyl-methyl-amino)-but-2-enyloxy] -2-methylsulphanyl-phenyl] -5-methyl-hex-4-en-1-one,
N- [5-[6-(allyl-methyl-amino)-hexyloxy] -2-(4-bromo-benzoyl)-phenyl]-methane-sulphonamide,
(4-bromo-phenyl)-(4'-dimethylaminomethyl-3-hydroxy-biphenyl-4-yl)-methanone,
N-[5-[6-(allyl-methyl-amino)-hexyloxy]-2-(4-bromo-benzoyl)-phenyl]-formamide.

**11.** Compounds according to claim 1, wherein L is an alkylene or alkenylene group with a total of up to 11 C atoms and at least 4 or, respectively, 3 C atoms between the two free valencies which is bonded to M via NH or N-alkanoyl.

**12.** Compounds according to claim 1 or 11 of the formula

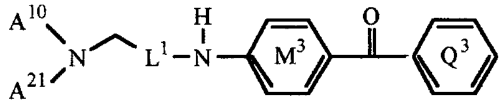

Id

wherein $A^{10}$ is alkyl, $A^{21}$ is alkenyl, $L^1$ is alkylene or alkenylene with a total of up to 11 C atoms and at least 4 or, respectively, 3 C atoms between the two free valencies, $M^3$ is halogenated 1,4-phenylene and $Q^3$ is halogenated phenyl, especially wherein $A^{10}$ is methyl, $A^{21}$ is allyl, $L^1$ is n-pentylene or n-propenylene, $M^3$ is fluorophenylene and $Q^3$ is bromophenyl, especially

(E)- [4- [4-(allyl-methyl-amillo)-but-2-enylamino] -3-fluoro-phenyl] -(4-bromo-phenyl) -methanone,
[4- [6-(allyl-methyl-amino)-hexylamino]-3-fluoro-phenyl]-(4-bromo-phenyl)-methanone.

**13.** Compounds according to claim 1 or 11 from the following group:

(E)-N-[4-(Allyl-methyl-amino)-but-2-enyl] -N- [4-(4-bromo-benzoyl)-2-fluorophenyl] -acetamide,
N- [6-(allyl-methyl-amino)-hexyl]-N-[4-(4-bromobenzoyl)-2-fluoro-phenyl]-acetamide.

**14.** Compounds according to claim 1, wherein M is a group of the formula

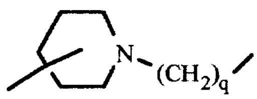

($M^1$)

and q is 1 or 0, especially wherein M and T together form the piperidin-1-yl-sulphonyl group.

**15.** Compounds according to claim 1 or 14 of the formula

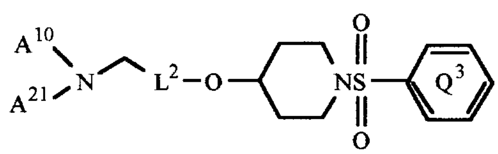

Ie

wherein $A^{10}$ is alkyl, $A^{21}$ is alkenyl, $L^2$ is alkylene with up to 11 C atoms and at least 4 C atoms between the two free valencies and $Q^3$ is halogenated phenyl, especially wherein $A^{10}$ is methyl, $A^{21}$ is allyl, $L^2$ is n-pentylene and $Q^3$ is bromophenyl, especially
allyl-[6- [1-(4-bromo-phenylsulphonyl)-piperidin-4-yloxy] -hexyl] -methyl-amine.

**16.** Compounds according to claim 1 or 14 from the group of

[4- [6-(allyl-methyl-amino)-hexyloxy] -piperidin-1-yl]-1-(4-bromo-phenyl)-methanone,
2-[4- [6-(allyl-methyl-amino)-hexyloxy] -piperidin- -yl]-1-(4-bromo-phenyl)-ethanone.

**17.** Compounds according to claim 1, wherein $A^1$ is alkyl and $A^2$ is OH or alkyl optionally substituted by a group $R^1$, $CONH_2$ or CN or

$A^1$ and $A^2$ or $A^3$ together form an alkylene, alkenylene or alkadienylene group $A^1$-$A^2$ or $A^1$-$A^3$ with up to 5 C atoms which is optionally substituted by $R^1$,
whereby a C atom in a group $A^1$-$A^2$ or $A^1$-$A^3$ can be replaced by a N atom and $R^1$ is OH, oxo, alkyl(O or S) or dialkylamino bonded to a saturated C atom of $A^2$, $A^1$-$A^2$ or $A^1$-$A^3$,

whereby a C atom substituted by $R^1$ or an unsaturated C atom present in $A^2$, $A^1$-$A^2$ or $A^1$-$A^3$ must be bonded in a position other than the $\alpha$-position to $N(A^1A^2)$,

**18.** Compounds according to claim 1 or 17, wherein $A^1$ and $A^2$ together are alkylene with up to 5 C atoms which is substituted with OH.

**19.** Compounds according to claim 1, 17 or 18 of the formula

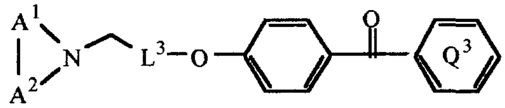

If

wherein $A^1$ and $A^2$ together signify an alkylene group with up to 5 C atoms which is substituted by OH, $L^3$ is alkenylene with a total of up to 11 C atoms and at least 3 C atoms between the two free valencies and $Q^3$ is halogenated phenyl, especially wherein $A^1$ and $A^2$ together are 4-hydroxy-piperidin-1-yl, $L^3$ is n-propenylene and $Q^3$ is bromophenyl, especially
(E)-(4-bromo-phenyl)-[4- [4-(4-hydroxy-piperidin-1-yl)-but-2-enyloxy] -phenyl]-methanone.

**20.** Compounds according to claim 1 or 17 from the following group:

Cyclohexyl p-[[(E)-4-(dimethylamino)-2-butenyl]oxy]phenyl ketone,
(E)-[[4-[4-(4-bromo-benzoyl)-phenoxy]-but-2-enyl]-methyl-amino]-acetonitrile,
(E)-3-[[4-[4-(4-bromo-benzoyl) -phenoxy] -but-2-enyl]-methyl-amino]-propionitrile,
(E)-(4-bromo-phenyl)-[4-[4-(4-dimethylamino-piperidin-1-yl)-but-2-enyloxy]-phenyl] -methanone,
(4-bromo-phenyl)-[4- [6- (hydroxy-methyl-amino)-hexyloxy] -phenyl] -methanone,
(E)-(4-bromo-phenyl)-[4-[4-(hydroxy-methyl-amino)-but-2-enylony]-phenyl]-methanone,
(E)-(4-bromo-phenyl) - [4-[4-[(2-methoxy-ethyl)-methyl-amino]-but-2-enyloxy]-phenyl] -methanone,
(E)-(4-bromo-phenyl)-[4-[4-[methyl-(2-methylsulphanyl-ethyl) -amino] -but-2-enyloxy] -phenyl] -methanone,
(E)-(4-bromo-phenyl)-[4-(4-imidazol-1-yl-but-2-enyloxy)-phenyl] -methanone,
(4-bromo-phenyl)-[4-(6-imidazol-1-yl-hexyloxy)-phenyl]-methanone,
(4-bromo-phenyl) - [4- [6-[(3-hydroxy-propyl)-methyl-amino]-hexyloxy] -phenyl]-methanone,
1-[[6-[4-(4-bromo-benzoyl)-phenoxy]-hexyl]-methyl-amino]-propan-2-one,
(E)-2-[[4-[4-(4-bromo-benzoyl)-phenoxy]-but-2-enyl]-methyl-amino]-acetamide,
($\pm$) (4-bromo-phenyl)-[4'-(1-methylpyrrolidin-2-yl)-biphenyl-4-yl]-methanone.

**21.** Compounds according to claim 1, wherein p = 0 and L is $C_{6-11}$-alkenylene or $C_{6-11}$-alkadienylene bonded to T.

**22.** Compounds according to claim 1 or 21 of the formula

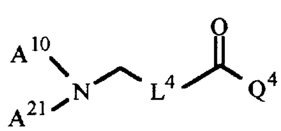

Ig

wherein $A^{10}$ is alkyl, $A^{21}$ is alkenyl, $L^4$ is $C_{6-11}$-alkadienylene and $Q^4$ is an alkenyl group with 0 to 3 methyl substituents and a total of 6 to 13 C atoms, especially wherein $A^{10}$ is methyl, $A^{21}$ is allyl, $L^4$ is dimethyloctadienylene and $Q^4$ is 4-methyl-3-pentenyl, especially
(9E,13E)-15-(allyl-methyl-amino) -2,9,13-trimethyl-pentadeca-2,9,13-trien-6-one.

**23.** Compounds according to claim 1 or 21 from the following group:

(4E,8E)-10-(Allyl-methyl-amino) -1-(4-bromo-phenyl) -4,8-dimethyl-deca-4,8-dien-1-one,
(4E,8E)-1-(4-bromo-phenyl)-10-dimethylamino-4,8-dimethyl-deca-4,8-dien-1-one,

(7E,11E)-13-(allyl-methyl-amino) -2,7,11-trimethyl-trideca-2,7,11-trien-6-one,
(7E,11E)-and(7Z,11E)-13-(allyl-methyl-amino)-2,7,11-trimethyl-trideca-2,7,11-trien-6-one,
(2E,6E)-8-(allyl-methyl-amino)-1-(4-bromo-phenyl)-2,6-dimethyl-octa-2,6-dien-1-one,
(7E,11E)-13-(allyl-methyl-amino)-7,11-dimethyl-trideca-1,7,11-trien-6-one,
(2E,6E)-(RS)-8-(allyl-methyl-amino)-1-(4-bromo-phenyl)-2,6-dimehtyl-octa-2,6-dien-1-ol,
(E)-(RS)-8-(allyl-methyl-amino) -1-(4-bromo-phenyl)-2,6-dimethyl-oct-6-en-1-one,
(2E,6E)-(RS)-10-(allyl-methyl-amino)-1-(4-bromo-phenyl)-3,7-dimethyl-deca-2,6-dien-1-ol,
(2E,6E)-(RS)-8-(allyl-methyl-amino)-1-(4-bromo-phenyl)-3,7-dimethyl-octa-2,6-dien-1-ol,
(2E,6E) -10-(allyl-methyl-amino)-1-(4-bromo-phenyl) -3,7-dimethyl-deca-2,6-dien-1-one,
(2E,6E)-8-(allyl-methyl-amino)-1-(4-bromo-phenyl)-3,7-dimethyl-octa-2,6-dien-1-one.

**24.** Compounds according to claim 1, wherein M is thienylene or pyridylene, especially from the following group:

(E)-1-[6-[6-(Allyl-methyl-amino) -hexyloxy] -pyridin-3-yl] -5-methyl-hexa-2,4-dien-1-one,
6- [6-(allyl-methyl-amino)-hexyloxy] -pyridin-3-yl]-(4-bromo-phenyl)-methanone,
(E)-[6- [4-(allyl-methyl-amino)-but-2-enyloxy] -pyridin-3-yl]-(4-bromo-phenyl)-methanone,
[5-[6-(allyl-methyl-amino)-hexyloxy]-pyridin-2-yl]-(4-bromo-phenyl)-methanone,
5-(4-[(allyl-methylamino)-methyl]-phenyl) -thiophen-2-yl) -(4-bromophenyl)-methanone,
5-(4-[dimethylamino)-methyl] -phenyl)-thiophen-2-yl)-(4-bromophenyl)-methanone,
5-(4-[(allyl-methylamino)-methyl] -phenyl)-thiophen-2-yl)-(4-(2,4-difluorophenyl))-methanone,
(2-dimethylamino-4-fluoro-phenyl)-[5-(4-dimethylaminomethyl-phenyl)-thiophen-2-yl] -methanone.

**25.** Compounds according to claim 1, wherein L is cycloalkylene-alkylene bonded to M via an O atom, especially

(1RS,2RS) -(4-bromo-phenyl) - [4- [2- [ (ethyl-methyl-amino) -methyl] -cyclopropylmethoxy] -phenyl] -methanone,
(1RS,2RS)-[4-[2-[ (allyl-methyl-amino)-methyl]-cyclopropylmethoxy]-phenyl]-(4-bromo-phenyl) -methanone.

**26.** Compounds according to any one of claims 1-25 for use as therapeutically active substances.

**27.** A process for the manufacture of the compounds according to claim 1, characterized by

a) reacting a bromide of the formula

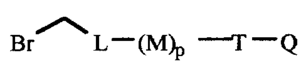

II

with an amine HN($A^1$,$A^2$),

b) methylating an amine of the formula

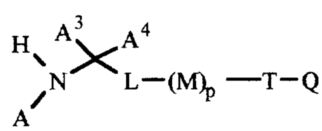

III

wherein A has the same significance as $A^2$ in claim 1,

c) reacting an amine of formula III, wherein A has the same significance as $A^1$ in claim 1, with a halide of the formula Hal-$A^o$, wherein Hal is halogen and $A^o$ is cycloalkyl-alkyl or alkyl or alkenyl substituted by a group $R^1$, $CONH_2$ or CN,

d) reacting an ethanone of the formula

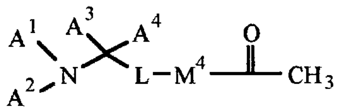 IV

wherein $M^4$ stands for 1,4-phenylene, which can be substituted as given in claim 1, or for thienylene or pyridylene,
with a halide of the formula

$$Hal\text{-}R^7$$

to give a ketone of formula I, wherein T and Q together are a group $C(O)C(R^7)_2$ and $R^7$ has the same significance as in claim 1,

e) reacting an ethanone of formula IV with an aldehyde of the formula

HC(O)Q"

wherein Q" is a straight-chain alkyl, alkenyl or alkadienyl group with 0 to 3 methyl substituents and a total of 4 to 11 C atoms,

f) reacting a β-hydroxyketone of the formula

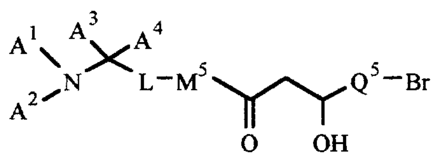 V

wherein $M^5$ stands for 1,4-phenylene, which can be substituted as given in claim 1, and $Q^5$ is a divalent group corresponding to the above monovalent group Q",
with an amine $HN(R^9,R^{10})$,

g) reacting an aminoalcohol of the formula

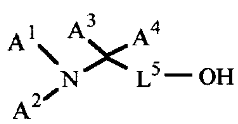 VI

wherein $L^5$ is alkylene or alkenylene with a total of up to 11 C atoms and at least 4 or, respectively, 3 C atoms between the two free valencies or cycloalkylene-alkylene,
with a compound of the formula

$$HO\text{-}M^6\text{-}T\text{-}Q,$$

wherein $M^6$ stands for 1,4-phenylene, which can be substituted as given in claim 1, or for thienylene,

h) reacting an aminoalcohol of formula VI with a chloride of the formula

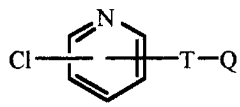

VII,

i) reacting an acid addition salt of an amine of the formula

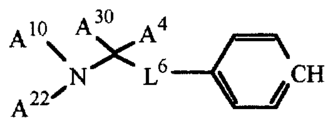

VIII

wherein $A^{10}$ is alkyl, $A^{22}$ is cycloalkyl, cycloalkyl-alkyl or an alkyl group optionally substituted by a group $R^{10}$ or CN,
$A^{30}$ and $A^4$ are hydrogen or alkyl or
$A^{10}$ and $A^{22}$ or $A^{30}$ together form an alkylene group optionally substituted by $R^{11}$, whereby a C atom in such an alkylene group can be replaced by N-alkyl,
$R^{11}$ is oxo, alkyl (O or S) or dialkylamino and
$L^6$ is phenylene or alkylene with a total of up to 11 C atoms and at least 4 C atoms between the two free valencies which is bonded to the phenyl ring directly or via O or N-alkyl,

with an acid chloride of the formula

$$ClC(O)\text{-}Q^6$$

wherein $Q^6$ is cycloalkyl, $C(R^{70},R^{80})$, phenyl substituted by one or more substituents from the group of alkyl, halogen, dialkylamino, CN, $NO_2$, $CF_3$, 1,2,4-triazol-1-yl and tetrazol-1-yl or a straight-chain alkyl group with 0 to 3 methyl substituents and a total of 6 to 13 C atoms and $R^{70}$ and $R^{80}$ stand for $C_{5\text{-}11}$-alkyl,

j) reacting a diamine of the formula

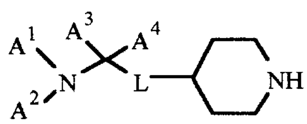

IX

with a halide of the formula

$$ClS(O)_2\text{-}Q,\ ClC(O)\text{-}Q \text{ or } BrCH_2C(O)\text{-}Q,$$

k) reacting an aldehyde of the formula

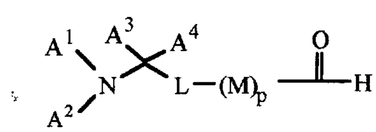

X

with an agent which introduces the group Q, whereby Q is cycloalkyl, $C(R^7,R^8)$, phenyl substituted by one or

more substituents from the group of alkyl, halogen, $N(R^{90},R^{100})$, CN, $NO_2$, $CF_3$, 1,2,4-triazol-1-yl and tetrazol-1-yl or a straight-chain alkyl, alkenyl, alkadienyl or alkatrienyl group $Q^{10}$ with 0 to 3 methyl substituents and a total of 6 to 13 C atoms, whereby such a group $Q^{10}$ can be substituted by $N(R^{90},R^{100})$ and $R^{90}$ and $R^{100}$ are alkyl or alkenyl,

l) if desired, functionally modifying reactive groups present in a compound of formula I and

m) if desired, converting an amine of formula I into a physiologically compatible acid addition salt or converting an acid addition salt of a compound of formula I into the amine of formula I.

**28.** An antimycotically-active and cholesterol-lowering medicament containing a compound according to any one of claims 1-25 as the active ingredient [and] optionally in addition a therapeutically inert carrier.

**29.** The use of compounds according to any one of claims 1-25 for the production of antimycotically-active and cholesterol-lowering medicaments.

**Revendications**

**1.** Amines tertiaires de formule :

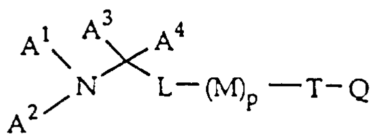

(I)

dans laquelle

$A^1$ représente un alkyle ou un alcényle et

$A^2$ représente un cycloalkyle, un cycloalkylalkyle ou un groupe alkyle ou alcényle éventuellement substitué par un groupe $R^1$, $CONH_2$ ou CN et, si $A^1$ est un alkyle, $A^2$ peut également représenter OH,

$A^3$ et $A^4$ représentent un hydrogène ou un alkyle ou

$A^1$ avec $A^2$ ou avec $A^3$ forment un groupe alkylène, alcénylène, ou alcadiénylène éventuellement substitué par $R^1$, $A^1$-$A^2$ ou selon les cas $A^1$-$A^3$ avec jusqu'à 5 atomes de carbone,

où dans un groupe $A^1$-$A^2$ ou $A^1$-$A^3$ jusqu'à 2 atomes de carbone peuvent être remplacés par un ou deux atomes d'azote et/ou par un groupe N-alkyle,

$R^1$ représente un OH, oxo, alkyle (O ou S) ou dialkylamino lié à un atome de carbone saturé de $A^2$, $A^1$-$A^2$ ou $A^1$-$A^3$,

où un atome de carbone substitué par $R^1$ ou selon les cas un atome de carbone insaturé contenu dans $A^1$, $A^2$, $A^1$-$A^2$ ou $A^1$-$A^3$ doit être lié à $N(A^1A^2)$ en une position autre que la position $\alpha$,

p = 1 et L représente un phénylène ou un alkylène ou alcénylène lié directement ou par l'intermédiaire de O, NH ou N(alkyle ou alcanoyle) à M avec au total jusqu'à 11 atomes de carbone et au moins quatre ou selon les cas trois atomes de carbone entre les deux valences libres ou un cycloalkylènealkylène ou

p = 0 et L représente un alcénylène en $C_6$ à $C_{11}$ ou alcadiénylène en $C_6$ à $C_{11}$ lié à T,

M représente un thiénylène, un pyridylène, un 1,4-phénylène, un 1,4-phénylène substitué par un l'un des divers substituants du groupe constitué par alkyle, halogène, $N(R^2,R^{21})$, $CONH_2$, CN, $NO_2$, $CF_3$, OH, alkyle (O ou S), 1,2,4-triazol-1-yle ou tétrazol-1-yle substitué par un 1,4-phénylène ou un groupe de formule :

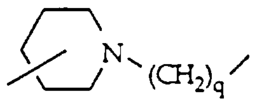

$(M^1)$

q vaut 1 ou 0,

$R^2$ et $R^{21}$ représentent H, un alkyle, un alcényle, un alcanoyle ou $SO_2$-alkyle,

T représente CO, $CH(R^3)$, $C(R^4,R^5)$ ou $C{=}NOR^6$ et, si M représente un groupe $M^1$ et q = 0, T peut également représenter $SO_2$,

$R^3$ représente OH, F, un alcoxy ou un alcanoyloxy,

$R^4$ représente OH, et $R^5$ représente un alkyle, un alcényle, un alcynyle, un cycloalkyle ou $CF_3$, ou

$R^4$ représente avec $R^5$ le groupe $CH_2$, $CH_2O$ ou $CH_2CH_2$,

$R^6$ représente H, un alkyle ou un alcényle,

Q représente un cycloalkyle, $C(R^7,R^8)$, un phényle substitué par un ou plusieurs substituants du groupe constitué par alkyle, halogène, $N(R^9,R^{10})$, $CONH_2$, CN, $NO_2$, $CF_3$, 1,2,4-triazol-1-yle et tétrazol-1-yle ou un groupe alkyle, alcényle, alcadiényle ou alcatriényle à chaîne droite Q' comportant de 0 à 3 substituants méthyle et ayant au total de 6 à 13 atomes de carbone, où un groupe Q' de ce genre peut être substitué par OH et/ou par $N(R^9,R^{10})$,

$R^7$ et $R^8$ représentent un alkyle en $C_5$ à $C_{11}$, un alcényle en $C_5$ à $C_{11}$ ou un alcadiényle en $C_5$ à $C_{11}$ et

$R^9$ et $R^{10}$ représente H, un alkyle, un alcényle ou un alcanoyle,

sous réserve que

a) dans un composé de formule 1 dans laquelle T représente un groupe CO ou CHOH, L représente un phénylène ou un groupe alkylène ou alcénylène lié à M directement ou par l'intermédiaire de O ou un N-alkyle, M représente un 1,4-phénylène ou un 1,4-phénylène monosubstitué par un alkyle, un alcoxy, un halogène, CN, $NO_2$ et $CF_3$ et Q représente un phényle substitué, un groupe alcényle ou un groupe alkyle éventuellement substitué par OH, $A^2$ ne doit pas représenter un alkyle ou un alcényle ou $A^1$ avec $A^2$ ne doit pas être un alkylène,

b) dans une composé de formule I dans laquelle $A^1$ représente avec $A^2$ un alkylène ou un alkylène substitué par $R^1$, $A^2$ représente un hydroxyalkyle ou $A^1$ et $A^2$ représentent chacun un groupe alkyle, M ne doit pas être un pyridylène et

c) dans un composé de formule I dans laquelle T représente un groupe $C(OH,R^{51})$, où $R^{51}$ représente un alkyle, un alcényle, un alcynyle ou un cycloalkyle, M représente un 1,4-phénylène ou un 1,4-phénylène substitué et L représente un groupe alkylène lié à M par l'intermédiaire d'un atome de O, ce groupe alkylène devant contenir au moins 5 atomes de carbone entre les deux valences libres et au total jusqu'à 11 atomes de carbone,

et leurs sels d'addition d'acides.

**2.** Composés selon la revendication 1, dans lesquels $A^2$ représente un cycloalkyle ou un cycloalkylalkyle et T représente CO.

**3.** Composés selon la revendication 1 ou 2 de formule :

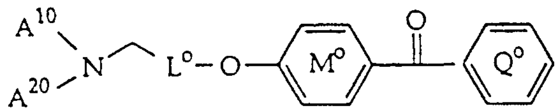

(Ia)

dans laquelle $A^{10}$ représente un alkyle, $A^{20}$ représente un cycloalkyle ou un cycloalkylalkyle, $L^o$ représente un alkylène ou un alcénylène ayant au total jusqu'à 11 atomes de carbone et contient au moins quatre ou selon les cas trois atomes de carbone entre les deux valences libres ou un cycloalkylènealkylène, $M^o$ représente un 1,4-phénylène éventuellement halogéné et $Q^o$ un phényle substitué par un halogène ou CN, en particulier où $A^{10}$ représente un méthyle, $A^{20}$ un cyclopropyle ou un cyclopropylméthyle, $L^o$ un n-pentylène, un n-propénylène, ou un cyclopropylèneméthylène, $M^o$ un 1,4-phénylène non substitué ou fluoré et $Q^o$ est un phényle substitué par Br ou CN, en particulier les composés suivants :

(4-bromophényl)-[4-[6-(cyclopropylméthylamino)-hexyloxy]-2-fluorophényl]-méthanone,

(E)-(4-bromophényl)-[4-[4-(cyclopropylméthylamino)-but-2-ényloxy]-phényl]-méthanone,

[6-[6-(cyclopropylméthylamino)-hexyloxy]-phényl]-(4-bromophényl)-méthanone,

(E)-4-[4-[4-cyclopropylméthylamino)-but-2-ényloxy]-3-fluorobenzoyl]-benzonitrile,

(4-bromophényl)-[4-[6-(cyclopropylméthylméthylamino)-hexyloxy]-phényl]-méthanone,

(1 RS,2RS)-(4-bromophényl)-[4-[2-[(cyclopropylméthylamino)-méthyl]-cyclopropylméthoxy]-phényl]-méthanone,

(1RS,2RS)-(4-bromophényl)-[4-[2-[(cyclopropylméthylamino)-méthyl]-cyclopropylméthoxy]-3-fluorophényl]-méthanone.

**4.** Composés selon la revendication 1 ou 2 du groupe des suivants :

1-[4-[6-(cyclopropylméthylméthylamino)-hexyloxy]-2-fluorophényl]-5-méthylhex-4-én-1-one,

1 -[4-[6-(cyclopropylméthylamino)-hexyloxy]-2-fluorophényl]-5-méthylhex-4-én-1-one,

(E)-(4-bromophényl)-[4-[4-(cyclopropylméthylméthylamino)-but-2-ényloxy]-phényl]-méthanone,

(E)-4-[4-[4-(cyclopropylméthylamino)-but-2-ényloxy]-3-fluorobenzoyl]-benzamide,

(1RS,2RS)-4-[4-[2-[(cyclopropylméthylamino)-méthyl]-cyclopropylméthoxy]-3-fluorobenzoyl]-benzonitrile,

(1RS,2RS)-(4-bromophényl)-[4-[2-[(cyclopropylméthylméthylamino)-méthyl]-cyclopropylméthoxy]-phényl]-méthanone,

(1RS,2RS)-(4-bromophényl)-[4-[2-[(cyclopropylméthylamino)-méthyl]-cyclopropylméthoxy]-2-fluorophényl]-méthanone,

(1RS,2RS)-[4-[2-[(allylcyclopropylamino)-méthyl]-cyclopropylméthoxy]-phényl]-(4-bromophényl]-méthanone,

(1RS,2RS)-[4-[2-[(cyclopropylméthylamino)-méthyl]-cyclopropylméthoxy]-phényl]-5-méthylhexane-1-one.

**5.** Composés selon la revendication 1, dans lesquels T représente un groupe CHOH, CHF, $C(R^4,R^5)$ ou $C=NOR^6$ et $R^4$, $R^5$ et $R^6$ ont la même signification que dans la revendication 1, en particulier où T représente un groupe C (OH, alkyle), C(OH, alcényle), $C=CH_2$ ou C=NO-alkyle.

**6.** Composés selon la revendication 1 ou 5 de formule :

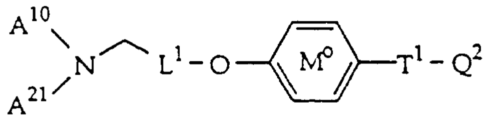

(Ib)

dans laquelle $A^{10}$ représente un alkyle, $A^{21}$ représente un alcényle, $L^1$ représente un alkylène ou un alcénylène ayant au total jusqu'à 11 atomes de carbone et au moins quatre ou selon les cas trois atomes de carbone entre les deux valences libres, $M^o$ représente un 1,4-phénylène éventuellement halogéné, $T^1$ représente un groupe C (OH, alkyle), C(OH, alcényle), $C=CH_2$ ou C=NO-alkyle et $Q^2$ représente un halophényle ou un alcényle ayant de 0 à 3 substituants méthyle et au total de 6 à 13 atomes de carbone, en particulier où $A^{10}$ représente un méthyle, $A^{21}$ représente un allyle, $L^1$ représente un n-pentylène ou un n-propénylène, $M^o$ représente un 1,4-phénylène non substitué ou fluoré et $Q^2$ représente un bromophényle ou un 4-méthyl-pent-3-ényle, en particulier les composés suivants :

(E)-(RS)-1-[4-[4-(allylméthylamino)-but-2-ényloxy]-phényl]-(4-bromophényl)-éthanol,

(E)-(RS)-1-[4-[4-(allylméthylamino)-but-2-ényloxy]-phényl]-1-(4-bromophényl)-prop-2-én-1-ol,

(E)-allyl-[4-[4-[1-(4-bromophényl)-vinyl]-phénoxy]-but-2-ényl]-méthylamine,

(RS)-1-[4-[6-(allylméthylamino)-hexyloxy]-2-fluorophényl]-1-(4-bromophényl)-éthanol,

(E)-(RS)-2-[4-[4-allylméthylamino)-but-2-ényloxy]-phényl]-6-méthylhept-5-én-2-ol.

**7.** Composés selon la revendication 1 ou 5, du groupe des suivants :

(E)-(RS)-[4-[4-(allylméthylamino)-but-2-ényloxy]-phényl]-(4-bromophényl)-cyclopropylméthanol,

(E)-(RS)-1-[4-[4-(allylméthylamino)-but-2-ényloxy]-phényl]-(4-bromophényl)-2,2,2-trifluoroéthanol,

(RS)-1-[4-[6-(allylméthylamino)-hexyloxy]-phényl]-1-(4-bromophényl)-2,2, 2-trifluoroéthanol,

(E)-allyl-[4-[4-[1-(4-bromophényl)-cyclopropyl]-phénoxy]-but-2-ényl]-méthylamine,

(E)-(R ou S)-1-[4-[4-(allylméthylamino)-but-2-ényloxy]-phényl]-(4-bromophényl)-éthanol,

(E)-(S ou R)-1-[4-[4-(allylméthylamino)-but-2-ényloxy]-phényl]-(4-bromophényl)-éthanol,

Allyl-[6-[4-[1-(4-bromophényl)-vinyl]-3-fluorophénoxy]-hexyl]-méthylamine,

(E)-(RS)-1-[4-[4-(allylméthylamino)-but-2-ényloxy]-3-fluorophényl]-(4-bromophényl)-prop-2-én-1-ol,

(RS)-1-[4-[(allylméthylamino)-méthyl]-biphényl-4-yl]-1-(4-bromophényl)-éthanol,

(RS)-5-[6-(allylméthylamino)-hexyloxy]-2-[1-(4-bromophényl)-1-hydroxyallyl]-phénol,

(RS)-1-[4-[6-(allylméthylamino)-hexyloxy]-2-aminophényl]-1-(4-bromophényl)-prop-2-én-1-ol,

(RS)-allyl-[4'-[(4-bromophényl)-fluorométhyl]-biphényl-4-ylméthyl]-méthylamine,

(E)-et/ou (Z)-[4-[(E)-4-(allylméthylamino)-but-2-ényloxy]-phényl]-(4-bromophényl)-méthanone-O-méthyloxime,

(E)-et/ou (Z)-[4-[(E)-4-allylméthylamino)-but-2-ényloxy]-phényl]-(4-bromophényl)-méthanoneoxime,

(E)-et/ou (Z)-[4-[(E)-4-allylméthylamino)-but-2-ényloxy]-phényl]-(4-bromophényl)-méthanone-O-tert-butyloxime,

(E)-et/ou (Z)-[4-[(E)-4-allylméthylamino)-but-2-ényloxy]-phényl]-(4-bromophényl)-méthanone-O-allyloxime,

(E)- et/ou (Z)-[4-[6-allylméthylamino)-hexyloxy]-2-fluorophényl]-(4-bromophényl)-méthanoneoxime.

**8.** Composés selon la revendication 1, dans lesquels M représente un 1,4-phénylène éventuellement substitué par un alkyle, un halogène, $NH_2$, un amino mono- ou dialkylé, un alcanoylamino, un alkylsulfonylamino, OH, un alkyle (O ou S), ou un 1,2,4-triazol-1-yle, en particulier où M est un 1,4-phénylène substitué par $NH_2$, un amino mono- ou dialkylé, OH, un S-alkyle ou par deux atomes d'halogène.

**9.** Composés selon la revendication 1 ou 8 de formule :

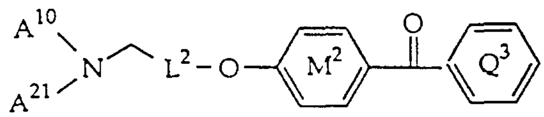

(Ic)

dans laquelle $A^{10}$ représente un alkyle, $A^{21}$ représente un alcényle, $L^2$ représente un alkylène ayant jusqu'à 11 atomes de carbone et au moins quatre atomes de carbone entre les deux valences libres, $M^2$ représente un 1,4-phénylène substitué par $NH_2$, un amino mono- ou dialkylé, OH, un S-alkyle ou deux atomes d'halogène, et $Q^3$ représente un phényle halogéné, en particulier où $A^{10}$ représente un méthyle, $A^{21}$ représente un allyle, $L^2$ représente un n-pentylène, $M^2$ représente un 1,4-phénylène substitué par $NH_2$, $NHCH_3$, $N(CH_3)_2$, OH, $SCH_3$ ou par deux atomes de F et $Q^3$ représente un bromophényle, en particulier les composés suivants :

(E)-[4-[4-(allylméthylamino)-but-2-ényloxy]-2,5-difluorophényl]-(4-bromophényl)-méthanone,

[4-[6-(allylméthylamino)-hexyloxy]-2-méthylsulfanylphényl]-(4-bromophényl)-méthanone,

[4-[6-(allylméthylamino)-hexyloxy]-2-méthylaminophényl]-(4-bromophényl)-méthanone,

[4-[6-(allylméthylamino)-hexyloxy]-2-diméthylaminophényl]-(4-bromophényl)-méthanone,

[4-[6-(allylméthylamino)-hexyloxy]-2-hydroxyphényl]-(4-bromophényl)-méthanone,

[2-amino-4-[6-(allylméthylamino)-hexyloxy]-phényl]-(4-bromophényl)-méthanone.

**10.** Composés selon la revendication 1 ou 8 du groupe des suivants :

(E)-(4-bromophényl)-[2,5-difluoro-4-(4-diméthylaminobut-2-ényloxy]-méthanone,

[4-[6-(allylméthylamino)-hexyloxy]-2,5-difluorophényl]-(4-bromophényl)-méthanone,

(E)-[4-[4-(allylméthylamino)-but-2-ényloxy]-2,5-difluorophényl-(2,4-difluorophényl)-méthanone,

(E)-[2,5-difluoro-4-(4-diméthylaminobut-2-ényloxy)-phényl]-(2,4-difluorophényl)-méthanone,

[4-[6-(allylméthylamino)-hexyloxy]-2,5-difluorophényl]-(2,4-difluorophényl)-méthanone,

(2,4-difluorophényl)-[4-(6-diméthylaminohexyloxy)-2,5-difluorophényl]-méthanone,

(E)-[4-[4-(allylméthylamino)-but-2-ényloxy]-2,3,5,6-tétrafluorophényl] (4-bromophényl)-méthanone,

(E)-[4-[4-allylméthylamino)-but-2-ényloxy]-3-fluoro-2-méthylphényl]-(4-bromophényl)-méthanone,

(E)-[4-[4-allylméthylamino)-but-2-ényloxy]-2-méthylsulfanylphényl]-(4-bromophényl)-méthanone,

(E)-N-[1-[4-[4-(allylméthylamino)-but-2-ényloxy]-3-fluorophényl]-11-oxo-undécyl]-acétamide,

(E)-[4-(4-allylméthylaminobut-2-ényloxy)-2-hydroxyphényl]-(4-bromophényl)-méthanone,

(E)-1-[4-[4-(allylméthylamino)-but-2-ényloxy]-3-fluorophényl]-11-aminoundécan-1-one,

(E)-1-[4-[(E)-4-(allylméthylamino)-but-2-ényloxy]-3-fluorophényl]-2-[(E)-3,7-diméthylocta-2,6-diényl]-5,9-diméthyldéca-4,8-dién-1-one,

(E)-1-[4-[4-(allylméthylamino)-but-2-ényloxy]-3-fluorophényl]-5-méthyl-2-( 3-méthylbut-2-ényl)-hex-4-én-1-one,

(E)-(R,S)-1-[4-[4-(allylméthylamino)-but-2-ényloxy]-3-fluorophényl]-3-hydroxy-5-méthyl-hex-4-én-1-one,

(E)-(R,S)-1-[4-[4-(allylméthylamino)-but-2-ényloxy]-2-fluorophényl]-3-hydroxy-5-méthylhex-4-én-1-one,

(E)-(RS)-1-[4-[4-(allylméthylamino)-but-2-ényloxy]-phényl]-3-hydroxy-5-méthylhex-4-én-1-one,

(E)-(RS)-1-[4-[(E)-4-(allylméthylamino)-but-2-ényloxy]-3-fluorophényl]-3-hydroxy-5,9-diméthyldéca-4,8-dién-1-one,

(E)-(RS)-13-(allylméthylamino)-1-[4-[4-(allylméthylamino)-but-2-ényloxy]-3-fluorophényl]-3-hydroxytridécan-1-one,

(E)-1-[4-[(E)-4-(allylméthylamino)-but-2-ényloxy]-3-fluorophényl]-5-méthylhexa-2,4-dién-1-one,

(E)-13-(allylméthylamino)-1-[4-[(E)-4-(allylméthylamino)-but-2-ényloxy]-3-fluorophényl]-tridéc-2-én-1-one,

(E)-1-[4-[(E)-4-(allylméthylamino)-but-2-ényloxy]-2-fluorophényl]-5-méthylhexa-2,4-dién-1-one,

(E)-1-[4-[(E)-4-(allylméthylamino)-but-2-ényloxy]-phényl]-5-méthylhexa-2,4-dién-1-one,

(2E,4E)-1-[4-(E)-4-(allylméthylamino)-but-2-ényloxy]-3-fluorophényl]-5,9-diméthyldéca-2,4,8-trién-1-one,

[4-[6-(allylméthylamino)-hexyloxy]-2-1H-[1,2,4]-triazol-1-yl-phényl]-(4-bromophényl)-méthanone,

1-[4-[6-(allylméthylamino)-hexyloxy]-2-méthylaminophényl]-4-méthylhex-5-én-1-one,

(E)-1-[4-[4-(allylméthylamino)-but-2-ényloxy]-2-méthylsulfanylphényl]-5-méthylhex-4-én-1-one,

N-[5-[6-(allylméthylamino)-hexyloxy]-2-(4-bromobenzoyl)-phényl]-méthanesulfonamide,

(4-bromophényl)-(4'-diméthylaminométhyl-3-hydroxybiphényl-4-yl)-méthanone,

N-[5-[6-allylméthylamino)-hexyloxy]-2-(4-bromobenzoyl)-phényl]-formamide.

**11.** Composés selon la revendication 1 dans lesquels L représente un groupe alkylène ou alcénylène lié par l'intermédiaire de NH ou d'un N-alcanoyle à M avec au total jusqu'à 11 atomes de carbone et au moins 4 ou selon les cas 3 atomes de carbone entre les deux valences libres.

**12.** Composés selon la revendication 1 ou 11 de formule :

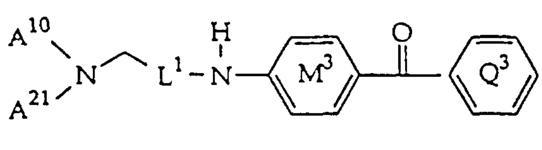

(Id)

dans laquelle $A^{10}$ représente un alkyle, $A^{21}$ représente un alcényle, $L^1$ représente un alkylène ou un alcénylène ayant jusqu'à 11 atomes de carbone et au moins quatre ou selon les cas trois atomes de carbone entre les deux valences libres, $M^3$ représente un 1,4-phénylène halogéné et $Q^3$ représente un phényle halogéné, en particulier où $A^{10}$ représente un méthyle, $A^{21}$ représente un allyle, $L^1$ représente un n-pentylène ou un n-propénylène, $M^3$ représente un fluorophénylène et $Q^3$ représente un bromophényle, en particulier les composés suivants :

(E)-[4-[4-(allylméthylamino)-but-2-énylamino]-3-fluorophényl]-(4-bromophényl)-méthanone,
[4-[6-(allylméthylamino)-hexylamino]-3-fluorophényl]-(4-bromophényl)-méthanone,

**13.** Composés selon la revendication 1 ou 11 du groupe des suivants :

(E)-N-[4-(allylméthylamino)-but-2-ényl]-N-[4-(4-bromobenzoyl)-3-fluorophényl]-acétamide,

N-[6-(allylméthylamino)-hexyl]-N-[4-(4-bromobenzoyl)-2-fluorophényl]-acétamide.

**14.** Composés selon la revendication 1, dans lesquels M représente un groupe de formule :

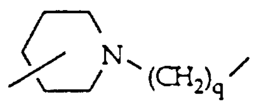

$(M^1)$

et q vaut 1 ou 0, en particulier où M forme avec T le groupe pipéridine-1-yl sulfonyle.

**15.** Composés selon la revendication 1 ou 14 de formule :

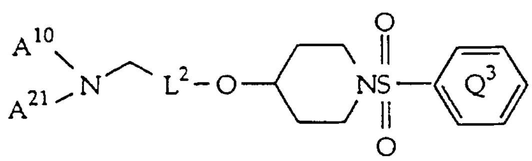

(Ie)

dans laquelle $A^{10}$ représente un alkyle, $A^{21}$ un alcényle, $L^2$ un alkylène ayant jusqu'à 11 atomes de carbone et au moins 4 atomes de carbone entre les deux valences libres et $Q^3$ un phényle halogéné, en particulier où $A^{10}$ représente un méthyle, $a^{21}$ un allyle, $L^2$ un n-pentylène et $Q^3$ un bromophényle, en particulier l'allyl-[6-[1-(4-bromophénylsulfonyl)-pipéridine-4-yloxy]-hexyl]-méthylamine.

**16.** Composés selon la revendication 1 ou 14 du groupe constitué par les suivants :

[4-[6-(allylméthylamino)-hexyloxy]-pipéridin-1-yl]-1-(4-bromophényl)-méthanone,
2-[4-[6-(allylméthylamino)-hexyloxy]-pipéridin-1-yl]-1-(4-bromophényl)-éthanone.

**17.** Composés selon la revendication 1, dans lesquels $A^1$ représente un alkyle et $A^2$OH ou un alkyle éventuellement substitué par un groupe $R^1$, $CONH_2$ ou CN ou

$A^1$ forme avec $A^2$ ou avec $A^3$ un groupe alkylène, alcénylène ou alcadiényle éventuellement substitué par $R^1$, soit $A^1$-$A^2$ ou selon les cas $A^1$-$A^3$ avec jusqu'à 5 atomes de carbone,

où dans un groupe $A^1$-$A^2$ ou $A^1$-$A^3$ un atome de carbone peut être remplacé par un atome d'azote et

$R^1$ est un OH, oxo, alkyl(O ou S) ou dialkylamino lié sur un atome de carbone saturé de $A^2$, $A^1$-$A^2$ ou $A^1$-$A^3$,

où un atome de carbone substitué par $R^1$ ou selon les cas un atome de carbone non saturé contenu dans $A^2$, $A^1$-$A^2$ ou $A^1$-$A^3$ doit être lié à $N(A^1A^2)$ en une position autre que la position $\alpha$.

**18.** Composés selon la revendication 1 ou 17, dans lesquels $A^1$ avec $A^2$ est un alkylène substitué par OH avec jusqu'à

5 atomes de carbone.

**19.** Composés selon la revendication 1, 17 ou 18 de formule :

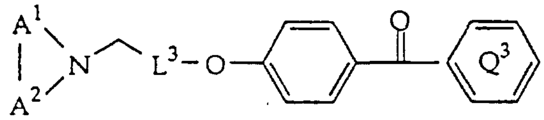

(If)

dans laquelle $A^1$ représente avec $A^2$ un groupe alkylène substitué par OH avec jusqu'à 5 atomes de carbone, $L^3$ un alcénylène avec au total jusqu'à 11 atomes de carbone et au moins trois atomes de carbone entre les deux valences libres et $Q^3$ un phényle halogéné, en particulier où $A^1$ avec $A^2$ est un 4-hydroxypipéridine-1-yle, $L^3$ un n-propénylène et $Q^3$ un bromophényle, en particulier le : (E)-(4-bromophényl)-1-[4-[4-(4-hydroxypipéridin-1-yl)-but-2-ényloxy]-phényl]-méthanone.

**20.** Composés selon la revendication 1 ou 17 du groupe des suivants :

cyclohexyl-p-[[(E)-4-(diméthylamino)-2-butényl]-oxy]-phénylcétone, (E)-[[4-[4-(4-bromobenzoyl)-phénoxy]-but-2-ényl]-méthylamino]-acétonitrile,

(E)-[[4-[4-(4-bromobenzoyl)-phénoxy]-but-2-ényl]-méthylamino]-propionitrile,

(E)-(4-bromophényl)-[4-[4-(4-diméthylaminopipéridin-1-yl)-but-2-ényloxy]-phényl]-méthanone,

(4-bromophényl)-[4-[6-(hydroxyméthylamino)-hexyloxy]-phényl]-méthanone,

(E)-(4-bromophényl)-[4-[4-(hydroxyméthylamino)-but-2-ényloxy]-phényl]-méthanone,

(E)-(4-bromophényl)-[4-[4-[(2-méthoxyéthyl)-méthylamino]-but-2-ényloxy]-phényl]-méthanone,

(E)-(4-bromophényl)-[4-[4-[méthyl-(2-méthylsulfanyléthyl)-amino]-but-2-ényloxy]-phényl]-méthanone,

(E)-(4-bromophényl)-[4-(4-imidazol-1-ylbut-2-ényloxy)-phényl]-méthanone,

(4-bromophényl)-[4-(6-imidazol-1-ylhexyloxy)-phényl]-méthanone,

(4-bromophényl)-[4-[6-[(3-hydroxypropyl)-méthylamino]-hexyloxy]-phényl]-méthanone,

1-[[6-[4-(4-bromobenzoyl)-phénoxy]-hexyl]-méthylamino]-propane-2-one,

(E)-2-[[4-[4-bromobenzoyl)-phénoxy]-but-2-ényl]-méthylaminoacétamide,

(±) (4-bromophényl)-[4'-(1-méthylpyrrolidine-2-yl)-biphényl-4-yl]-méthanone.

**21.** Composés selon la revendication 1, dans lesquels p = 0 et L est un alcénylène en $C_6$ à $C_{11}$ ou un alcadiénylène en $C_6$ à $C_{11}$ lié sur T.

**22.** Composés selon la revendication 1 ou 21 de formule :

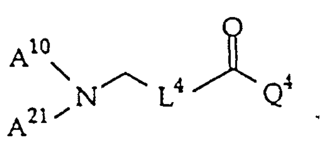

(Ig)

dans laquelle $A^{10}$ représente un alkyle, $A^{21}$ un alcényle, $L^4$ un alcadiénylène en $C_6$ à $C_{11}$ et $Q^4$ un groupe alcényle

ayant de 0 à 3 substituants méthyle et au total de 6 à 13 atomes de carbone, en particulier où $A^{10}$ représente un méthyle, $A^{21}$ un allyle, $L^4$ un diméthyloctadiénylène et $Q^4$ un 4-méthyl-3-penténylе, en particulier la (9E,13E)-15-(allylméthylamino)-2,9,13-triméthyl-pentadéca-2,9,13-trién-6-one.

**23.** Composés selon la composition 1 ou 21 du groupe des suivants :

(4E,8E)-10-(allylméthylamino)-1-(4-bromophényl)-4,8-diméthyldéca-4,8-dién-1-one,

(4E,8E)-1-(4-bromophényl)-10-diméthylamino-4,8-diméthyldéca-4,8-dién-1-one,

(7E,11E)-13-(allylméthylamino)-2,7,11-triméthyltridéca-2,7,11-trién-6-one,

(7E,11E)-et (7Z,11E)-13-(allylméthylamino)-2,7,11-triméthyltridéca-2,7,11-trién-6-one,

(2 E,6E)-8-(allylméthylamino)-1-(4-bromophényl)-2,6-diméthylocta-2,6-dién-1-one,

(7E,11E)-13-(allylméthylamino)-7,11-diméthyltridéca-1,7,11-trién-6-one,

(2E,6E)-(RS)-8-(allylméthylamino)-1-(4-bromophényl)-2,6-diméthylocta-2,6-dién-1-ol,

(E)-(RS)-8-(allylméthylamino)-1-(4-bromophényl)-2,6-diméthyloct-6-én-1-one,

(2E,6E)-(RS)-10-(allylméthylamino)-1-(4-bromophényl)-3,7-diméthyldéca-2,6-dién-1-ol,

(2E,6E)-(RS)-8-(allylméthylamino)-1-(4-bromophényl)-3,7-diméthyldéca-2,6-dién-1-ol,

(2E,6E)-10-(allylméthylamino)-1-(4-bromophényl)-3,7-diméthyidéca-2,6-dién-1-one,

(2E,6E)-8-(allylméthylamino)-1-(4-bromophényl)-3,7-diméthyldéca-2,6-dién-1-one.

**24.** Composés selon la revendication 1, dans lesquels M est un thiénylène ou un pyridylène, en particulier du groupe des suivants :

(E)-1-[6-[6-(allylméthylamino)-hexyloxy]-pyridin-3-yl]-5-méthylhexa-2,4-dién-1-one,

6-[6-(allylméthylamino)-hexyloxy]-pyridin-3-yl]-(4-bromophényl)-méthanone,

(E)-[6-[4-(allylméthylamino)-but-2-ényloxy]-pyridin-3-yl]-(4-bromophényl)-méthanone,

[5-[6-(allylméthylamino)-hexyloxy]-pyridin-2-yl]-(4-bromophényl)-méthanone,

5-(4-[(allylméthylamino)-méthyl]-phényl)-thiophén-2-yl)-(4-bromophényl)-méthanone,

5-(4-[(diméthylamino)-méthyl]-phényl)-thiophén-2-yl)-(4-bromophényl)-méthanone,

5-(4-[(allylméthylamino)-méthyl]-phényl)-thiophén-2-yl)-(4-(2,4-difluorophényl))-méthanone,

(2-diméthylamino-4-fluorophényl)-[5-(4-diméthylaminométhylphényl)-thiophén-2-yl]-méthanone.

**25.** Composés selon la revendication 1, dans lesquels L est un cycloalkylènealkylène lié par un atome d'oxygène à M, en particulier les suivants :

(1RS,2RS)-(4-bromophényl)-[4-[2-[(éthylméthylamino)-méthyl]-cyclopropylméthoxy]-phényl]-méthanone,

(1RS,2RS)-[4-[2-[(allylméthylamino)-méthyl]-cyclopropylméthoxy]-phényl]-(4-bromophényl)-méthanone.

**26.** Composés selon l'une des revendications 1 à 25, aux fins d'application comme substances actives thérapeutiques.

**27.** Procédé de préparation des composés selon la revendication 1, caractérisé en ce que :

a) on fait réagir un bromure de formule :

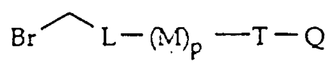

(II)

avec une amine $HN(A^1,A^2)$,

b) on méthyle une amine de formule :

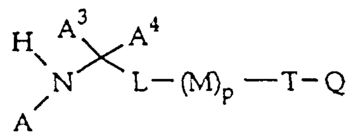

(III)

dans laquelle A a la même signification que $A^2$ dans la revendication 1,

c) on fait réagir une amine de formule III dans laquelle A a la même signification que $A^1$ dans la revendication 1, avec un halogénure de formule Hal-A°, où Hal représente un halogène et A° un cycloalkylalkyle ou un alkyle ou alcényle substitué par un groupe $R^1$, $CONH_2$ ou CN,

d) on fait réagir une éthanone de formule :

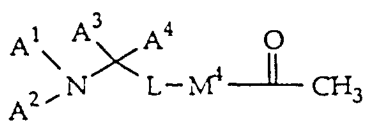

(IV)

dans laquelle $M^4$ représente un 1,4-phénylène, qui peut être substitué comme il est indiqué dans la revendication 1, ou un thiénylène ou un pyridylène, avec un halogénure de formule

Hal-$R^7$

pour donner une cétone de formule I, dans laquelle T avec Q est un groupe $C(O)C(R^7)_2$ et $R^7$ a la même signification que dans la revendication 1,

e) on fait réagir une éthanone de formule IV avec un aldéhyde de formule

HC(O)Q"

dans laquelle Q" représente un groupe alkyle, alcényle ou alcadiényle à chaîne droite avec de 0 à 3 substituants méthyle et au total de 4 à 11 atomes de carbone,

f) on fait réagir une β-hydroxycétone de formule :

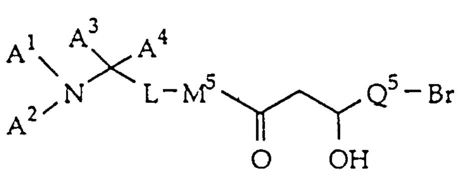

(V)

dans laquelle $M^5$ représente un 1,4-phénylène, qui peut être substitué comme il est indiqué dans la revendication 1, et $Q^5$ représente un groupe à deux liaisons correspondant à l'un des groupes à une liaison Q" ci-dessus,
avec une amine $HN(R^9,R^{10})$,

g) on fait réagir un aminoalcool de formule :

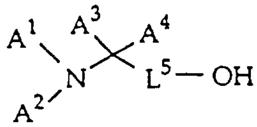

(VI)

dans laquelle $L^5$ représente un alkylène ou un alcénylène avec au total jusqu'à 11 atomes de carbone et au moins 4 ou selon les cas 3 atomes de carbone entre les deux valences libres ou un cycloalkylènealkylène, avec un composé de formule :

$$HO\text{-}M^6\text{-}T\text{-}Q$$

dans laquelle $M^6$ représente un 1,4-phénylène, qui peut être substitué comme il est indiqué dans la revendication 1, ou un thiénylène,

h) on fait réagir un aminoalcool de formule VI avec un chlorure de formule :

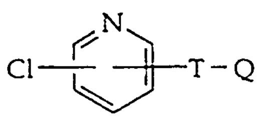

(VII)

i) on fait réagir un sel d'addition d'acide d'une amine de formule :

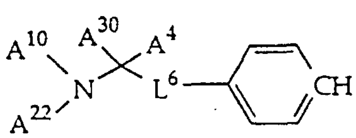

(VIII)

dans laquelle $A^{10}$ représente un alkyle, $A^{22}$ un cycloalkyle, un cycloalkylalkyle ou un groupe alkyle éventuellement substitué par un groupe $R^{10}$ ou CN,

$A^{30}$ et $A^4$ représentent un hydrogène ou un alkyle ou

$A^{10}$ forme avec $A^{22}$ ou avec $A^{30}$ un groupe alkylène éventuellement substitué par $R^{11}$, où dans un tel groupe alkylène un atome de carbone peut être remplacé par un N-alkyle,

$R^{11}$ représente un oxo, un alkyl(O ou S) ou un dialkylamino et

$L^6$ représente un phénylène ou un alkylène lié directement ou par l'intermédiaire d'un O ou d'un N-alkyle au noyau phényle avec au total jusqu'à 11 atomes de carbone et au moins 4 atomes de carbone entre les deux valences libres,

avec un chlorure d'acide de formule :

$ClC(O)-Q^6$

dans laquelle $Q^6$ représente un cycloalkyle, $C(R^{70},R^{80})$, un phényle substitué par un ou plusieurs substituants du groupe constitué par alkyle, halogène, dialkylamino, CN, $NO_2$, $CF_3$, 1,2-triazol-1-yle et un tétrazol-1-yle ou un groupe alkyle à chaîne droite ayant de 0 à 3 substituants méthyle et au total de 6 à 13 atomes de carbone et $R^{70}$ et $R^{80}$ représentent un alkyle en $C_5$ à $C_{11}$,

j) on fait réagir une diamine de formule :

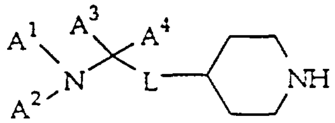

(IX)

avec un halogénure de formule :

$ClS(O)_2-Q$, $ClC(O)-Q$ ou $BrCH_2C(O)-Q$

k) on fait réagir un aldéhyde de formule :

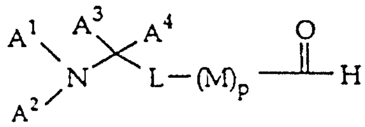

(X)

avec un agent introduisant le groupe Q, où Q représente un cycloalkyle, $C(R^7,R^8)$, un phényle substitué par un ou plusieurs substituants du groupe constitué par alkyle, halogène, $N(R^{90},R^{100})$, CN, $NO_2$, $CF_3$, 1,2,4-triazol-1-yle et tétrazol-1-yle ou un groupe alkyle, alcényle, alcadiényle ou alcatriényle à chaîne droite $Q^{10}$ avec de 0 à 3 substituants méthyle et au total de 6 à 13 atomes de carbone, où un tel groupe $Q^{10}$ peut être substitué par $N(R^{90},R^{100}$, et $R^{90}$ et $R^{100}$ représentent un alkyle ou un alcényle,

l) si on le désire on fait subir une transformation fonctionnelle à un groupe réactif contenu dans un composé de formule I et

m) si on le désire on transforme une amine de formule I en un sel d'addition d'acide physiologiquement acceptable ou un sel d'addition d'acide d'un composé de formule I en l'amine de formule I.

**28.** Agent à action antimycotique et abaissant le niveau de cholestérol, contenant un composé selon l'une des revendications 1 à 25 comme substance active, le cas échéant en plus d'un support thérapeutiquement inerte.

**29.** Utilisation des composés selon l'une des revendications 1 à 25 pour la préparation d'agents à action antimycotique et abaissant le niveau de cholestérol.